(19) Europäisches Patentamt
European Patent Office
Office européen des brevets

(11) EP 4 289 814 A1

(12) EUROPEAN PATENT APPLICATION
published in accordance with Art. 153(4) EPC

(43) Date of publication:
**13.12.2023 Bulletin 2023/50**

(21) Application number: **22749755.9**

(22) Date of filing: **02.02.2022**

(51) International Patent Classification (IPC):
***C07C 217/08*** *(2006.01)*

(52) Cooperative Patent Classification (CPC):
**A61K 9/127; A61K 31/7105; A61K 31/713; A61K 47/18; A61K 48/00; A61P 25/00; C07C 217/08; C07C 217/12; C07C 311/26; C07D 205/04; C07D 207/12; C07D 211/46; C07D 211/54; C07D 223/08; C07D 295/03;** (Cont.)

(86) International application number:
**PCT/JP2022/004118**

(87) International publication number:
**WO 2022/168884 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.02.2021 JP 2021016910**

(71) Applicant: **Shionogi & Co., Ltd**
**Osaka-shi, Osaka 541-0045 (JP)**

(72) Inventors:
- **KUGIMIYA Akira**
  **Osaka-shi, Osaka 541-0045 (JP)**
- **NAKAMURA Jun**
  **Osaka-shi, Osaka 541-0045 (JP)**
- **KURODA Norikazu**
  **Osaka-shi, Osaka 541-0045 (JP)**
- **TANINO Tetsuya**
  **Osaka-shi, Osaka 541-0045 (JP)**
- **HAYATA Atsushi**
  **Osaka-shi, Osaka 541-0045 (JP)**
- **MORITA Ippei**
  **Osaka-shi, Osaka 541-0045 (JP)**
- **KITAHATA Shun**
  **Osaka-shi, Osaka 541-0045 (JP)**

(74) Representative: **Boult Wade Tennant LLP**
**Salisbury Square House**
**8 Salisbury Square**
**London EC4Y 8AP (GB)**

Remarks:
The complete document including Reference Table(s) and the Sequence Listing(s) can be downloaded from the EPO website

# (54) CATIONIC LIPID

(57) The present invention provides novel cationic lipids having excellent encapsulation and delivery stability of nucleic acid medicines. Provided is a compound represented by Formula (I) or a pharmacologically acceptable salt thereof. In the Formula (I), $R^1$ is a substituted or unsubstituted formula: $-(CH_2)_a-L_1-(CH_2)_b-CH_3$; $R^2$ is a substituted or unsubstituted C5-C20 alkyl group or a substituted or unsubstituted formula: $-(CH_2)_c-L_2-(CH_2)_d-CH_3$; $L^1$ and $L^2$ are each independently -C(=O)O-, -OC(=O)-, or -OC(=O)O-; a, b, c, and d are each independently an integer of at least 1, and the total of a and b and the total of c and d are each an integer of 5 to 25; $R^3$ to $R^7$ are each independently a hydrogen atom, a substituted or unsubstituted $C^1$-$C^6$ alkyl group, or the like; $R^8$, $R^9$, and $R^{10}$ are each a hydrogen atom; the constituent atoms in the Formula (I) may form a ring; Z is -OC(=O)-, -C(=O)O-, -OC(=O)O-, or the like; and X is O or S.

EP 4 289 814 A1

(52) Cooperative Patent Classification (CPC): (Cont.)
**C07D 295/088; C07D 309/08; C07D 471/10;
C07D 471/22; C07D 491/113; C07F 9/59;
C12N 5/10; C12N 15/11; C12N 15/113;
C12N 15/88**

## Description

[TECHNICAL FIELD]

The present invention relates to a novel cationic lipid.

[BACKGROUND ART]

Unlike conventional drugs that target proteins, nucleic acid drugs can introduce and suppress sequence-specific target genes in vivo and in vitro. Since it is possible to promote or suppress the expression of any protein simply by changing the design of the target sequence portion, it is attracting attention as a next-generation drug that can treat not only general diseases but also hereditary diseases and intractable diseases with significant unmet medical needs.

Examples of nucleic acids to be introduced into living bodies as nucleic acid drugs include siRNA (small interfering RNA), mRNA (messenger RNA), miRNA (micro RNA), shRNA (small hairpin RNA or short hairpin RNA), antisense oligonucleotides, immunostimulatory nucleic acids and the like.

Although these nucleic acids are chemically stable, they have problems for therapeutic use that it is easily degraded by in vivo nucleases, and that it is difficult to permeate cell membranes when used alone, so that it has a low uptake efficiency into target cells. In order to overcome these two major problems, research has been conducted for many years on chemical modification of nucleic acids themselves and drug delivery systems (DDS) for delivering nucleic acids into target cells.

Examples of DDS include the use of carriers such as cationic liposomes and polymeric micelles. For example, it is known that encapsulating nucleic acids in microparticles containing cationic lipids protects the nucleic acids from degradation in the blood and allows permeation to lipophilic cell membranes. Patent Document 1 describes the following compound as such cationic lipids. The lipid formulation described in Patent Document 1 is a composition in which a therapeutic nucleic acid is encapsulated in microparticles containing the lipid, and protects the nucleic acid from degradation in serum. The formulation is efficiently delivered systemically and the encapsulated nucleic acid is also efficiently delivered intracellularly.

[Chemical Formula 1]

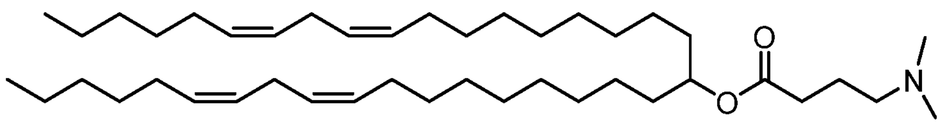

In addition, various compounds have been developed as cationic lipids that are expected to be used in nucleic acid drugs (Patent Documents 2 to 7 and 9, Non-Patent Documents 1 to 3).

Patent Document 2 describes the following compound.

[Chemical Formula 2]

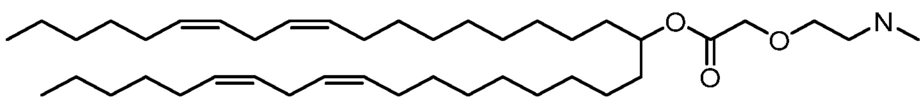

Patent Document 3 describes the following compound.

[Chemical Formula 3]

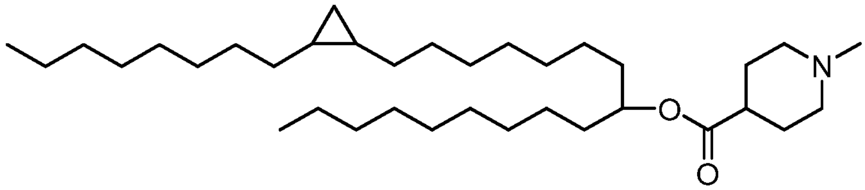

Patent Document 4 and Non-Patent Document 1 describe the following compound ALN-319.

[Chemical Formula 4]

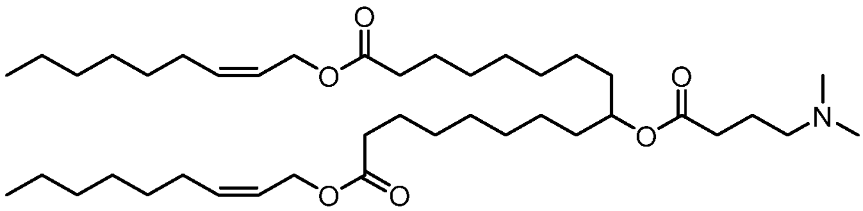

Patent Document 5 describes the following compound YS-119.

[Chemical Formula 5]

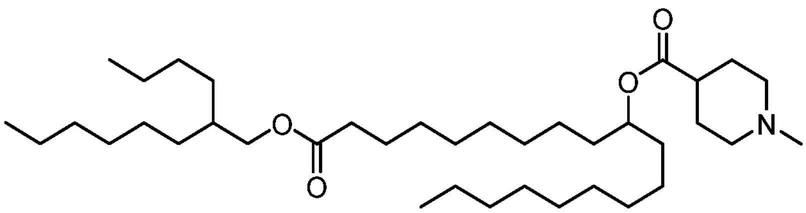

Patent Document 6 describes the following compound.

[Chemical Formula 6]

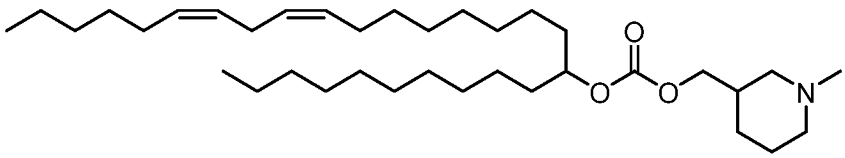

Patent Document 7 describes the following compound.

[Chemical Formula 7]

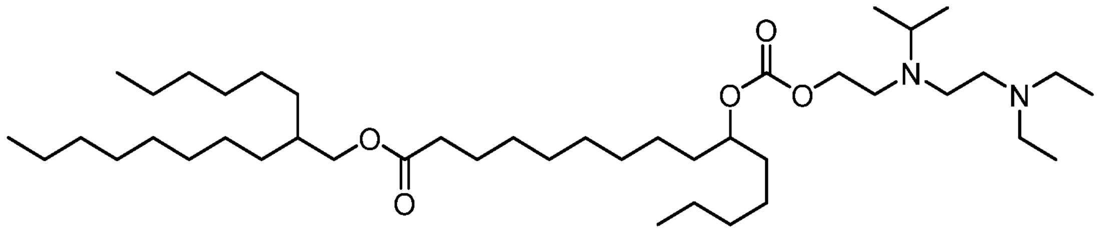

Patent Document 9 describes the following compound.

[Chemical Formula 8]

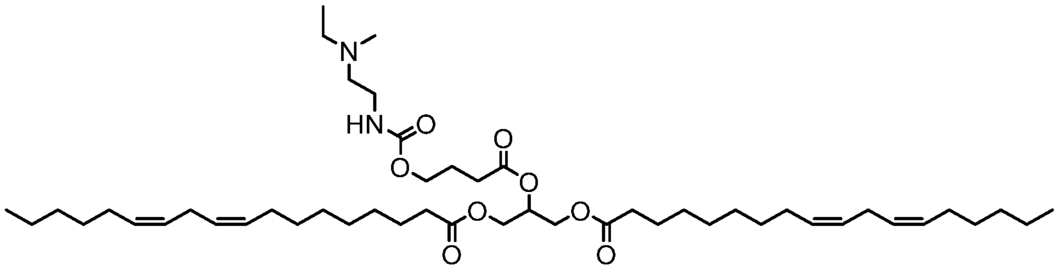

Non-Patent Document 2 describes the following compound.

[Chemical Formula 9]

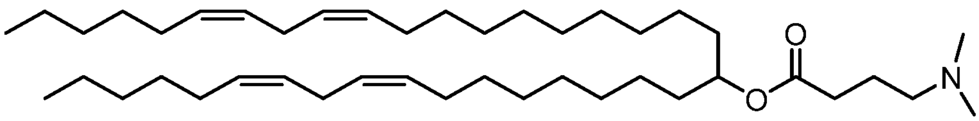

Non-Patent Document 3 describes the following compound.

[Chemical Formula 10]

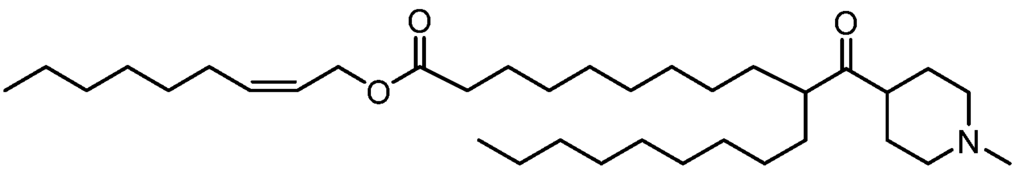

Patent Document 8 describes the following compound as a lubricant.

[Chemical Formula 11]

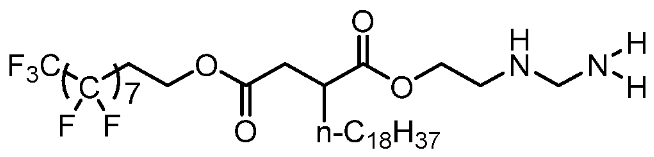

However, the cationic lipid of the present invention is neither described nor suggested in the prior arts.

[PRIOR ART]

[Patent Document]

Patent Document 1: WO2010/144740
Patent Document 2: WO2010/054405
Patent Document 3: WO2015/105131
Patent Document 4: WO2011/153493
Patent Document 5: WO2016/104580
Patent Document 6: WO2015/005253
Patent Document 7: WO2019/235635
Patent Document 8: JP2003/040847
Patent Document 9: WO2020/118041

[Non-patent Document]

Non-Patent Document 1: Molecular Therapy, 2013, vol. 21(8), 1570-1578
Non-Patent Document 2: Angewandte Chemie, 2012, vol.51(34), 8529-33
Non-Patent Document 3: International Journal of Pharmaceutics, 2017, vol. 519, 34-43

[SUMMARY OF THE INVENTION]

[PROBLEMS TO BE SOLVED BY THE INVENTION]

The purpose of the present invention is to provide a novel cationic lipid that forms a lipid particle.

[MEANS FOR SOLVING THE PROBLEMS]

The present inventors have intensively studied, designed and synthesized novel cationic lipids. They have found that a Lipid nanoparticle (LNP) containing the cationic lipid has excellent properties as a pharmaceutical that the LNP can efficiently encapsulate nucleic acids such as single-stranded or double-stranded polynucleotides such as siRNA and mRNA and that the LNP has high stability. They have also found that the nucleic acid contained in the LNP represses the target sequence expression and promotes the target protein expression in multiple cell lines.

The present invention specifically relates to the followings.

(1') A compound represented by Formula (I):

[Chemical Formula 12]

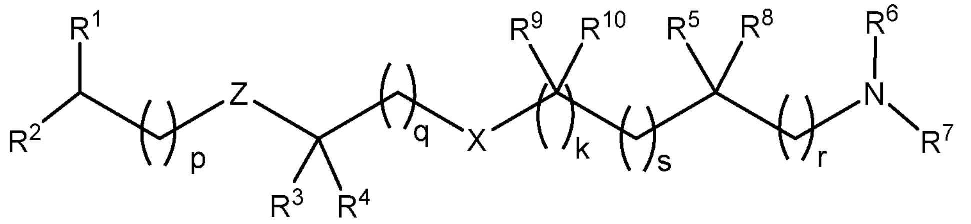

(I)

wherein

$R^1$ is a substituted or unsubstituted formula: $-(CH_2)_a-L_1-(CH_2)_b-CH_3$;
$R^2$ is substituted or unsubstituted $C_5$-$C_{20}$ alkyl or a substituted or unsubstituted formula: $-(CH_2)_c-L_2-(CH_2)_d-CH_3$;
$L_1$ and $L_2$ are each independently -C(=O)O-, -OC(=O)- or -OC(=O)O-;
a and b are each independently an integer of 1 or more, the total of a and b is an integer of 5 to 25;
c and d are each independently an integer of 1 or more, the total of c and d is an integer of 5 to 25;
substituents for the formula: $-(CH_2)_a-L_1-(CH_2)_b-CH_3$ in $R^1$, and $C_5$-$C_{20}$ alkyl and the formula: $-(CH_2)_c-L_2-(CH_2)_d-CH_3$ in $R^2$ are each independently selected from substituent group $\alpha$;
the substituent group $\alpha$ is the group consisting of a halogen atom, oxo, cyano, nitro, sulfanyl, carboxy, $C_1$-$C_{10}$ alkyl, halogenated $C_1$-$C_{10}$ alkyl, $C_2$-$C_{10}$ alkenyl, halogenated $C_2$-$C_{10}$ alkenyl, $C_2$-$C_{10}$ alkynyl, halogenated $C_2$-$C_{10}$ alkynyl, $C_1$-$C_{10}$ alkoxy, halogenated $C_1$-$C_{10}$ alkoxy, $C_1$-$C_{10}$ alkylsulfanyl, halogenated $C_1$-$C_{10}$ alkylsulfanyl, $C_1$-$C_{11}$ alkanoyl, $C_1$-$C_{11}$ alkanoyloxy, $C_1$-$C_{10}$ alkoxy $C_1$-$C_{10}$ alkoxy, $C_1$-$C_{10}$ alkoxycarbonyl, $C_1$-$C_{10}$ alkylcarbamoyl, di($C_1$-$C_{10}$ alkyl)carbamoyl, $C_1$-$C_{10}$ alkoxycarbonyloxy, $C_1$-$C_{11}$ alkanoyl($C_1$-$C_{10}$ alkyl)amino, $C_1$-$C_{10}$ alkoxycarbonylamino and $C_1$-$C_{10}$ alkylcarbamoyloxy;
$R^3$ and $R^4$ are each independently a hydrogen atom or substituted or unsubstituted $C_1$-$C_6$ alkyl;
or $R^3$ and $R^4$ may be taken together to form a substituted or unsubstituted $C_3$-$C_5$ non-aromatic carbocycle;
$R^5$ is a hydrogen atom or substituted or unsubstituted $C_1$-$C_6$ alkyl;
$R^6$ and $R^7$ are each independently a hydrogen atom, substituted or unsubstituted $C_1$-$C_6$ alkyl, substituted or unsubstituted $C_2$-$C_6$ alkenyl, substituted or unsubstituted $C_2$-$C_6$ alkynyl or substituted or unsubstituted $C_3$-$C_7$ non-aromatic carbocyclyl;
$R^8$, $R^9$ and $R^{10}$ are a hydrogen atom;
or $R^3$ and $R^7$ may be taken together with the atom to which each $R^3$ and $R^7$ are attached to form a substituted or unsubstituted non-aromatic heterocycle;
$R^3$ and $R^8$ may be taken together with the atom to which each $R^3$ and $R^8$ are attached to form a substituted or unsubstituted non-aromatic heterocycle;
$R^5$ and $R^6$ may be taken together with the atom to which each $R^5$ and $R^6$ are attached to form a substituted or unsubstituted non-aromatic heterocycle;
$R^6$ and $R^7$ may be taken together with the atom to which each $R^6$ and $R^7$ are attached to form a substituted or unsubstituted non-aromatic heterocycle;
$R^8$ and $R^9$ may be taken together with the atom to which each $R^8$ and $R^9$ are attached to form a substituted or unsubstituted non-aromatic carbocycle or a substituted or unsubstituted non-aromatic heterocycle, when $R^8$ and $R^9$ form the ring, k is 1 or 2;
$R^7$ and $R^8$, when $R^5$ and $R^6$ form a ring, may be taken together with the atom to which each $R^7$ and $R^8$ are attached to form a substituted or unsubstituted non-aromatic heterocycle;
Z is -OC(=O)-, -C(=O)O-, -OC(=O)O-, -C(=O)N(R)-, -N(R)C(=O)-, - OC(=O)N(R)-, -N(R)C(=O)N(R)-, -N(R)C(=O)O-, -C(=S)N(R)-, -N(R)C(=S)-, - C(=O)S-, -SC(=O)-, $-N(R)S(=O)_2-$, $-OS(=O)_2-$, -OP(=O)(-OR)O-, -OP(=S)(-OR)O-, - $OS(=O)_2-O-$, $-S(=O)_2-N(R)-$, -OP(=O)(-NR)O-, -C(=S)O- or -OC(=S)-;
X is O or S;
p, q, s and k are each independently an integer of 0 to 2;
r is an integer of 0 to 5;
R are each independently a hydrogen atom or substituted or unsubstituted $C_1$-$C_6$ alkyl;
substituents for $C_1$ -$C_6$ alkyl in $R^3$ to $R^7$ and R, and $C_2$-$C_6$ alkenyl or $C_2$-$C_6$ alkynyl in $R^6$ and $R^7$ are each independently selected from substituent group $\beta$1;
the substituent group $\beta$1 is the group consisting of a halogen atom, oxo, hydroxy, cyano, sulfanyl, amino, $C_1$-$C_6$ alkoxy, $C_1$-$C_6$ alkylsulfanyl, $C_1$-$C_6$ alkylamino, di$C_1$-$C_6$ alkylamino and $C_1$-$C_7$ alkanoyl;
substituents for a ring formed by $R^3$ and $R^4$, $C_3$-$C_7$ non-aromatic carbocyclyl in $R^6$ and $R^7$, a ring formed by $R^3$ and $R^7$, a ring formed by $R^3$ and $R^8$, a ring formed by $R^5$ and $R^6$, a ring formed by $R^6$ and $R^7$, a ring formed by

$R^7$ and $R^8$ and a ring formed by $R^8$ and $R^9$ are each independently selected from substituent group β2; the substituent group β2 is the group consisting of the substituent group β1, $C_1$-$C_6$ alky and halogenated $C_1$-$C_6$ alkyl,

or a pharmacologically acceptable salt thereof.

(1) A compound represented by Formula (I):

[Chemical Formula 13]

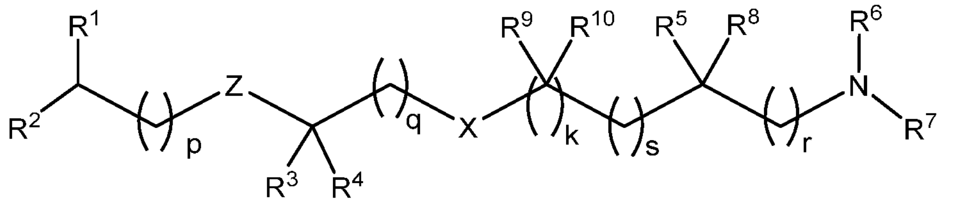

(I)

wherein

$R^1$ is a substituted or unsubstituted formula: $-(CH_2)_a-L_1-(CH_2)_b-CH_3$;
$R^2$ is substituted or unsubstituted $C_5$-$C_{20}$ alkyl or a substituted or unsubstituted formula: $-(CH_2)_c-L_2-(CH_2)_d-CH_3$;
$L_1$ and $L_2$ are each independently -C(=O)O-, -OC(=O)- or -OC(=O)O-;
a and b are each independently an integer of 1 or more, the total of a and b is an integer of 5 to 25;
c and d are each independently an integer of 1 or more, the total of c and d is an integer of 5 to 25;
substituents for the formula: $-(CH_2)_a-L_1-(CH_2)_b-CH_3$ in $R^1$, and $C_5$-$C_{20}$ alkyl and the formula: $-(CH_2)_c-L_2-(CH_2)_d-CH_3$ in $R^2$ are each independently selected from substituent group α;
the substituent group α is the group consisting of a halogen atom, oxo, cyano, nitro, sulfanyl, carboxy, $C_1$-$C_{10}$ alkyl, halogenated $C_1$-$C_{10}$ alkyl, $C_2$-$C_{10}$ alkenyl, halogenated $C_2$-$C_{10}$ alkenyl, $C_2$-$C_{10}$ alkynyl, halogenated $C_2$-$C_{10}$ alkynyl, $C_1$-$C_{10}$ alkoxy, halogenated $C_1$-$C_{10}$ alkoxy, $C_1$-$C_{10}$ alkylsulfanyl, halogenated $C_1$-$C_{10}$ alkylsulfanyl, $C_1$-$C_{11}$ alkanoyl, $C_1$-$C_{11}$ alkanoyloxy, $C_1$-$C_{10}$ alkoxy $C_1$-$C_{10}$ alkoxy, $C_1$-$C_{10}$ alkoxycarbonyl, $C_1$-$C_{10}$ alkylcarbamoyl, di($C_1$-$C_{10}$ alkyl)carbamoyl, $C_1$-$C_{10}$ alkoxycarbonyloxy, $C_1$-$C_{11}$ alkanoyl($C_1$-$C_{10}$ alkyl)amino, $C_1$-$C_{10}$ alkoxycarbonylamino and $C_1$-$C_{10}$ alkylcarbamoyloxy;
$R^3$ and $R^4$ are each independently a hydrogen atom or substituted or unsubstituted $C_1$-$C_6$ alkyl;
or $R^3$ and $R^4$ may be taken together to form a substituted or unsubstituted $C_3$-$C_5$ non-aromatic carbocycle;
$R^5$ is a hydrogen atom or substituted or unsubstituted $C_1$-$C_6$ alkyl;
$R^6$ and $R^7$ are each independently a hydrogen atom, substituted or unsubstituted $C_1$-$C_6$ alkyl, substituted or unsubstituted $C_2$-$C_6$ alkenyl, substituted or unsubstituted $C_2$-$C_6$ alkynyl or substituted or unsubstituted $C_3$-$C_7$ non-aromatic carbocyclyl;
$R^8$, $R^9$ and $R^{10}$ are a hydrogen atom; or
$R^3$ and $R^7$ may be taken together with the atom to which each $R^3$ and $R^7$ are attached to form a substituted or unsubstituted non-aromatic heterocycle;
$R^3$ and $R^8$ may be taken together with the atom to which each $R^3$ and $R^8$ are attached to form a substituted or unsubstituted non-aromatic heterocycle;
$R^5$ and $R^6$ may be taken together with the atom to which each $R^5$ and $R^6$ are attached to form a substituted or unsubstituted non-aromatic heterocycle;
$R^6$ and $R^7$ may be taken together with the atom to which each $R^6$ and $R^7$ are attached to form a substituted or unsubstituted non-aromatic heterocycle;
$R^8$ and $R^9$ may be taken together with the atom to which each $R^8$ and $R^9$ are attached to form a substituted or unsubstituted non-aromatic carbocycle or a substituted or unsubstituted non-aromatic heterocycle, when $R^8$ and $R^9$ form the ring, k is 1 or 2;
$R^7$ and $R^8$, when $R^5$ and $R^6$ form a ring, may be taken together with the atom to which each $R^7$ and $R^8$ are attached to form a substituted or unsubstituted non-aromatic heterocycle;
Z is -OC(=O)-, -C(=O)O-, -OC(=O)O-, -C(=O)N(R)-, -N(R)C(=O)-, - OC(=O)N(R)-, -N(R)C(=O)N(R)-, -N(R)C(=O)O-, -C(=S)N(R)-, -N(R)C(=S)-, - C(=O)S-, -SC(=O)-, -N(R)S(=O)$_2$-, -OS(=O)$_2$-, -OP(=O)(-OR)O-, -OP(=S)(-OR)O-, - OS(=O)$_2$-O-, -S(=O)$_2$-N(R)-, -OP(=O)(-NR)O-, -C(=S)O- or -OC(=S)-;
X is O or S;
p, q, s and k are each independently an integer of 0 to 2;
r is an integer of 0 to 5;

R are each independently a hydrogen atom or substituted or unsubstituted $C_1$-$C_6$ alkyl;
substituents for $C_1$-$C_6$ alkyl in $R^3$ to $R^7$ and R, a ring formed by $R^3$ and $R^4$, $C_2$-$C_6$ alkenyl, $C_2$-$C_6$ alkynyl or $C_3$-$C_7$ non-aromatic carbocyclyl in $R^6$ and $R^7$, a ring formed by $R^3$ and $R^7$, a ring formed by $R^3$ and $R^8$, a ring formed by $R^5$ and $R^6$, a ring formed by $R^6$ and $R^7$, a ring formed by $R^7$ and $R^8$ and a ring formed by $R^8$ and $R^9$ are each independently selected from substituent group $\beta$;
the substituent group $\beta$ is the group consisting of a halogen atom, oxo, hydroxy, cyano, sulfanyl, amino, $C_1$-$C_6$ alky, halogenated $C_1$-$C_6$ alkyl, $C_1$-$C_6$ alkoxy, $C_1$-$C_6$ alkylsulfanyl, $C_1$-$C_6$ alkylamino, di$C_1$-$C_6$ alkylamino and $C_1$-$C_7$ alkanoyl,

or a pharmacologically acceptable salt thereof.

(2) The compound according to above (1) or (1'), wherein $L_1$ is -C(=O)O- or - OC(=O)-, or a pharmacologically acceptable salt thereof.

(3) The compound according to above (1), (2) or (1'), wherein a and b are each independently an integer of 5 to 10, or a pharmacologically acceptable salt thereof.

(4) The compound according to any one of above (1) to (3) and (1'), wherein $R^1$ is a formula: $-(CH_2)_a-L_1-(CH_2)_b-CH_3$, substituted with $C_1$-$C_{10}$ alkyl, or a pharmacologically acceptable salt thereof.

(5) The compound according to any one of above (1) to (4) and (1'), wherein $R^2$ is a substituted or unsubstituted formula: $-(CH_2)_c-L_2-(CH_2)_d-CH_3$, or a pharmacologically acceptable salt thereof.

(6) The compound according to above (5), wherein $R^2$ is a formula: $-(CH_2)_c-L_2-(CH_2)_d-CH_3$, substituted with $C_1$-$C_{10}$ alkyl, or a pharmacologically acceptable salt thereof.

(7) The compound according to above (5) or (6), wherein $L_2$ is -C(=O)O- or - OC(=O)-, or a pharmacologically acceptable salt thereof.

(8) The compound according to any one of above (5) to (7), wherein c and d are each independently an integer of 5 to 10, or a pharmacologically acceptable salt thereof.

(9) The compound according to any one of above (1) to (4) and (1'), wherein $R^2$ is substituted or unsubstituted $C_5$-$C_{20}$ alkyl, or a pharmacologically acceptable salt thereof.

(10') The compound according to any one of above (1) to (9) and (1'), wherein

Z is -OC(=O)-, -C(=O)O-, -OC(=O)O-, -C(=O)N(R)-, -N(R)C(=O)O- or - N(R)C(=O)-;
R are each independently a hydrogen atom or unsubstituted $C_1$ alkyl; or a pharmacologically acceptable salt thereof.

(10) The compound according to any one of above (1) to (9), (1') and (10'), wherein Z is -OC(=O)-, -C(=O)O- or -OC(=O)O-, or a pharmacologically acceptable salt thereof.

(11) The compound according to any one of above (1) to (10), (1') and (10'), wherein $R^6$ and $R^7$ are each independently substituted or unsubstituted $C_1$-$C_6$ alkyl, or a pharmacologically acceptable salt thereof.

(12) The compound according to any one of above (1) to (10), (1') and (10'), wherein $R^5$ and $R^6$ are taken together with the atom to which each $R^5$ and $R^6$ are attached to form a substituted or unsubstituted non-aromatic heterocycle, or a pharmacologically acceptable salt thereof.

(13) The compound according to any one of above (1) to (10), (1') and (10'), wherein $R^6$ and $R^7$ are taken together with the atom to which each $R^6$ and $R^7$ are attached to form a substituted or unsubstituted non-aromatic heterocycle, or a pharmacologically acceptable salt thereof.

(14) The compound according to any one of above (1) to (10), (1') and (10'), wherein

$R^5$ and $R^6$ are taken together with the atom to which each $R^5$ and $R^6$ are attached to form a substituted or unsubstituted non-aromatic heterocycle;
and $R^7$ and $R^8$ are taken together with the atom to which each $R^7$ and $R^8$ are attached to form a substituted or unsubstituted non-aromatic heterocycle;

or a pharmacologically acceptable salt thereof.

(16') The compound according to any one of above (1) to (10), (1') and (10'), wherein r is an integer of 0 to 3, or a pharmacologically acceptable salt thereof.

(17') The compound according to any one of above (1) to (10), (1'), (10') and (16'), selected from the group consisting of compounds I-6, I-8, I-37, I-39, I-55, I-56, I-58, I-60, I-65, I-66 and I-69, or a pharmacologically acceptable salt thereof.

(15) A pharmaceutical composition comprising the compound according to any one of above (1) to (14), (1'), (10'), (16') and (17') or a pharmaceutically acceptable salt thereof and a nucleic acid.

(16) The pharmaceutical composition according to above (15), wherein the nucleic acid is siRNA or mRNA.

[EFFECT OF THE INVENTION]

A LNP containing the cationic lipid of the present invention can efficiently encapsulate nucleic acids and stably retain nucleic acids within the particle. In addition, the nucleic acid contained in the LNP can act on the target cells. Therefore, the cationic lipid of the present invention can be used as a lipid for nucleic acid delivery into cells.

[EMBODIMENTS FOR CARRYING OUT THE INVENTION]

Hereinafter, the meaning of each term used in the present description will be described. Unless particularly stated otherwise, each term is used in the same sense, either alone or in combination with other terms.
Hereinafter, the present invention will be described while showing exemplary embodiments. Throughout the present description, it should be understood that, unless particularly stated otherwise, an expression of a singular form also includes the concept of a plural form thereof. Therefore, it should be understood that, unless particularly stated otherwise, an article for a singular form (for example, in the case of English, "a", "an", "the", or the like) also includes the concept of a plural form thereof.
Furthermore, it should be understood that, unless particularly stated otherwise, the terms used in the present description are used in the meanings normally used in the above-described art. Accordingly, unless otherwise defined, all terminologies and scientific and technical terms used in the present description have the same meanings as commonly understood by those having ordinary skill in the art to which the present invention belongs. In a case of contradiction, priority is given to the present description (including definitions).
"Alkyl" includes a C1 to C20 linear or branched hydrocarbon group. Examples thereof include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl and the like.
"$C_5$-$C_{20}$ alkyl" in $R^2$ means a C5 to C20 linear hydrocarbon group. Linear $C_5$-$C_{15}$ alkyl is preferable. Linear $C_5$-$C_{10}$ alkyl is more preferable.
"$C_1$-$C_6$ alkyl" in $R^3$ to $R^7$ and R means a C1 to C6 linear or branched hydrocarbon group. Linear or branched $C_1$-$C_4$ alkyl is preferable. Linear or branched $C_1$-$C_3$ alkyl is more preferable. Methyl is further preferable.
"$C_1$-$C_{10}$ alkyl" in the substituent group $\alpha$ means a C1 to C10 linear or branched hydrocarbon group. Linear or branched $C_2$-$C_8$ alkyl is preferable.
"$C_1$-$C_6$ alkyl" in the substituent group $\beta$ and the substituent group $\beta$2 means a C1 to C6 linear or branched hydrocarbon group. Linear or branched $C_1$-$C_3$ alkyl is preferable. Methyl is more preferable.
"A halogen atom" includes a fluorine atom, a chlorine atom, a bromine atom, and an iodine atom. A fluorine atom and a chlorine atom are particularly preferable.
"Halogenated alkyl" includes a C1 to C20 linear or branched alkyl substituted with one or more halogen(s).
"Halogenated $C_1$-$C_{10}$ alkyl" in the substituent group $\alpha$ means above "$C_1$-$C_{10}$ alkyl" substituted with one or more halogen(s). Linear or branched $C_2$-$C_8$ alkyl substituted with one to ten halogen(s) is preferable.
"Halogenated $C_1$-$C_6$ alkyl" in the substituent group $\beta$ and the substituent group $\beta$2 means above "$C_1$-$C_6$ alkyl" substituted with one or more halogen(s). Linear or branched $C_1$-$C_3$ alkyl substituted with one to four halogen(s) is preferable.
"Alkenyl" includes a C2 to C20 linear or branched hydrocarbon group having one or more double bond(s) at any position(s). Examples thereof include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, and pentadecenyl and the like.
"$C_2$-$C_{10}$ alkenyl" in the substituent group $\alpha$ means a C2 to C10 linear or branched alkenyl. Linear or branched $C_2$-$C_{10}$ alkenyl having one to four double bond(s) is preferable.
"$C_2$-$C_6$ alkenyl" in $R^6$ and $R^7$ means a C2 to C6 linear or branched alkenyl. Linear or branched $C_2$-$C_4$ alkenyl having one or two double bond(s) is preferable.
"Halogenated alkenyl" includes a C2 to C20 linear or branched alkenyl substituted with one or more halogen(s).
"Halogenated $C_2$-$C_{10}$ alkenyl" in the substituent group $\alpha$ means above "$C_2$-$C_{10}$ alkenyl" substituted with one or more halogen(s). Linear or branched $C_2$-$C_8$ alkenyl substituted with one to ten halogen(s) is preferable.
"Alkynyl" includes a C2 to C20 linear or branched hydrocarbon group having one or more triple bond(s) at any position(s). Furthermore, it may have double bond(s) at any position(s). Examples thereof include ethynyl, propynyl, butynyl, pentynyl, hexynyl, heptynyl, octynyl, nonynyl, and decynyl and the like.
"$C_2$-$C_{10}$ alkynyl" in the substituent group $\alpha$ means a C2 to C10 linear or branched alkynyl. Linear or branched $C_2$-$C_{10}$ alkynyl having one or two triple bond(s) is preferable.
"$C_2$-$C_6$ alkynyl" in $R^6$ and $R^7$ means a C2 to C6 linear or branched alkynyl. Linear or branched $C_2$-$C_4$ alkynyl having one triple bond is preferable.
"Halogenated alkynyl" includes a C2 to C20 linear or branched alkynyl substituted with one or more halogen(s).

"Halogenated $C_2$-$C_{10}$ alkynyl" in the substituent group $\alpha$ means above "$C_2$-$C_{10}$ alkynyl" substituted with one or more halogen(s). Linear or branched $C_2$-$C_8$ alkynyl substituted with one to ten halogen(s) is preferable.

"Alkoxy" means alkyloxy.

"Halogenated alkoxy" includes a C1 to C20 linear or branched alkoxy substituted with one or more halogen(s).

"Halogenated $C_1$-$C_{10}$ alkoxy" in the substituent group $\alpha$ means a C1 to C10 linear or branched alkoxy substituted with one or more halogen(s). Linear or branched $C_2$-$C_8$ alkoxy substituted with one to ten halogen(s) is preferable.

"$C_1$-$C_{11}$ alkanoyl" in the substituent group $\alpha$ includes formyl, or carbonyl substituted with $C_1$-$C_{10}$ alkyl, halogenated $C_1$-$C_{10}$ alkyl, $C_2$-$C_{10}$ alkenyl, halogenated $C_2$-$C_{10}$ alkenyl, $C_2$-$C_{10}$ alkynyl or halogenated $C_2$-$C_{10}$ alkynyl.

"$C_1$-$C_7$ alkanoyl" in the substituent group $\beta$ and the substituent group $\beta$1 includes formyl, or carbonyl substituted with $C_1$-$C_6$ alkyl or halogenated $C_1$-$C_6$ alkyl.

"Alkoxyalkoxy" includes a C1 to C20 linear or branched alkoxy substituted with one or more alkoxy.

"$C_1$-$C_{10}$ alkoxy $C_1$-$C_{10}$ alkoxy" in the substituent group $\alpha$ means a C1 to C10 linear or branched alkoxy substituted with one or more $C_1$-$C_{10}$ alkoxy. Linear or branched $C_2$-$C_8$ alkoxy substituted with one to ten $C_1$-$C_{10}$ alkoxy is preferable.

"Non-aromatic carbocycle" means a cyclic saturated non-aromatic hydrocarbon ring or a cyclic unsaturated non-aromatic hydrocarbon ring, which is monocyclic or polycyclic having two or more rings. A 3- to 12-membered cyclic saturated non-aromatic hydrocarbon ring or cyclic unsaturated non-aromatic hydrocarbon ring is preferable.

In addition, "a cyclic saturated non-aromatic hydrocarbon ring" includes a ring having a bridge or a ring to form a spiro ring as follows.

[Chemical Formula 14]

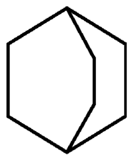

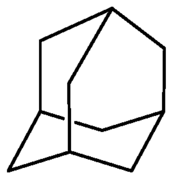

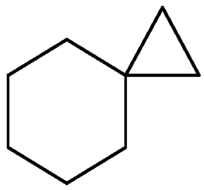

"A cyclic unsaturated non-aromatic hydrocarbon ring" means a ring having one or several (preferably one to three) double bond(s) in a cyclic saturated non-aromatic hydrocarbon ring and includes a ring having a bridge or forming a spiro ring.

Examples of non-aromatic carbocycle which is monocyclic include cyclopropane, cyclobutane, cyclopentane, cyclohexane, cycloheptane, cyclooctane, cyclononane, cyclodecane, cyclopropene, cyclobutene, cyclopentene, cyclohexene, cycloheptene and cyclohexadiene and the like.

"Non-aromatic carbocyclyl" means a group derived from above "Non-aromatic carbocycle".

"$C_3$-$C_5$ non-aromatic carbocycle" formed by $R^3$ and $R^4$ means a cyclic saturated $C_3$-$C_5$ hydrocarbon ring or a cyclic unsaturated $C_3$-$C_5$ non-aromatic hydrocarbon ring. Examples of a cyclic saturated $C_3$-$C_5$ hydrocarbon ring include cyclopropane, cyclobutane and cyclopentane and the like. A cyclic unsaturated $C_3$-$C_5$ non-aromatic hydrocarbon ring means a cyclic saturated $C_3$-$C_5$ hydrocarbon ring containing one double bond, and examples thereof include cyclopentene and the like.

"$C_3$-$C_7$ non-aromatic carbocyclyl" in $R^6$ or $R^7$ means a cyclic saturated $C_3$-$C_7$ hydrocarbon group or a cyclic unsaturated $C_3$-$C_7$ non-aromatic hydrocarbon group. Examples of a cyclic saturated $C_3$-$C_7$ hydrocarbon group include cyclopropyl, cyclobutyl, cyclopentyl, cyclohexyl and cycloheptyl and the like. A cyclic unsaturated $C_3$-$C_7$ non-aromatic hydrocarbon group means a cyclic saturated $C_3$-$C_7$ hydrocarbon group containing one double bond, and examples thereof include cyclopentenyl and cyclohexenyl and the like.

"Non-aromatic heterocycle" means a cyclic saturated non-aromatic heterocyclic ring or a cyclic unsaturated non-aromatic heterocyclic ring, which is monocyclic or polycyclic having two or more rings, containing one to three and identical or different heteroatoms selected independently from O, S and N(R') in the ring. A 3- to 12-membered non-aromatic heterocycle is preferable. Herein, R' is a hydrogen atom or substituted or unsubstituted $C_1$-$C_6$ alkyl. The substituent is selected from the substituent group $\beta$1.

In addition, "non-aromatic heterocycle" includes a ring having a bridge or a ring to form a spiro ring as follows.

[Chemical Formula 15]

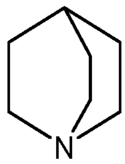
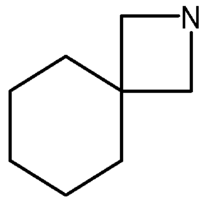
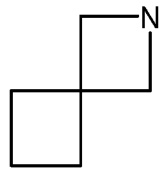

Examples of non-aromatic heterocycle which is monocyclic include thiirane, oxirane, aziridine, oxetane, azetidine, oxathiolane, thiazolidine, pyrrolidine, pyrroline, imidazolidine, imidazoline, pyrazolidine, pyrazolone, tetrahydrofuran and dihydrothiazolyl and the like.

"Non-aromatic carbocyclyl" means a group derived from above "Non-aromatic heterocycle".

"Non-aromatic heterocycle" formed by $R^3$ and $R^7$ may or may not contain a heteroatom other than the nitrogen atom to which $R^7$ is attached and X.

"Non-aromatic heterocycle" formed by $R^3$ and $R^8$ may or may not contain a heteroatom other than X.

"Non-aromatic heterocycle" formed by $R^5$ and $R^6$ may or may not contain a heteroatom other than the nitrogen atom to which $R^6$ is attached.

"Non-aromatic heterocycle" formed by $R^6$ and $R^7$ may or may not contain a heteroatom other than the nitrogen atom to which $R^6$ and $R^7$ is attached.

"Non-aromatic heterocycle" formed by $R^7$ and $R^8$ may or may not contain a heteroatom other than the nitrogen atom to which $R^7$ is attached.

Two $C_1$-$C_{10}$ alkyl in the "di($C_1$-$C_{10}$ alkyl)carbamoyl" may be identical or different.

Two $C_1$-$C_6$ alkyl in the "di$C_1$-$C_6$ alkylamino" may be identical or different.

In the "substituted or unsubstituted $C_5$-$C_{20}$ alkyl" for R2, any substitutable hydrogen atom may be substituted with one or more substituent(s) selected from the substituent group α. One to ten any hydrogen atoms may be substituted, and one to four any hydrogen atoms may be substituted.

In the "substituted or unsubstituted $C_1$-$C_6$ alkyl", "substituted or unsubstituted $C_2$-$C_6$ alkenyl" or "substituted or unsubstituted $C_2$-$C_6$ alkynyl", any substitutable hydrogen atom may be substituted with one to four substituent(s) selected from the substituent group β1. In the "substituted or unsubstituted monocyclic $C_3$-$C_5$ non-aromatic carbocycle", "substituted or unsubstituted $C_3$-$C_7$ non-aromatic carbocyclyl" or "substituted or unsubstituted non-aromatic heterocycle", any substitutable hydrogen atom may be substituted with one to four substituent(s) selected from the substituent group β2.

Here, the present invention is explained in detail.

The compound of the present invention is cationic lipid.

The cationic lipid of the present invention is a compound represented by Formula (I):

[Chemical Formula 16]

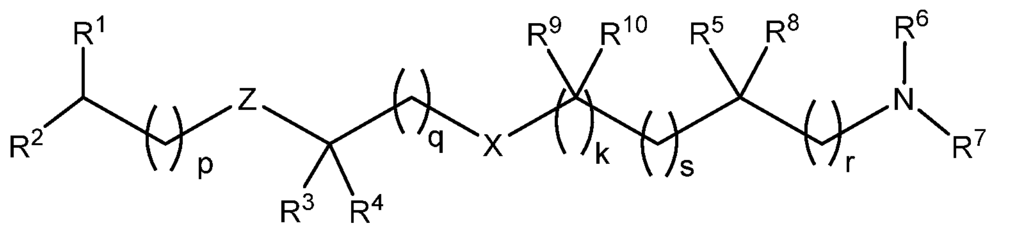

(I)

wherein

$R^1$ is a substituted or unsubstituted formula: $-(CH_2)_a-L_1-(CH_2)_b-CH_3$;
$R^2$ is substituted or unsubstituted $C_5$-$C_{20}$ alkyl or a substituted or unsubstituted formula: $-(CH_2)_c-L_2-(CH_2)_d-CH_3$;
$L_1$ and $L_2$ are each independently -C(=O)O-, -OC(=O)- or -OC(=O)O-;
a and b are each independently an integer of 1 or more, the total of a and b is an integer of 5 to 25;
c and d are each independently an integer of 1 or more, the total of c and d is an integer of 5 to 25;
substituents for the formula: $-(CH_2)_a-L_1-(CH_2)_b-CH_3$ in $R^1$, and $C_5$-$C_{20}$ alkyl and the formula: $-(CH_2)_c-L_2-(CH_2)_d-CH_3$ in $R^2$ are each independently selected from substituent group α;
the substituent group α is the group consisting of a halogen atom, oxo, cyano, nitro, sulfanyl, carboxy, $C_1$-$C_{10}$ alkyl, halogenated $C_1$-$C_{10}$ alkyl, $C_2$-$C_{10}$ alkenyl, halogenated $C_2$-$C_{10}$ alkenyl, $C_2$-$C_{10}$ alkynyl, halogenated $C_2$-$C_{10}$ alkynyl,

$C_1$-$C_{10}$ alkoxy, halogenated $C_1$-$C_{10}$ alkoxy, $C_1$-$C_{10}$ alkylsulfanyl, halogenated $C_1$-$C_{10}$ alkylsulfanyl, $C_1$-$C_{11}$ alkanoyl, $C_1$-$C_{11}$ alkanoyloxy, $C_1$-$C_{10}$ alkoxy $C_1$-$C_{10}$ alkoxy, $C_1$-$C_{10}$ alkoxycarbonyl, $C_1$-$C_{10}$ alkylcarbamoyl, di($C_1$-$C_{10}$ alkyl)carbamoyl, $C_1$-$C_{10}$ alkoxycarbonyloxy, $C_1$-$C_{11}$ alkanoyl($C_1$-$C_{10}$ alkyl)amino, $C_1$-$C_{10}$ alkoxycarbonylamino and $C_1$-$C_{10}$ alkylcarbamoyloxy;

$R^3$ and $R^4$ are each independently a hydrogen atom or substituted or unsubstituted $C_1$-$C_6$ alkyl;

or $R^3$ and $R^4$ may be taken together to form a substituted or unsubstituted $C_3$-$C_5$ non-aromatic carbocycle;

$R^5$ is a hydrogen atom or substituted or unsubstituted $C_1$-$C_6$ alkyl;

$R^6$ and $R^7$ are each independently a hydrogen atom, substituted or unsubstituted $C_1$-$C_6$ alkyl, substituted or unsubstituted $C_2$-$C_6$ alkenyl, substituted or unsubstituted $C_2$-$C_6$ alkynyl or substituted or unsubstituted $C_3$-$C_7$ non-aromatic carbocyclyl;

$R^8$, $R^9$ and $R^{10}$ are a hydrogen atom;

or $R^3$ and $R^7$ may be taken together with the atom to which each $R^3$ and $R^7$ are attached to form a substituted or unsubstituted non-aromatic heterocycle;

$R^3$ and $R^8$ may be taken together with the atom to which each $R^3$ and $R^8$ are attached to form a substituted or unsubstituted non-aromatic heterocycle;

$R^5$ and $R^6$ may be taken together with the atom to which each $R^5$ and $R^6$ are attached to form a substituted or unsubstituted non-aromatic heterocycle;

$R^6$ and $R^7$ may be taken together with the atom to which each $R^6$ and $R^7$ are attached to form a substituted or unsubstituted non-aromatic heterocycle;

$R^8$ and $R^9$ may be taken together with the atom to which each $R^8$ and $R^9$ are attached to form a substituted or unsubstituted non-aromatic carbocycle or a substituted or unsubstituted non-aromatic heterocycle, when $R^8$ and $R^9$ form the ring, k is 1 or 2;

$R^7$ and $R^8$, when $R^5$ and $R^6$ form a ring, may be taken together with the atom to which each $R^7$ and $R^8$ are attached to form a substituted or unsubstituted non-aromatic heterocycle;

Z is -OC(=O)-, -C(=O)O-, -OC(=O)O-, -C(=O)N(R)-, -N(R)C(=O)-, - OC(=O)N(R)-, -N(R)C(=O)N(R)-, -N(R)C(=O)O-, -C(=S)N(R)-, -N(R)C(=S)-, - C(=O)S-, -SC(=O)-, -N(R)S$(=O)_2$-, -OS$(=O)_2$-, -OP(=O)(-OR)O-, -OP(=S)(-OR)O-, - OS$(=O)_2$-O-, -S$(=O)_2$-N(R)-, -OP(=O)(-NR)O-, -C(=S)O- or -OC(=S)-;

X is O or S;

p, q, s and k are each independently an integer of 0 to 2;

r is an integer of 0 to 5;

R are each independently a hydrogen atom or substituted or unsubstituted $C_1$-$C_6$ alkyl;

substituents for $C_1$-$C_6$ alkyl in $R^3$ to $R^7$ and R, and $C_2$-$C_6$ alkenyl or $C_2$-$C_6$ alkynyl in $R^6$ and $R^7$ are each independently selected from substituent group $\beta$1;

the substituent group $\beta$1 is the group consisting of a halogen atom, oxo, hydroxy, cyano, sulfanyl, amino, $C_1$-$C_6$ alkoxy, $C_1$-$C_6$ alkylsulfanyl, $C_1$-$C_6$ alkylamino, di$C_1$-$C_6$ alkylamino and $C_1$-$C_7$ alkanoyl;

substituents for a ring formed by $R^3$ and $R^4$, $C_3$-$C_7$ non-aromatic carbocyclyl in $R^6$ and $R^7$, a ring formed by $R^3$ and $R^7$, a ring formed by $R^3$ and $R^8$, a ring formed by $R^5$ and $R^6$, a ring formed by $R^6$ and $R^7$, a ring formed by $R^7$ and $R^8$ and a ring formed by $R^8$ and $R^9$ are each independently selected from substituent group $\beta$2;

the substituent group $\beta$2 is the group consisting of the substituent group $\beta$1, $C_1$-$C_6$ alky and halogenated $C_1$-$C_6$ alkyl,

or a pharmacologically acceptable salt thereof.

The features of the cationic lipid of the present invention include having X (wherein X is O or S) and a substituted or unsubstituted formula: - $(CH_2)_a$-$L_1$-$(CH_2)_b$-$CH_3$ as $R^1$ (wherein the total of a and b is an integer of 5 to 25 and $L_1$ is -C(=O)O-, -OC(=O)- or -OC(=O)O-) in the above compound represented by Formula (I).

Preferred embodiments of the cationic lipid of the present invention are explained below.

$R^1$ is a substituted or unsubstituted formula: -$(CH_2)_a$-$L_1$-$(CH_2)_b$-$CH_3$. Any one or more hydrogen atoms may be substituted with a substituent selected from the substituent group $\alpha$. One to ten any hydrogen atoms may be substituted, and one to four any hydrogen atoms may be substituted.

$L_1$ is preferably -C(=O)O- or -O-C(=O)-, more preferably -C(=O)O-.

a and b are preferably each independently an integer of 3 to 15, more preferably each independently an integer of 5 to 10, further preferably each independently an integer of 6 to 8.

The substituent selected from the substituent group $\alpha$ for the formula - $(CH_2)_a$-$L_1$-$(CH_2)_b$-$CH_3$ in $R^1$ is preferably a halogen atom, oxo, cyano, $C_1$-$C_{10}$ alkyl, halogenated $C_1$-$C_{10}$ alkyl, $C_1$-$C_{11}$ alkanoyl or $C_1$-$C_{11}$ alkanoyloxy, more preferably a halogen atom, $C_1$-$C_{10}$ alkyl, halogenated $C_1$-$C_{10}$ alkyl, $C_1$-$C_{11}$ alkanoyl or $C_1$-$C_{11}$ alkanoyloxy, further preferably $C_1$-$C_{10}$ alkyl or $C_1$-$C_{11}$ alkanoyloxy.

$R^1$ is preferably a substituted or unsubstituted formula: -$(CH_2)_a$-C(=O)O-$(CH_2)_b$-$CH_3$ or a substituted or unsubstituted formula: -$(CH_2)_a$-O-C(=O)-$(CH_2)_b$-$CH_3$, the total of a and b is an integer of 5 to 25, a and b are each independently an integer of 3 to 15, more preferably a substituted or unsubstituted formula: -$(CH_2)_a$-C(=O)O-$(CH_2)_b$-$CH_3$, a and b are

each independently an integer of 5 to 10, further preferably a formula: $-(CH_2)_a-C(=O)O-(CH_2)_b-CH_3$ substituted with $C_1$-$C_{10}$ alkyl, a and b are each independently an integer of 6 to 8.

$R^2$ is preferably a substituted or unsubstituted formula: $-(CH_2)_c-L_2-(CH_2)_d-CH_3$. Any one or more hydrogen atoms may be substituted with a substituent selected from the substituent group $\alpha$. One to ten any hydrogen atoms may be substituted, and one to four any hydrogen atoms may be substituted.

$L_2$ is preferably -C(=O)O- or -O-C(=O)-, more preferably -C(=O)O-.
c and d are preferably each independently an integer of 3 to 15, more preferably each independently an integer of 5 to 10, further preferably each independently an integer of 6 to 8.

The substituent for the formula: $-(CH_2)_c-L_2-(CH_2)_d-CH_3$ is selected from the substituent group $\alpha$, preferably a halogen atom, oxo, cyano, $C_1$-$C_{10}$ alkyl, halogenated $C_1$-$C_{10}$ alkyl, $C_1$-$C_{11}$ alkanoyl or $C_1$-$C_{11}$ alkanoyloxy, more preferably a halogen atom, $C_1$-$C_{10}$ alkyl, halogenated $C_1$-$C_{10}$ alkyl, $C_1$-$C_{11}$ alkanoyl or $C_1$-$C_{11}$ alkanoyloxy, further preferably $C_1$-$C_{10}$ alkyl or $C_1$-$C_{11}$ alkanoyloxy.

$R^2$ is preferably a substituted or unsubstituted formula: $-(CH_2)_c-C(=O)O-(CH_2)_d-CH_3$ or a substituted or unsubstituted formula: $-(CH_2)_c-OC(=O)-(CH_2)_d-CH_3$, the total of c and d is an integer of 5 to 25, c and d are each independently an integer of 3 to 15, more preferably a substituted or unsubstituted formula: $-(CH_2)_c-C(=O)O-(CH_2)_d-CH_3$, c and d are each independently an integer of 5 to 10, further preferably a formula: $-(CH_2)_c-C(=O)O-(CH_2)_d-CH_3$ substituted with $C_1$-$C_{10}$ alkyl, c and d are each independently an integer of 6 to 8.

As another embodiment, $R^2$ is also preferably substituted or unsubstituted $C_5$-$C_{20}$ alkyl, more preferably substituted or unsubstituted $C_5$-$C_{10}$ alkyl, further preferably $C_5$-$C_{10}$ alkyl.

The substituent selected from the substituent group $\alpha$ for $C_5$-$C_{20}$ alkyl is preferably a halogen atom, oxo, cyano, $C_1$-$C_{10}$ alkyl, halogenated $C_1$-$C_{10}$ alkyl, $C_1$-$C_{11}$ alkanoyl or $C_1$-$C_{11}$ alkanoyloxy, more preferably a halogen atom, $C_1$-$C_{10}$ alkyl, halogenated $C_1$-$C_{10}$ alkyl, $C_1$-$C_{11}$ alkanoyl or $C_1$-$C_{11}$ alkanoyloxy, further preferably $C_1$-$C_{10}$ alkyl or $C_1$-$C_{11}$ alkanoyloxy.

The substituent selected from the substituent group $\beta$ or the substituent group $\beta 1$ for $C_1$-$C_6$ alkyl in $R^3$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

$R^3$ is preferably a hydrogen atom or substituted or unsubstituted $C_1$-$C_6$ alkyl, more preferably a hydrogen atom or $C_1$-$C_6$ alkyl, further preferably a hydrogen atom or $C_1$-$C_3$ alkyl.

The substituent selected from the substituent group $\beta$ or the substituent group $\beta 1$ for $C_1$-$C_6$ alkyl in $R^4$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

$R^4$ is preferably a hydrogen atom or substituted or unsubstituted $C_1$-$C_6$ alkyl, more preferably a hydrogen atom or $C_1$-$C_6$ alkyl, further preferably a hydrogen atom or $C_1$-$C_3$ alkyl.

$R^3$ and $R^4$ may be taken together to form a substituted or unsubstituted $C_3$-$C_5$ non-aromatic carbocycle.

The substituent selected from the substituent group $\beta$ or the substituent group $\beta 2$ for the ring formed by $R^3$ and $R^4$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

A $C_3$-$C_5$ non-aromatic carbocycle is preferably a $C_3$-$C_5$ monocyclic saturated non-aromatic hydrocarbon ring or a $C_3$-$C_5$ monocyclic unsaturated non-aromatic hydrocarbon ring, more preferably cyclopropane, cyclobutene or cyclopentane, specifically as follows,

[Chemical Formula 17]

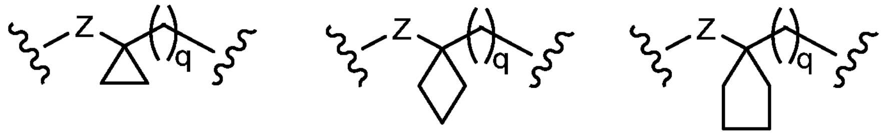

further preferably cyclopropane or cyclopentane.

The substituent selected from the substituent group $\beta$ or the substituent group $\beta 1$ for $C_1$-$C_6$ alkyl in $R^5$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

$R^5$ is preferably a hydrogen atom or substituted or unsubstituted $C_1$-$C_3$ alkyl, more preferably a hydrogen atom, or substituted with a halogen atom, oxo or hydroxy or unsubstituted $C_1$-$C_6$ alkyl, further preferably a hydrogen atom.

The substituent selected from the substituent group $\beta$ or the substituent group $\beta 1$ for $C_1$-$C_6$ alkyl, $C_2$-$C_6$ alkenyl or $C_2$-$C_6$ alkynyl in $R^6$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The substituent selected from the substituent group β or the substituent group β2 for $C_3$-$C_7$ non-aromatic carbocyclyl in $R^6$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

$R^6$ is preferably a hydrogen atom, substituted or unsubstituted $C_1$-$C_6$ alkyl or substituted or unsubstituted $C_2$-$C_6$ alkenyl, more preferably a hydrogen atom or substituted or unsubstituted $C_1$-$C_6$ alkyl, further preferably a hydrogen atom or $C_1$-$C_6$ alkyl.

The substituent selected from the substituent group β or the substituent group β1 for $C_1$-$C_6$ alkyl, $C_2$-$C_6$ alkenyl or $C_2$-$C_6$ alkynyl in $R^7$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The substituent selected from the substituent group β or the substituent group β2 for $C_3$-$C_7$ non-aromatic carbocyclyl in $R^7$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

$R^7$ is preferably a hydrogen atom, substituted or unsubstituted $C_1$-$C_6$ alkyl or substituted or unsubstituted $C_2$-$C_6$ alkenyl, more preferably a hydrogen atom or substituted or unsubstituted $C_1$-$C_6$ alkyl, further preferably a hydrogen atom or $C_1$-$C_6$ alkyl.

The substituent selected from the substituent group β or the substituent group β2 for the ring formed by $R^3$ and $R^7$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The ring formed by $R^3$ and $R^7$ is preferably a substituted or unsubstituted 3- to 7-membered non-aromatic heterocycle containing two to four heteroatoms in the ring, more preferably a substituted or unsubstituted 5-to 7-membered monocyclic non-aromatic heterocycle containing two heteroatoms in the ring.

For example,

[Chemical Formula 18]

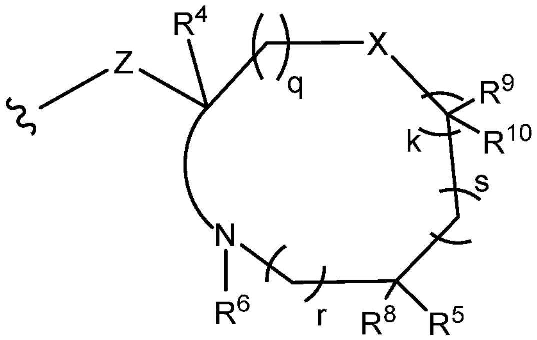

and the like.

The substituent selected from the substituent group β or the substituent group β2 for the ring formed by $R^3$ and $R^8$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The ring formed by $R^3$ and $R^8$ is preferably a substituted or unsubstituted 3- to 7-membered non-aromatic heterocycle containing one to three heteroatom(s) in the ring, more preferably a substituted or unsubstituted 3- to 6-membered monocyclic non-aromatic heterocycle containing one to three heteroatom(s) in the ring, further preferably a substituted or unsubstituted 6-membered monocyclic non-aromatic heterocycle containing one heteroatom in the ring.

Examples include the following:

[Chemical Formula 19]

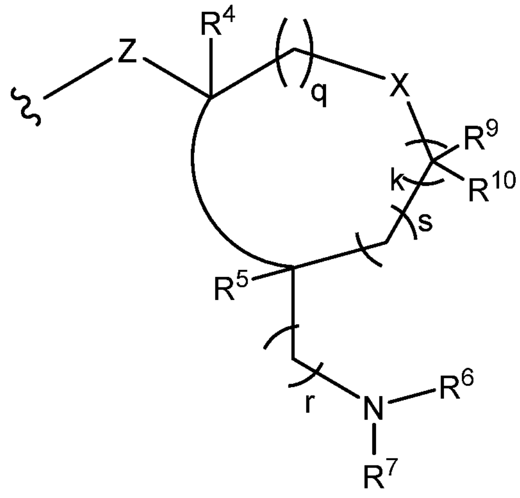

and the like. In the above formula, the case where q is 0 and X is O is preferable.

The substituent selected from the substituent group β or the substituent group β2 for the ring formed by $R^5$ and $R^6$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The ring formed by $R^5$ and $R^6$ is preferably a substituted or unsubstituted 3- to 8-membered non-aromatic heterocycle containing one to three heteroatom(s) in the ring, more preferably a substituted or unsubstituted 4- to 7-membered non-aromatic heterocycle containing one to three heteroatom(s) in the ring, further preferably a substituted or unsubstituted 6-membered monocyclic non-aromatic heterocycle or a substituted or unsubstituted bicyclic non-aromatic heterocycle containing one or two heteroatom(s) in the ring. Examples include the following:

[Chemical Formula 20]

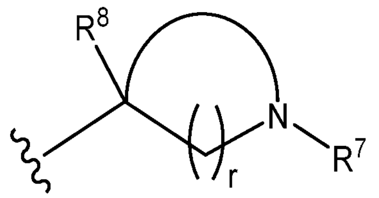

and the like.

In the above formula, the ring-constituting atoms of the non-aromatic heterocycle formed by $R^5$ and $R^6$ may be bonded to each other at any possible position or may be bridged.

Examples are shown below.

[Chemical Formula 21]

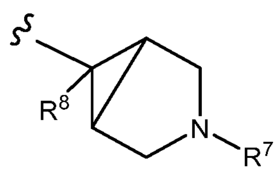
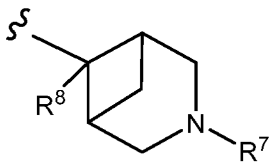
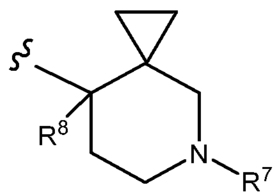
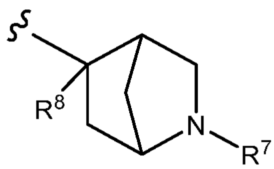
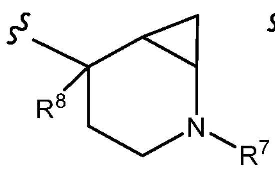
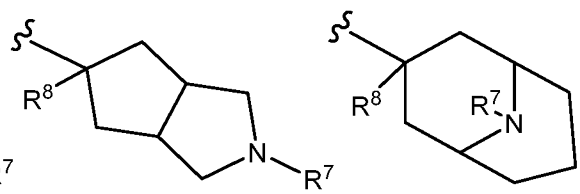
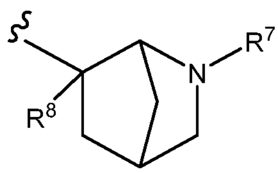

The substituent selected from the substituent group β or the substituent group β2 for the ring formed by $R^6$ and $R^7$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The ring formed by $R^6$ and $R^7$ is preferably a substituted or unsubstituted 4- to 7-membered non-aromatic heterocycle containing one to three heteroatom(s) in the ring, more preferably a substituted or unsubstituted 6-membered monocyclic non-aromatic heterocycle containing one or two heteroatom(s) in the ring.

The substituent selected from the substituent group β or the substituent group β2 for the ring formed by $R^8$ and $R^9$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

The ring formed by $R^8$ and $R^9$ is preferably a substituted or unsubstituted 3- to 7-membered non-aromatic carbocycle or a substituted or unsubstituted 4- to 7-membered non-aromatic heterocycle containing one to three heteroatom(s) in the ring, more preferably a substituted or unsubstituted 6-membered non-aromatic carbocycle or a substituted or unsubstituted 6-membered monocyclic non-aromatic heterocycle containing one or two heteroatom(s) in the ring. When $R^8$ and $R^9$ form the ring, k is 1 or 2.

Examples include the following:

[Chemical Formula 22]

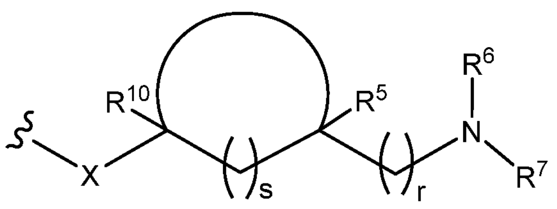

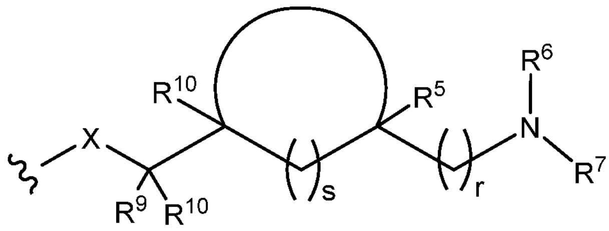

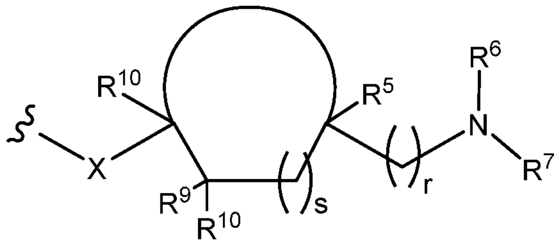

and the like.

When $R^5$ and $R^6$ form a ring, $R^7$ and $R^8$ may form a ring. In this case, the ring formed by $R^7$ and $R^8$ is preferably a substituted or unsubstituted 4- to 7-membered non-aromatic heterocycle containing one to three heteroatom(s) in the ring, more preferably a substituted or unsubstituted 6-membered monocyclic non-aromatic heterocycle containing one or two heteroatom(s) in the ring.

Examples include the following:

[Chemical Formula 23]

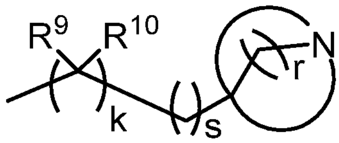

and the like.

Specific example is the following:

[Chemical Formula 24]

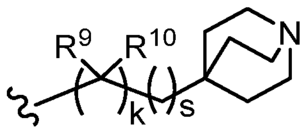

The non-aromatic heterocycle formed by $R^5$ and $R^6$ is formed by $R^5$-C($R^8$)-($CH_2$)$_r$-N($R^7$)-$R^6$. Also, the non-aromatic heterocycle formed by $R^7$ and $R^8$ is formed by $R^8$-C($R^5$)-($CH_2$)$_r$-N($R^6$)-$R^7$. When $R^5$ and $R^6$ form a ring and when $R^7$ and $R^8$ form a ring, r is preferably 1 to 3, more preferably 2.

The substituent selected from the substituent group β or the substituent group β2 for the ring formed by $R^7$ and $R^8$ is preferably a halogen atom, oxo, hydroxy, cyano, sulfanyl, amino, $C_1$-$C_6$ alky or halogenated $C_1$-$C_6$ alkyl, more preferably a halogen atom, oxo, hydroxy, cyano or sulfanyl, further preferably a halogen atom, oxo or hydroxy.

In addition, when one combination shown above forms a ring, other combinations may also form a ring. For example, when $R^3$ and $R^7$ form a ring, $R^5$ and $R^6$ or $R^8$ and $R^9$ may form a ring.

When $R^3$ and $R^8$ form a ring, $R^5$ and $R^6$ or $R^6$ and $R^7$ may form a ring.

When $R^5$ and $R^6$ form a ring, $R^3$ and $R^7$, $R^3$ and $R^8$, $R^7$ and $R^8$ or $R^8$ and $R^9$ may form a ring.

When $R^6$ and $R^7$ form a ring, $R^3$ and $R^8$ or $R^8$ and $R^9$ may form a ring.

When $R^8$ and $R^9$ form a ring, $R^3$ and $R^7$, $R^5$ and $R^6$ or $R^6$ and $R^7$ may form a ring.

For example, $R^8$ and $R^9$ may form a ring at the same time that $R^5$ and $R^6$ form a ring. In this case, k is 1 or 2.

Examples include the following:

[Chemical Formula 25]

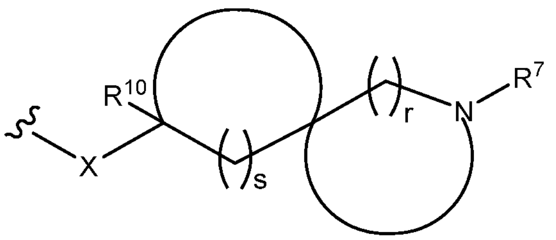

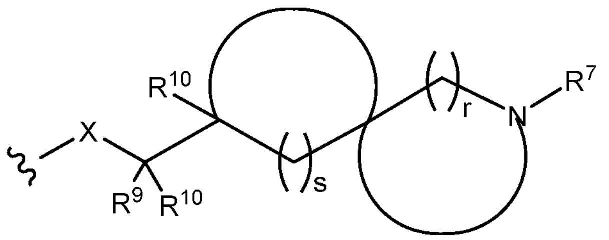

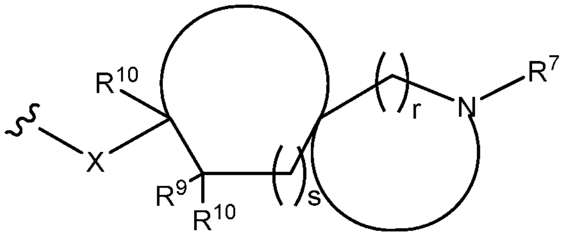

and the like.

When $R^8$ and $R^9$ form a ring at the same time that $R^5$ and $R^6$ form a ring, the ring formed by $R^5$ and $R^6$ is preferably a 3- to 7-membered non-aromatic heterocycle containing one to three heteroatom(s) in the ring, more preferably a substituted or unsubstituted 4-membered non-aromatic heterocycle containing one or two heteroatom(s) in the ring. When $R^8$ and $R^9$ form a ring at the same time that $R^5$ and $R^6$ form a ring, the ring formed by $R^8$ and $R^9$ is preferably a substituted or unsubstituted 3- to 7-membered non-aromatic carbocycle or a substituted or unsubstituted 3- to 7-membered non-aromatic heterocycle containing one to three heteroatom(s) in the ring, more preferably a substituted or unsubstituted 4- to 6-membered non-aromatic carbocycle or a substituted or unsubstituted 4- to 6-membered non-aromatic heterocycle containing one or two heteroatom(s) in the ring, further preferably a substituted or unsubstituted 4-membered non-aromatic carbocycle.

Z is preferably -OC(=O)-, -C(=O)O- or -OC(=O)O-, more preferably - OC(=O)- or -C(=O)O-, further preferably -OC(=O)-.

p is preferably 0 or 1, further preferably 0.
q is preferably 0 or 1, further preferably 0.
r is preferably 1 or 2.
s is preferably 0 or 1.

k is preferably 0 or 1, further preferably 0.
R is preferably a hydrogen atom.

A preferred embodiment of $R^1$, $R^2$, $L_1$, $L_2$, a, b, c, d, $R^3$, $R^4$, $R^5$, $R^6$, $R^7$, $R^8$, $R^9$, $R^{10}$, Z, X, p, q, s, k, r, R, the ring formed by $R^3$ and $R^4$, the ring formed by $R^3$ and $R^7$, the ring formed by $R^3$ and $R^8$, the ring formed by $R^5$ and $R^6$, the ring formed by $R^6$ and $R^7$, the ring formed by $R^7$ and $R^8$ and the ring formed by $R^8$ and $R^9$ in the compound represented by the formula (I) is described below. Examples of the compound represented by the formula (I) include embodiments having all combinations of specific examples given below.

$R^1$ is a substituted or unsubstituted formula: $-(CH_2)_a-L_1-(CH_2)_b-CH_3$ (hereinafter referred to as A-1).

$R^1$ is a substituted formula: $-(CH_2)_a-L_1-(CH_2)_b-CH_3$ (hereinafter referred to as A-2).

$R^1$ is a formula: $-(CH_2)_a-L_1-(CH_2)_b-CH_3$, substituted with $C_1$-$C_{10}$ alkyl or $C_2$-$C_{10}$ alkenyl (hereinafter referred to as A-3).

$R^1$ is a formula: $-(CH_2)_a-L_1-(CH_2)_b-CH_3$, substituted with $C_1$-$C_{10}$ alkyl (hereinafter referred to as A-4).

$R^1$ is an unsubstituted formula: $-(CH_2)_a-L_1-(CH_2)_b-CH_3$ (hereinafter referred to as A-5).

$L_1$ is -C(=O)O-, -OC(=O)- or -OC(=O)O- (hereinafter referred to as B-1).

$L_1$ is -C(=O)O- or -OC(=O)- (hereinafter referred to as B-2).

$L_1$ is -C(=O)O- (hereinafter referred to as B-3).

$L_1$ is -OC(=O)- (hereinafter referred to as B-4).

a and b are each independently an integer of 1 or more, the total of a and b is an integer of 5 to 25 (hereinafter referred to as C-1).
a and b are each independently an integer of 3 to 15 (hereinafter referred to as C-2).
a and b are each independently an integer of 5 to 10 (hereinafter referred to as C-3).
a and b are each independently an integer of 6 to 9 (hereinafter referred to as C-4).
a is an integer of 6 to 8 (hereinafter referred to as D-1).
b is an integer of 6 to 8 (hereinafter referred to as E-1).
a is 6 (hereinafter referred to as D-2).
a is 7 (hereinafter referred to as D-3).
a is 8 (hereinafter referred to as D-4).
b is 6 (hereinafter referred to as E-2).
b is 7 (hereinafter referred to as E-3).
b is 8 (hereinafter referred to as E-4).

$R^1$ is a group represented by formula:

[Chemical Formula 26]

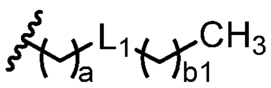
or
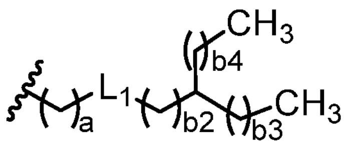

wherein, a and $L_1$ are the same as defined above item (1);

b1 is an integer of 1 or more, the total of a and b1 is an integer of 5 to 25;
b2 and b3 are each independently an integer of 0 or more, the total of a, b2 and b3 is an integer of 4 to 24;
b4 is an integer of 0 to 9 (hereinafter referred to as F-1).

$R^1$ is a group represented by formula:

[Chemical Formula 27]

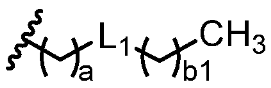
or
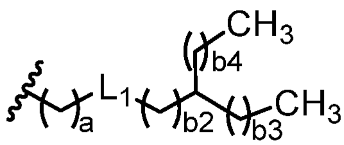

wherein, a is an integer of 6 to 8;

$L_1$ is -C(=O)O-;
b1 is an integer of 5 to 12;
b2 is an integer of 0 to 2;
b3 is an integer of 3 to 6;
b4 is an integer of 1 to 6 (hereinafter referred to as F-2).

$R^1$ is a group represented by formula:

[Chemical Formula 28]

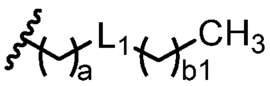

or

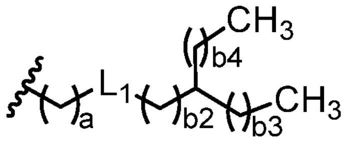

wherein, a is an integer of 6 to 8;

$L_1$ is -C(=O)O-;
b1 is 7;
b2 is an integer of 0 to 2;
b3 is an integer of 3 to 6;
b4 is an integer of 3 to 6 (hereinafter referred to as F-3).
$R^1$ is a group represented by formula:

[Chemical Formula 29]

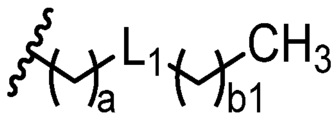

wherein, a, $L_1$ and blare the same as defined above item (F-3) (hereinafter referred to as F-4).
$R^1$ is a group represented by formula:

[Chemical Formula 30]

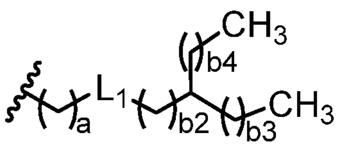

wherein, a, $L_1$, b2, b3 and b4 are the same as defined above item (F-3);
the total of b2, b3 and b4 is 8, 9, 10 or 12 (hereinafter referred to as F-5).
$R^2$ is substituted or unsubstituted $C_5$-$C_{20}$ alkyl or a substituted or unsubstituted formula: $-(CH_2)_c-L_2-(CH_2)_d-CH_3$ (hereinafter referred to as G-1).
$R^2$ is substituted or unsubstituted $C_5$-$C_{20}$ alkyl (hereinafter referred to as G-2).
$R^2$ is substituted $C_5$-$C_{20}$ alkyl (hereinafter referred to as G-3).
$R^2$ is $C_5$-$C_{20}$ alkyl substituted with $C_1$-$C_{10}$ alkyl or $C_2$-$C_{10}$ alkenyl (hereinafter referred to as G-4).
$R^2$ is unsubstituted $C_5$-$C_{20}$ alkyl (hereinafter referred to as G-5).
$R^2$ is unsubstituted $C_5$-$C_{10}$ alkyl (hereinafter referred to as G-6).
$R^2$ is unsubstituted $C_9$-$C_{10}$ alkyl (hereinafter referred to as G-7).
$R^2$ is unsubstituted $C_9$ alkyl (hereinafter referred to as G-8).
$R^2$ is a substituted or unsubstituted formula: $-(CH_2)_c-L_2-(CH_2)_d-CH_3$ (hereinafter referred to as G-9).
$R^2$ is a substituted formula: $-(CH_2)_c-L_2-(CH_2)_d-CH_3$ (hereinafter referred to as G-10).

$R^2$ is a formula: $-(CH_2)_c-L_2-(CH_2)_d-CH_3$, substituted with $C_1$-$C_{10}$ alkyl or $C_2$-$C_{10}$ alkenyl (hereinafter referred to as G-11).

$R^2$ is a formula: $-(CH_2)_c-L_2-(CH_2)_d-CH_3$, substituted with $C_1$-$C_{10}$ alkyl (hereinafter referred to as G-12).

$R^2$ is an unsubstituted formula: $-(CH_2)_c-L_2-(CH_2)_d-CH_3$ (hereinafter referred to as G-13).

$L_2$ is -C(=O)O-, -OC(=O)- or -OC(=O)O- (hereinafter referred to as H-1).

$L_2$ is -C(=O)O- or -OC(=O)- (hereinafter referred to as H-2).

$L_2$ is -C(=O)O- (hereinafter referred to as H-3).

$L_2$ is -OC(=O)- (hereinafter referred to as H-4).

c and d are each independently an integer of 1 or more, the total of a and b is an integer of 5 to 25 (hereinafter referred to as J-1).
c and d are each independently an integer of 3 to 15 (hereinafter referred to as J-2).
c and d are each independently an integer of 5 to 10 (hereinafter referred to as J-3).
c and d are each independently an integer of 6 to 9 (hereinafter referred to as J-4).
c is an integer of 6 to 8 (hereinafter referred to as K-1).
d is an integer of 6 to 8 (hereinafter referred to as L-1).
c is 6 (hereinafter referred to as K-2).
c is 7 (hereinafter referred to as K-3).
c is 8 (hereinafter referred to as K-4).
d is 6 (hereinafter referred to as L-2).
d is 7 (hereinafter referred to as L-3).
d is 8 (hereinafter referred to as L-4).

$R^2$ is a group represented by formula:

[Chemical Formula 31]

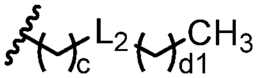

or

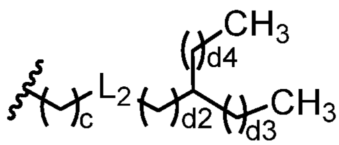

wherein, c and $L_2$ are the same as defined above item (1);

d1 is an integer of 1 or more, the total of c and d1 is an integer of 5 to 25;
d2 and d3 are each independently an integer of 0 or more, the total of c, d2 and d3 is an integer of 4 to 24;
d4 is an integer of 0 to 9 (hereinafter referred to as M-1).

$R^2$ is a group represented by formula:

[Chemical Formula 32]

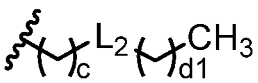

or

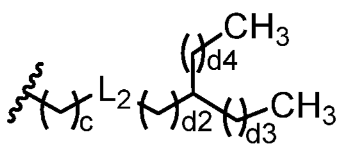

wherein, c is an integer of 6 to 8;

$L_2$ is -C(=O)O-;
d1 is an integer of 5 to 12;
d2 is an integer of 0 to 2;
d3 is an integer of 3 to 6;
d4 is an integer of 1 to 6 (hereinafter referred to as M-2).
$R^2$ is a group represented by formula:

[Chemical Formula 33]

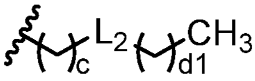 or 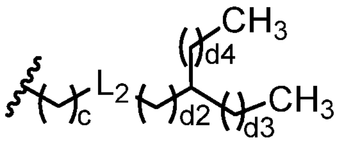

wherein, c is an integer of 6 to 8;

$L_2$ is -C(=O)O-;
d1 is 7;
d2 is an integer of 0 to 2;
d3 is an integer of 3 to 6;
d4 is an integer of 3 to 6 (hereinafter referred to as M-3).

$R^2$ is a group represented by formula:

[Chemical Formula 34]

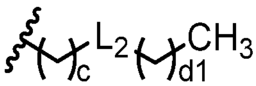

wherein, c, $L_2$ and d1 are the same as defined above item (M-3) (hereinafter referred to as M-4).

$R^2$ is a group represented by formula:

[Chemical Formula 35]

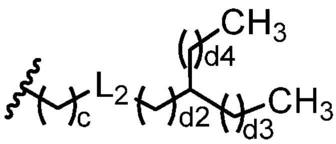

wherein, c, $L_2$, d2, d3 and d4 are the same as defined above item (M-3);
the total of d2, d3 and d4 is 8, 9, 10 or 12 (hereinafter referred to as M-5).

$R^1$ and $R^2$ are each independently a group selected from formula:

[Chemical Formula 36]

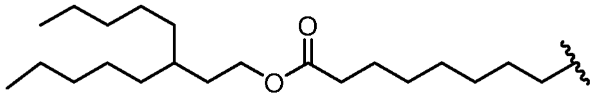
,

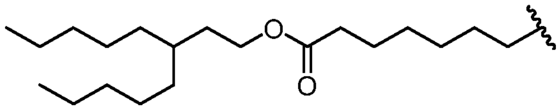
,

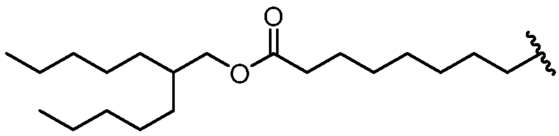
,

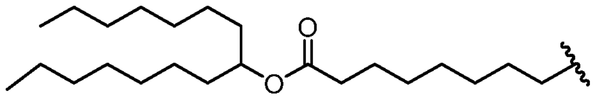
,

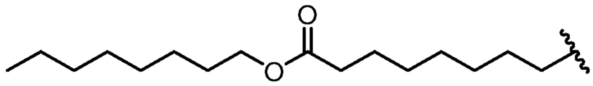
or

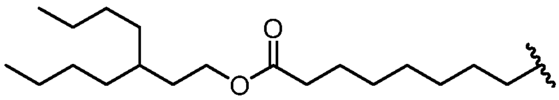

(hereinafter referred to as N-1).

[Chemical Formula 37]

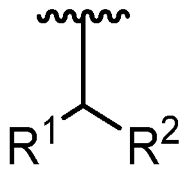

is a group selected from formula:

[Chemical Formula 38]

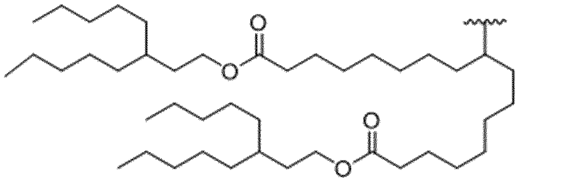

,

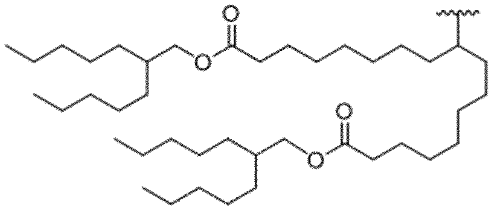

,

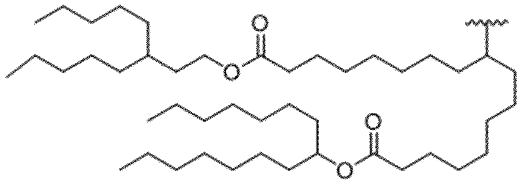

,

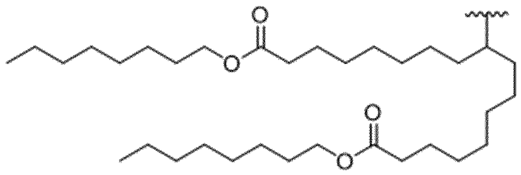

,

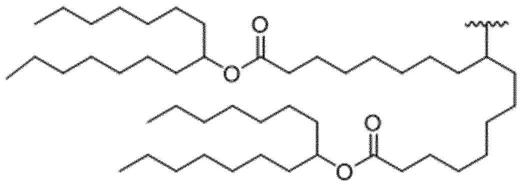

,

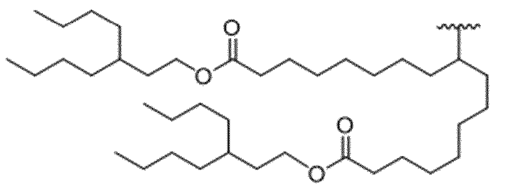

,

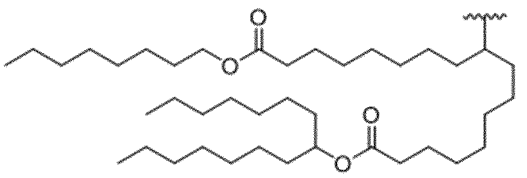

,

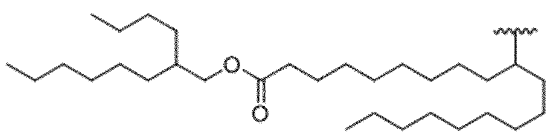

or

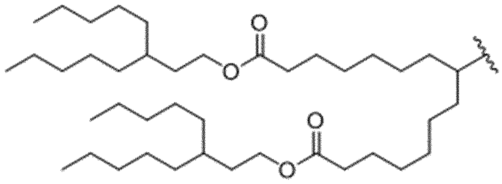

(hereinafter referred to as N-2).

[Chemical Formula 39]

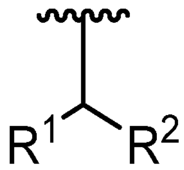

is a group selected from formula:

[Chemical Formula 40]

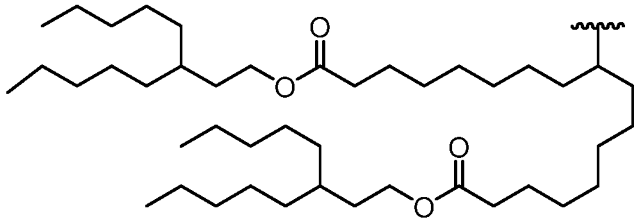

,

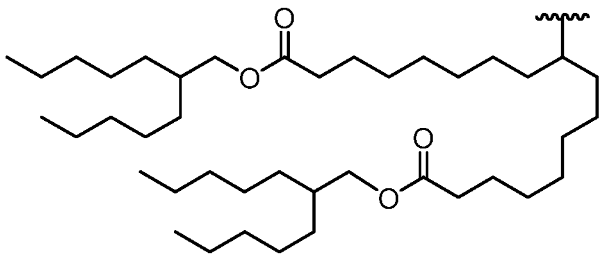

or

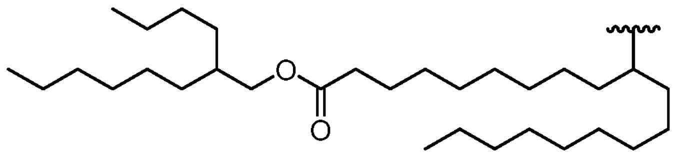

(hereinafter referred to as N-3).

[Chemical Formula 41]

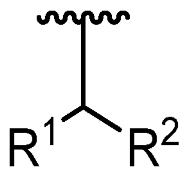

is a group selected from formula:

[Chemical Formula 42]

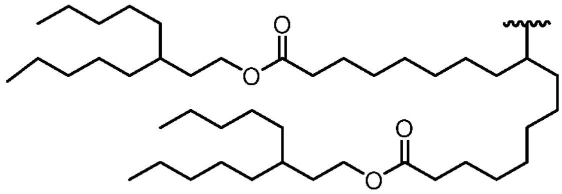

(hereinafter referred to as N-4).

[Chemical Formula 43]

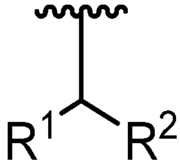

is a group selected from formula:

[Chemical Formula 44]

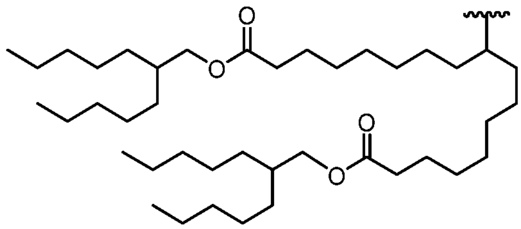

(hereinafter referred to as N-5).

[Chemical Formula 45]

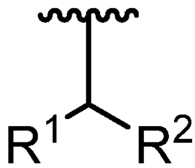

is a group selected from formula:

[Chemical Formula 46]

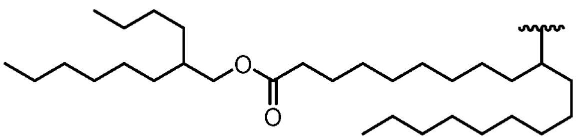

(hereinafter referred to as N-6).

$R^3$ is a hydrogen atom or substituted or unsubstituted $C_1$-$C_6$ alkyl (hereinafter referred to as O-1).

$R^3$ is a hydrogen atom or unsubstituted $C_1$-$C_6$ alkyl (hereinafter referred to as O-2).

$R^3$ is a hydrogen atom or unsubstituted $C_1$-$C_3$ alkyl (hereinafter referred to as O-3).

$R^3$ is a hydrogen atom (hereinafter referred to as O-4).

$R^3$ is unsubstituted $C_1$-$C_3$ alkyl (hereinafter referred to as O-5).

$R^4$ is a hydrogen atom or substituted or unsubstituted $C_1$-$C_6$ alkyl (hereinafter referred to as P-1).

$R^4$ is a hydrogen atom or unsubstituted $C_1$-$C_6$ alkyl (hereinafter referred to as P-2).

$R^4$ is a hydrogen atom or unsubstituted $C_1$-$C_3$ alkyl (hereinafter referred to as P-3).

$R^4$ is a hydrogen atom (hereinafter referred to as P-4).

$R^4$ is unsubstituted $C_1$-$C_3$ alkyl (hereinafter referred to as P-5).

$R^3$ and $R^4$ are taken together to form a substituted or unsubstituted $C_3$-$C_5$ non-aromatic carbocycle (hereinafter referred to as Q-1).

$R^3$ and $R^4$ are taken together to form an unsubstituted $C_3$-$C_5$ non-aromatic carbocycle (hereinafter referred to as Q-2).

$R^3$ and $R^4$ are taken together to form an unsubstituted cyclopropane or an unsubstituted cyclopentane (hereinafter referred to as Q-3).

$R^5$ is a hydrogen atom or substituted or unsubstituted $C_1$-$C_6$ alkyl (hereinafter referred to as S-1).

$R^5$ is a hydrogen atom or unsubstituted $C_1$-$C_6$ alkyl (hereinafter referred to as S-2).

$R^5$ is a hydrogen atom (hereinafter referred to as S-3).

$R^6$ is a hydrogen atom, substituted or unsubstituted $C_1$-$C_6$ alkyl, substituted or unsubstituted $C_2$-$C_6$ alkenyl, substituted or unsubstituted $C_2$-$C_6$ alkynyl or substituted or unsubstituted $C_3$-$C_7$ non-aromatic carbocyclyl (hereinafter referred to as T-1).

$R^6$ is a hydrogen atom, substituted or unsubstituted $C_1$-$C_6$ alkyl or substituted or unsubstituted $C_2$-$C_6$ alkenyl (hereinafter referred to as T-2).

$R^6$ is a hydrogen atom or substituted or unsubstituted $C_1$-$C_6$ alkyl (hereinafter referred to as T-3).

$R^6$ is substituted or unsubstituted $C_1$-$C_6$ alkyl (hereinafter referred to as T-4).

$R^6$ is unsubstituted $C_1$-$C_6$ alkyl (hereinafter referred to as T-5).

$R^7$ is a hydrogen atom, substituted or unsubstituted $C_1$-$C_6$ alkyl, substituted or unsubstituted $C_2$-$C_6$ alkenyl, substituted or unsubstituted $C_2$-$C_6$ alkynyl or substituted or unsubstituted $C_3$-$C_7$ non-aromatic carbocyclyl (hereinafter referred to as U-1).

$R^7$ is a hydrogen atom, substituted or unsubstituted $C_1$-$C_6$ alkyl or substituted or unsubstituted $C_2$-$C_6$ alkenyl (hereinafter referred to as U-2).

$R^7$ is a hydrogen atom or substituted or unsubstituted $C_1$-$C_6$ alkyl (hereinafter referred to as U-3).

$R^7$ is substituted or unsubstituted $C_1$-$C_6$ alkyl (hereinafter referred to as U-4).

$R^7$ is substituted with hydroxy or unsubstituted $C_1$-$C_6$ alkyl (hereinafter referred to as U-5).

$R^7$ is unsubstituted $C_1$-$C_6$ alkyl (hereinafter referred to as U-6).

$R^7$ is unsubstituted methyl (hereinafter referred to as U-7).

$R^8$ is a hydrogen atom (hereinafter referred to as V-1).

$R^9$ is a hydrogen atom (hereinafter referred to as W-1).

$R^{10}$ is a hydrogen atom (hereinafter referred to as Y-1).

$R^3$ and $R^7$ are taken together with the atom to which each $R^3$ and $R^7$ are attached to form a substituted or unsubstituted non-aromatic heterocycle (hereinafter referred to as Z-1).

$R^3$ and $R^7$ are taken together with the atom to which each $R^3$ and $R^7$ are attached to form a substituted or unsubstituted 3- to 7-membered non-aromatic heterocycle containing two to four heteroatoms in the ring (hereinafter referred to as Z-2).

$R^3$ and $R^7$ are taken together with the atom to which each $R^3$ and $R^7$ are attached to form a substituted or unsubstituted 5- to 7-membered monocyclic non-aromatic heterocycle containing two heteroatoms in the ring (hereinafter referred to as Z-3).

$R^3$ and $R^7$ are taken together with the atom to which each $R^3$ and $R^7$ are attached to form a substituted with alkyl or unsubstituted 6-membered monocyclic non-aromatic heterocycle containing two heteroatoms in the ring (hereinafter referred to as Z-4).

$R^3$ and $R^8$ are taken together with the atom to which each $R^3$ and $R^8$ are attached to form a substituted or unsubstituted non-aromatic heterocycle (hereinafter referred to as AA-1).

$R^3$ and $R^8$ are taken together with the atom to which each $R^3$ and $R^8$ are attached to form a substituted or unsubstituted 3- to 6-membered monocyclic non-aromatic heterocycle containing one to three heteroatom(s) in the ring (hereinafter referred to as AA-2).

$R^3$ and $R^8$ are taken together with the atom to which each $R^3$ and $R^8$ are attached to form a substituted or unsubstituted 5- to 6-membered monocyclic non-aromatic heterocycle containing one heteroatom in the ring (hereinafter referred to as AA-3).

$R^3$ and $R^8$ are taken together with the atom to which each $R^3$ and $R^8$ are attached to form an unsubstituted 5- to 6-membered monocyclic non-aromatic heterocycle containing one heteroatom in the ring (hereinafter referred to as AA-4).

$R^5$ and $R^6$ are taken together with the atom to which each $R^5$ and $R^6$ are attached to form a substituted or unsubstituted non-aromatic heterocycle (hereinafter referred to as AB-1).

$R^5$ and $R^6$ are taken together with the atom to which each $R^5$ and $R^6$ are attached to form a substituted or unsubstituted 3- to 8-membered non-aromatic heterocycle containing one to three heteroatom(s) in the ring (hereinafter referred to as AB-2).

$R^5$ and $R^6$ are taken together with the atom to which each $R^5$ and $R^6$ are attached to form a substituted or unsubstituted 4- to 7-membered non-aromatic heterocycle containing one to three heteroatom(s) in the ring (hereinafter referred to as AB-3).

$R^5$ and $R^6$ are taken together with the atom to which each $R^5$ and $R^6$ are attached to form a substituted or unsubstituted 6-membered monocyclic non-aromatic heterocycle or a substituted or unsubstituted bicyclic non-aromatic heterocycle, containing one or two heteroatom(s) in the ring (hereinafter referred to as AB-4).

$R^5$ and $R^6$ are taken together with the atom to which each $R^5$ and $R^6$ are attached to form a substituted or

unsubstituted 5- or 6-membered monocyclic non-aromatic heterocycle containing one heteroatom in the ring (hereinafter referred to as AB-5).

$R^5$ and $R^6$ are taken together with the atom to which each $R^5$ and $R^6$ are attached to form a substituted or unsubstituted 6-membered monocyclic non-aromatic heterocycle containing one heteroatom in the ring (hereinafter referred to as AB-6).

$R^5$ and $R^6$ are taken together with the atom to which each $R^5$ and $R^6$ are attached to form a substituted or unsubstituted piperidine (hereinafter referred to as AB-7).

$R^5$ and $R^6$ are taken together with the atom to which each $R^5$ and $R^6$ are attached to form a substituted with $C_1$-$C_3$ alkyl and/or halogen, or unsubstituted piperidine (hereinafter referred to as AB-8).

$R^5$ and $R^6$ are taken together with the atom to which each $R^5$ and $R^6$ are attached to form a substituted piperidine (hereinafter referred to as AB-9).

$R^5$ and $R^6$ are taken together with the atom to which each $R^5$ and $R^6$ are attached to form a piperidine substituted with $C_1$-$C_3$ alkyl and/or halogen (hereinafter referred to as AB-10).

$R^5$ and $R^6$ are taken together with the atom to which each $R^5$ and $R^6$ are attached to form an unsubstituted piperidine or an unsubstituted pyrrolidine (hereinafter referred to as AB-12).

$R^5$ and $R^6$ are taken together with the atom to which each $R^5$ and $R^6$ are attached to form an unsubstituted piperidine (hereinafter referred to as AB-11).

$R^6$ and $R^7$ are taken together with the atom to which each $R^6$ and $R^7$ are attached to form a substituted or unsubstituted non-aromatic heterocycle (hereinafter referred to as AC-1).

$R^6$ and $R^7$ are taken together with the atom to which each $R^6$ and $R^7$ are attached to form a substituted or unsubstituted 4- to 7-membered non-aromatic heterocycle containing one to three heteroatom(s) in the ring (hereinafter referred to as AC-2).

$R^6$ and $R^7$ are taken together with the atom to which each $R^6$ and $R^7$ are attached to form a substituted or unsubstituted 6-membered monocyclic non-aromatic heterocycle containing one or two heteroatom(s) in the ring (hereinafter referred to as AC-3).

$R^8$ and $R^9$ are taken together with the atom to which each $R^8$ and $R^9$ are attached to form a substituted or unsubstituted non-aromatic carbocycle or a substituted or unsubstituted non-aromatic heterocycle (hereinafter referred to as AD-1).

$R^8$ and $R^9$ are taken together with the atom to which each $R^8$ and $R^9$ are attached to form a substituted or unsubstituted 3- to 7-membered non-aromatic carbocycle or a substituted or unsubstituted 5- to 7-membered non-aromatic heterocycle containing one to three heteroatom(s) in the ring (hereinafter referred to as AD-2).

$R^8$ and $R^9$ are taken together with the atom to which each $R^8$ and $R^9$ are attached to form a substituted or unsubstituted 4- to 6-membered monocyclic non-aromatic carbocycle or a substituted or unsubstituted 4- to 6-membered monocyclic non-aromatic heterocycle containing one or two heteroatom(s) in the ring (hereinafter referred to as AD-3).

$R^8$ and $R^9$ are taken together with the atom to which each $R^8$ and $R^9$ are attached to form a substituted or unsubstituted 4- to 6-membered monocyclic non-aromatic carbocycle (hereinafter referred to as AD-4).

$R^8$ and $R^9$ are taken together with the atom to which each $R^8$ and $R^9$ are attached to form an unsubstituted 4- to 6-membered monocyclic non-aromatic carbocycle (hereinafter referred to as AD-5).

$R^7$ and $R^8$, when $R^5$ and $R^6$ form a ring, are taken together with the atom to which each $R^7$ and $R^8$ are attached to form a substituted or unsubstituted non-aromatic heterocycle (hereinafter referred to as AE-1).

$R^7$ and $R^8$, when $R^5$ and $R^6$ form a ring, are taken together with the atom to which each $R^7$ and $R^8$ are attached to form a substituted or unsubstituted 4- to 7-membered non-aromatic heterocycle containing one to three heteroatom(s) in the ring (hereinafter referred to as AE-2).

$R^7$ and $R^8$, when $R^5$ and $R^6$ form a ring, are taken together with the atom to which each $R^7$ and $R^8$ are attached to form a substituted or unsubstituted 6-membered monocyclic non-aromatic heterocycle containing one or two heteroatom(s) in the ring (hereinafter referred to as AE-3).

$R^8$ and $R^9$ form a substituted or unsubstituted 3- to 7-membered non-aromatic carbocycle or a substituted or unsubstituted 3- to 7-membered non-aromatic heterocycle containing one to three heteroatom(s) in the ring, at the same time that $R^5$ and $R^6$ form a 3- to 7-membered non-aromatic heterocycle containing one to three heteroatom(s) in the ring (hereinafter referred to as AF-1).

$R^8$ and $R^9$ form an unsubstituted 4-membered non-aromatic carbocycle, at the same time that $R^5$ and $R^6$ form an unsubstituted 4-membered non-aromatic heterocycle containing one or two heteroatom(s) in the ring (hereinafter referred to as AF-2).

Z is -OC(=O)-, -C(=O)O-, -OC(=O)O-, -C(=O)N(R)-, -N(R)C(=O)-, - OC(=O)N(R)-, -N(R)C(=O)N(R)-, -N(R)C(=O)O-, -C(=S)N(R)-, -N(R)C(=S)-, - C(=O)S-, -SC(=O)-, -N(R)S(=O)$_2$-, -OS(=O)$_2$-, -OP(=O)(-OR)O-, -OP(=S)(-OR)O-, - OS(=O)$_2$-O-, -S(=O)$_2$-N(R)-, -OP(=O)(-NR)O-, -C(=S)O- or -OC(=S)- (hereinafter referred to as AG-1).

Z is -OC(=O)-, -C(=O)O-, -OC(=O)O-, -C(=O)N(R)-, -N(R)C(=O)O- or - N(R)C(=O)- (hereinafter referred to as AG-2).

Z is -OC(=O)-, -C(=O)O-, -OC(=O)O-, -C(=O)N(H)-, -C(=O)N(Me)-, - N(H)C(=O)O- or -N(H)C(=O)- (hereinafter referred to as AG-3).

Z is -OC(=O)-, -C(=O)O- or -OC(=O)O- (hereinafter referred to as AG-4).

Z is -OC(=O)- or -C(=O)O- (hereinafter referred to as AG-5).

Z is -OC(=O)- (hereinafter referred to as AG-6).

X is O or S (hereinafter referred to as AH-1).

X is O (hereinafter referred to as AH-2).

X is S (hereinafter referred to as AH-3).

p is an integer of 0 to 2 (hereinafter referred to as AI-1).
p is 0 or 1 (hereinafter referred to as AI-2).
p is 0 (hereinafter referred to as AI-3).
p is 1 (hereinafter referred to as AI-4).
q is an integer of 0 to 2 (hereinafter referred to as AJ-1).
q is 0 or 1 (hereinafter referred to as AJ-2).
q is 0 (hereinafter referred to as AJ-3).
q is 1 (hereinafter referred to as AJ-4).
s is an integer of 0 to 2 (hereinafter referred to as AK-1).
s is 0 or 1 (hereinafter referred to as AK-2).
s is 0 (hereinafter referred to as AK-3).
s is 1 (hereinafter referred to as AK-4).
k is an integer of 0 to 2 (hereinafter referred to as AL-1).
k is 0 or 1 (hereinafter referred to as AL-2).
k is 0 (hereinafter referred to as AL-3).
k is 1 (hereinafter referred to as AL-4).
r is an integer of 0 to 5 (hereinafter referred to as AM-1).
r is an integer of 0 to 3 (hereinafter referred to as AM-2).
r is 1 or 2 (hereinafter referred to as AM-3).
r is 1 (hereinafter referred to as AM-4).
r is 2 (hereinafter referred to as AM-5).

R is each independently a hydrogen atom or substituted or unsubstituted $C_1$-$C_6$ alkyl (hereinafter referred to as AN-1).

R is each independently a hydrogen atom (hereinafter referred to as AN-1).

In the compound represented by Formula (I), the following embodiments are more preferable. Embodiments of all combinations of the following specific examples are exemplified.

(i) A compound represented by Formula (Ia):

[Chemical Formula 47]

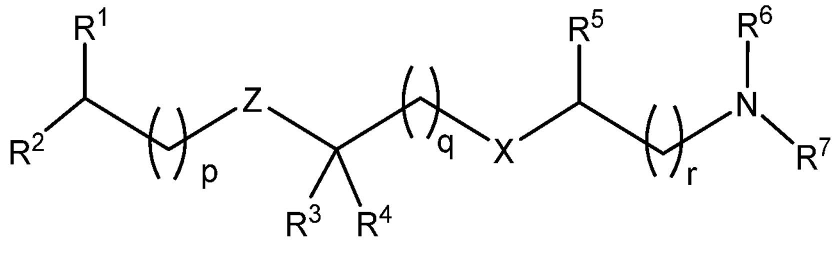

(Ia)

or a pharmaceutically acceptable salt thereof.

wherein $R^1$ includes the above (F-2), (F-3), (F-4) or (F-5).
$R^2$ includes the above (M-2), (M-3), (M-4) or (M-5).
Alternatively, $R^1$ and $R^2$ include the above (N-1), (N-2), (N-3), (N-4) or (N-5).
p includes the above (AI-2), (AI-3) or (AI-4).
Z includes the above (AG-2), (AG-3), (AG-4), (AG-5) or (AG-6).
$R^3$ includes the above (O-3), (O-4) or (O-5).

$R^4$ includes the above (P-3), (P-4) or (P-5).
Alternatively, $R^3$ and $R^4$ include the above (Q-2) or (Q-3).
q includes the above (AJ-2), (AJ-3) or (AJ-4).
X includes the above (AH-1), (AH-2) or (AH-3).
r includes the above (AM-2), (AM-3), (AM-4) or (AM-5).
$R^5$ includes the above (S-2) or (S-3).
$R^6$ includes the above (T-4) or (T-5).
Alternatively, $R^5$ and $R^6$ are the above (AB-3), (AB-5), (AB-6), (AB-7), (AB-8), (AB-9), (AB-10) or (AB-11).
$R^7$ includes the above (U-3), (U-4), (U-5), (U-6) or (U-7).

(ii) A compound represented by Formula (Ib):

[Chemical Formula 48]

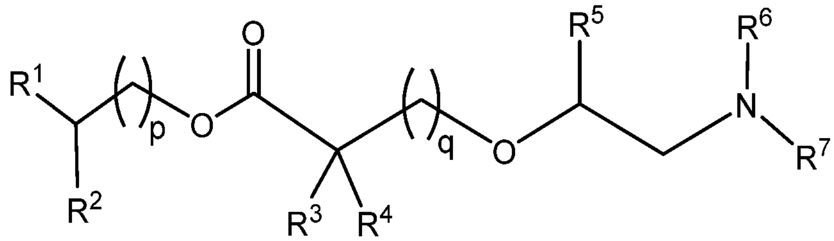

(Ib)

or a pharmaceutically acceptable salt thereof.

wherein $R^1$ and $R^2$ include the above (N-1), (N-2), (N-3), (N-4) or (N-5).
p includes the above (AI-2), (AI-3) or (AI-4).
$R^3$ includes the above (O-3).
$R^4$ includes the above (P-4).
q includes the above (AJ-3).
$R^5$ includes the above (S-2) or (S-3).
$R^6$ includes the above (T-5).
$R^7$ includes the above (U-6).

(iii) A compound represented by Formula (Ic):

[Chemical Formula 49]

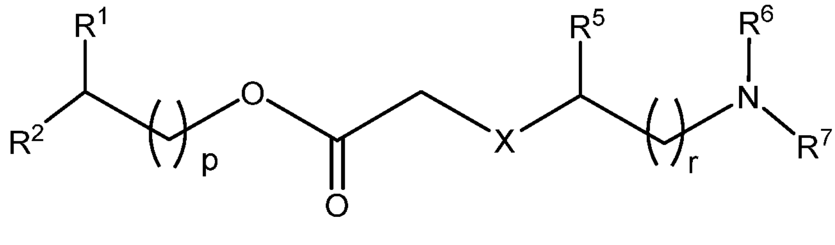

(Ic)

or a pharmaceutically acceptable salt thereof.

wherein $R^1$ and $R^2$ include the above (N-2), (N-3), (N-4) or (N-5).
p includes the above (AI-3) or (AI-4).
X includes the above (AH-3).
r includes the above (AM-5).
$R^5$ and R6 include the above (AB-11) or (AB-12).
$R^7$ includes the above (U-5) or (U-7).

(iv) A compound represented by Formula (Ic):

[Chemical Formula 50]

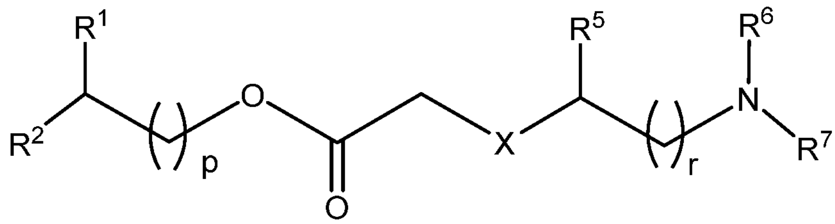

(Ic)

or a pharmaceutically acceptable salt thereof.

wherein $R^1$ and $R^2$ include the above (N-2), (N-3), (N-4) or (N-5).
p includes the above (AI-3) or (AI-4).
X includes the above (AH-2).
r includes the above (AM-5).
$R^5$ and R6 include the above (AB-11) or (AB-12).
$R^7$ includes the above (U-5) or (U-7).

One or more hydrogen, carbon and/or other atoms in the compounds represented by Formula (I) may be replaced with isotopes of hydrogen, carbon and/or other atoms respectively. Examples of isotopes include hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$ and $^{36}Cl$, respectively. The compounds represented by Formula (I) include the compounds replaced with these isotopes. The compounds replaced with the isotopes are useful as medicaments and include all radiolabeled compounds of the compound represented by Formula (I). A "method of radiolabeling" in the manufacture of the "radiolabeled compounds" is encompassed by the present invention, and the "radiolabeled compounds" are useful for studies on metabolized drug pharmacokinetics, studies on binding assay and/or diagnostic tools.
A radiolabeled compound of the compounds represented by Formula (I) can be prepared using well-known methods in the art. For example, a tritium-labeled compound represented by Formula (I) can be prepared by introducing tritium to a certain compound represented by Formula (I), through a catalytic dehalogenation reaction using tritium. This method comprises reacting an appropriately-halogenated precursor of the compound represented by Formula (I) with tritium gas in the presence of an appropriate catalyst, such as Pd/C, and in the presence or absence of a base. The other appropriate method for preparing a tritium-labeled compound can be referred to "Isotopes in the Physical and Biomedical Sciences, Vol. 1, Labeled Compounds (Part A), Chapter 6 (1987)". A $^{14}C$-labeled compound can be prepared by using a raw material having $^{14}C$.
The "pharmacologically acceptable salts" mean salts of the compounds represented by Formula (I) with alkaline metal (e.g., lithium, sodium, potassium or the like), alkaline earth metal (e.g., calcium, magnesium,barium or the like), magnesium, transition metal (e.g. zinc, iron, copper, nickel, cobalt or the like), aluminum, ammonia, organic bases (e.g. trimethylamine, triethylamine, trioctylamine, diethylamine, dibenzylamine, dicyclohexylamine, N, N'-dibenzylethylenediamine, salt ethanolamine, diethanolamine, triethanolamine, meglumine, ethylenediamine, N-benzyl-phenethylamine, pyridine, picoline, quinoline, morpholine, phenylglycine alkyl ester, N-methylglucamine, guanidine, chloroprocaine salt, procaine, piperazine, tetramethylammonium, hydroxymethylaminomethane or the like), amino acids (e.g. glycine, arginine, ornithine, methionine, tyrosine, glutamine, aspartic acid or the like), or with inorganic acids (e.g., hydrochloric acid, perchloric acid, sulfuric acid, nitric acid, carbonic acid, hydrobromic acid, phosphoric acid, hydrofluoric acid, hydroiodic acid, phosphoric acid or the like) or organic acids (e.g. formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, trifluoroacetic acid, trifluoromethanesulfonic acid or the like), preferably salts of the compounds represented by Formula (I) with inorganic acids or organic acids.
The compounds represented by Formula (I) of the present invention can be, for example, prepared by the general synthetic procedures described below. The methods for extraction, purification and the like may be carried out by usual methods for the experiments of organic chemistry.
The compounds of the present invention can be synthesized by referring to the known methods in the art.
A compound represented by Formula (I) wherein Z is -OC(=O)- can be, for example, prepared as follows.

[Chemical Formula 51]

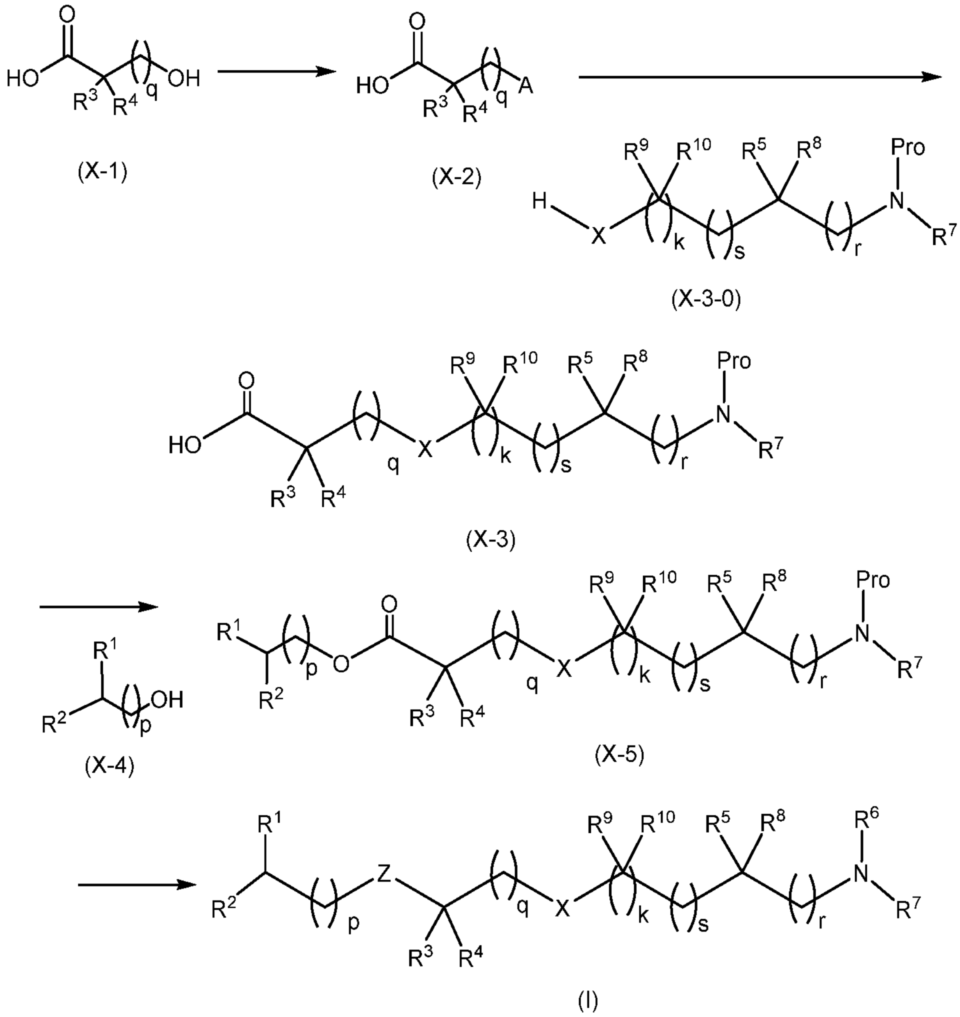

(X-1) (X-2) (X-3-0) (X-3) (X-4) (X-5) (I)

wherein A is a leaving group (e.g., a halogen atom, tosyloxy, mesyloxy or the like); Pro is a protecting group (e.g., tert-butoxycarbonyl, benzyloxycarbonyl or the like); and other symbols each are the same as defined above.

A compound represented by Formula (X-2) can be prepared by reacting a compound represented by Formula (X-1) with a hydrohalic acid or a sulfonyl halide.

As the hydrohalic acid, hydrofluoric acid, hydrochloric acid, hydrobromic acid, hydroiodic acid and the like are exemplified.

As the sulfonyl halide, mesyl chloride, tosyl chloride and the like are exemplified.

The reaction temperature is 0°C to reflux temperature of the solvent, preferably 20 to 60°C.

The reaction time is 1 to 48 hours, preferably 1 to 6 hours.

As the reaction solvent, tetrahydrofuran, dimethylformamide, dichloromethane, chloroform, toluene, xylene, neat and the like are exemplified. The reaction solvent can be used alone or in combination.

A compound represented by Formula (X-3) can be prepared by reacting the compound represented by Formula (X-2) with a compound represented by Formula (X-3-0) in the presence or absence of a base.

As the base, sodium hydride, DIEA, potassium-tert-butoxide, lithium hydroxide, potassium hydroxide, sodium hydroxide, potassium carbonate and the like are exemplified. The base can be used in 2 to 10 mole equivalents per an equivalent of the compound represented by Formula (X-2).

The reaction temperature is 0°C to reflux temperature of the solvent, preferably 20 to 80°C.

The reaction time is 1 to 24 hours, preferably 1 to 6 hours.

As the reaction solvent, tetrahydrofuran, dimethylformamide, tert-butanol, water and the like are exemplified. The reaction solvent can be used alone or in combination.

A compound represented by Formula (X-5) can be prepared by reacting the compound represented by Formula (X-3) with a compound represented by Formula (X-4) in the presence or absence of a base and in the presence or absence of a condensing agent.

As the base, triethylamine, DIEA, DMAP, pyridine and the like are exemplified. The base can be used in 2 to 20 mole equivalents per an equivalent of the compound represented by Formula (X-4).

As the condensing agent, DCC, DIC, EDC, 2-methyl-6-nitrobenzoic anhydride, thionyl chloride and the like are exemplified.

The reaction temperature is 0°C to reflux temperature of the solvent, preferably 20 to 60°C.

The reaction time is 1 to 72 hours, preferably 1 to 20 hours.

As the reaction solvent, dichloromethane, tetrahydrofuran, dioxane, dimethylformamide, N-methylpyrrolidone, benzene and the like are exemplified. The reaction solvent can be used alone or in combination.

The compound represented by Formula (I) can be prepared by deprotecting the amino protecting group of the compound represented by Formula (X-5), followed by introducing $R^6$.

As the deprotection condition, reacting with an acid such as hydrochloric acid, trifluoroacetic acid and the like, or reacting with hydrogen gas in the presence of a metal catalyst are exemplified.

The reaction temperature is 0 to 60°C, preferably 10 to 40°C.

The reaction time is 1 to 6 hours, preferably 1 to 2 hours.

As the reaction solvent, ethyl acetate, dioxane, tetrahydrofuran, dichloromethane, methanol and the like are exemplified. The reaction solvent can be used alone or in combination.

$R^6$ can be introduced into the deprotected compound by, for example, using aldehyde or formaldehyde to form an iminium cation intermediate, followed by reduction.

As the reducing agent, sodium triacetoxyborohydride, sodium cyanoborohydride and the like can be used.

The reaction temperature is 0°C to reflux temperature of the solvent, preferably 20 to 40°C.

The reaction time is 1 to 6 hours, preferably 1 to 2 hours.

As the reaction solvent, dichloromethane, acetonitrile, tetrahydrofuran and the like are exemplified. The reaction solvent can be used alone or in combination.

[Chemical Formula 52]

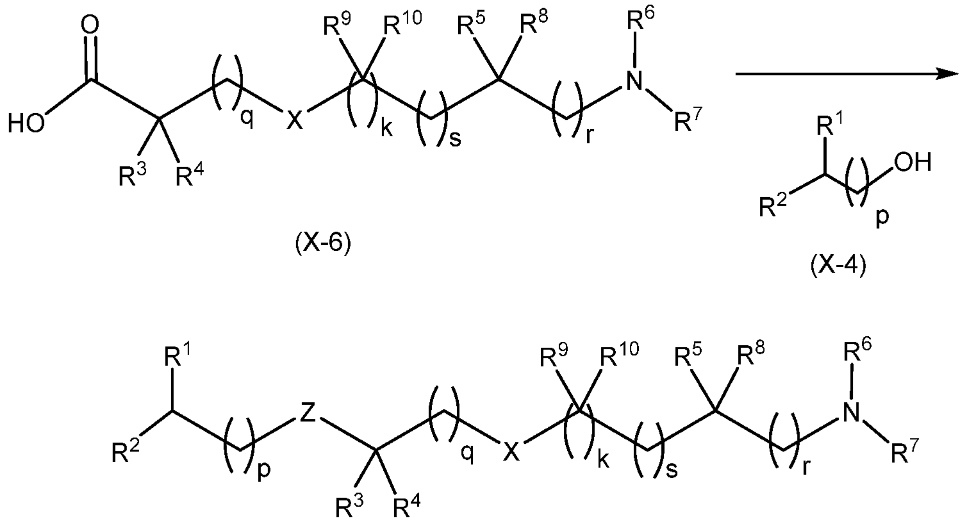

(X-6)

(X-4)

(I)

wherein symbols each are the same as defined above.

The compound represented by Formula (I) can be prepared by reacting a compound represented by Formula (X-6) with the compound represented by Formula (X-4) in the presence or absence of a base and in the presence or absence of a condensing agent.

This step can be performed in the same manner as the step of preparing the compound represented by Formula (X-5) from the compound represented by Formula (X-3) described above.

[Chemical Formula 53]

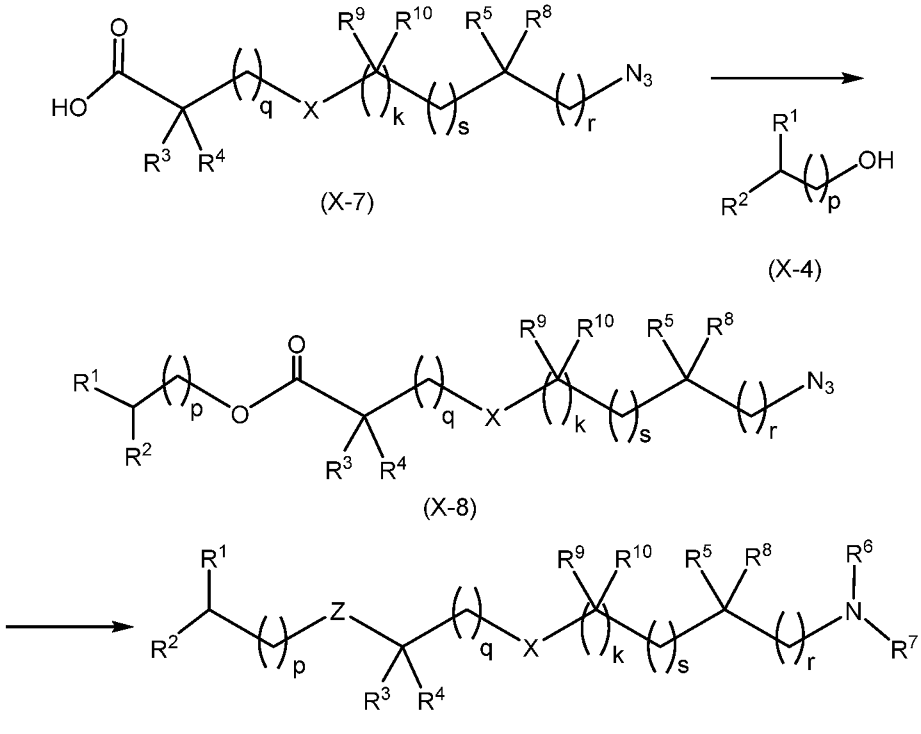

(X-7) (X-4) (X-8) (I)

wherein symbols each are the same as defined above.

A compound represented by Formula (X-8) can be prepared by reacting a compound represented by Formula (X-7) with the compound represented by Formula (X-4) in the presence or absence of a base.

This step can be performed in the same manner as the step of preparing the compound represented by Formula (I) from the compound represented by Formula (X-6) described above.

The compound represented by Formula (I) can be prepared by reacting a compound represented by Formula (X-8) with hydrogen gas in the presence of a metal catalyst, followed by introducing $R^6$ and $R^7$.

As the metal catalyst, palladium-carbon (Pd-C), platinum oxide, rhodium-aluminum oxide, chlorotris(triphenylphosphine)rhodium (I) and the like are exemplified. The metal catalyst can be used in an amount of 0.01 to 100% by weight with respect to the compound represented by Formula (X-8).

The hydrogen pressure can be 1 to 50 atm. As a hydrogen source, cyclohexene, 1,4-cyclohexadiene, formic acid, ammonium formate and the like can be used.

The reaction temperature is 0°C to reflux temperature of the solvent, preferably 20 to 40°C.

The reaction time is 1 to 72 hours, preferably 1 to 8 hours.

As the reaction solvent, methanol, ethyl acetate, tetrahydrofuran, dioxane and the like are exemplified. The reaction solvent can be used alone or in combination.

In order to stabilize the produced amino compound, it is desirable to carry out in an acidic solution. For example, it can be carried out by adding a hydrochloric acid-1,4-dioxane solution and the like.

The step of preparing the compound represented by Formula (I) by introducing $R^6$ and $R^7$ into the produced amino compound can be performed in the same manner as the step of preparing the compound represented by Formula (I) by introducing $R^6$ into the amino deprotected compound of the compound represented by formula (X-5) described above.

[Chemical Formula 54]

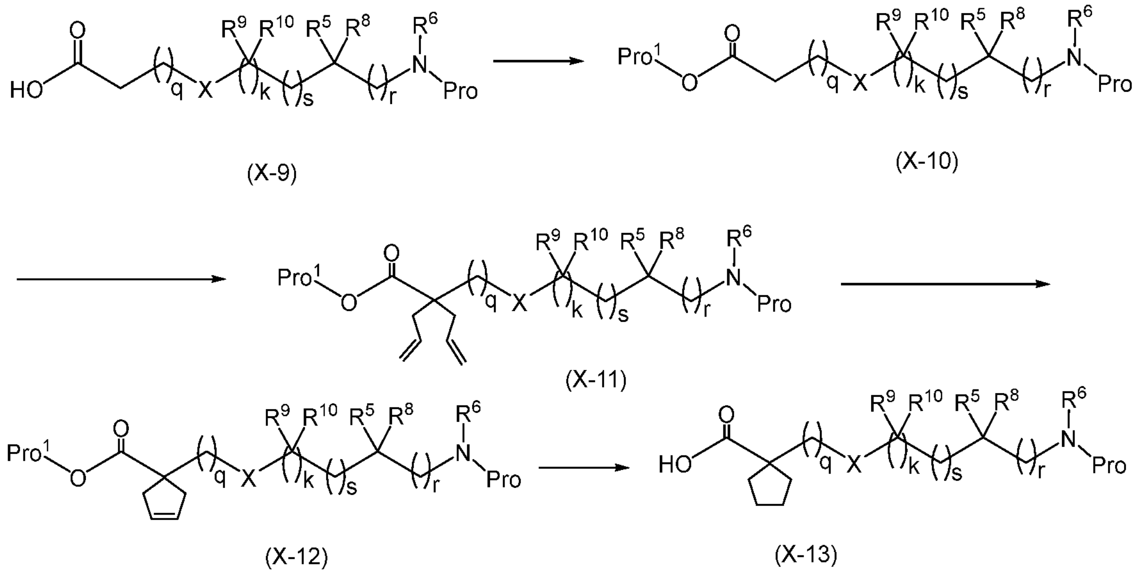

(X-9) (X-10) (X-11) (X-12) (X-13)

wherein $Pro^1$ is benzyl or the like; and other symbols each are the same as defined above.

A compound represented by Formula (X-10) can be prepared by introducing a protecting group into a compound represented by Formula (X-9) in the presence or absence of a base.

As the base, sodium hydrogen carbonate, cesium carbonate, potassium carbonate, sodium carbonate and the like are exemplified. The base can be used in 1 to 10 mole equivalent(s) per an equivalent of the compound represented by Formula (X-9).

The reaction temperature is 0°C to reflux temperature of the solvent, preferably 20 to 50°C.

The reaction time is 1 to 72 hours, preferably 2 to 18 hours.

As the reaction solvent, DMF, tetrahydrofuran, dioxane, acetonitrile and the like are exemplified. The reaction solvent can be used alone or in combination.

A compound represented by Formula (X-11) can be prepared by reacting the compound represented by Formula (X-10) with allyl halide in the presence or absence of a base.

The reaction temperature is -78°C to 30°C, preferably -78°C to 0°C.

The reaction time is 1 to 8 hours, preferably 1 to 2 hours.

As the base, lithium hexamethyldisilazide (LHMDS), LDA, NaH and the like are exemplified.

As the reaction solvent, n-hexane, tetrahydrofuran, dimethylformamide and the like are exemplified. The reaction solvent can be used alone or in combination.

A compound represented by Formula (X-12) can be prepared by reacting the compound represented by Formula (X-11) with metal catalyst.

The reaction temperature is 0°C to reflux temperature of the solvent, preferably 30°C to reflux temperature of the solvent.

The reaction time is 1 to 18 hours, preferably 1 to 2 hours.

As the reaction solvent, benzene, dichloromethane, toluene and the like are exemplified. The reaction solvent can be used alone or in combination.

As the metal catalyst, first generation Grubbs catalyst, second generation Grubbs catalyst, Schrock catalyst and the like are exemplified.

A compound represented by Formula (X-13) can be prepared by reacting the compound represented by Formula (X-12) with hydrogen gas in the presence of a metal catalyst.

As the metal catalyst, palladium-carbon (Pd-C), platinum oxide, rhodium-aluminum oxide, chlorotris(triphenylphosphine)rhodium (I) and the like are exemplified. The metal catalyst can be used in an amount of 0.01 to 100% by weight with respect to the compound represented by Formula (X-12).

The hydrogen pressure can be 1 to 50 atm. As a hydrogen source, cyclohexene, 1,4-cyclohexadiene, formic acid, ammonium formate and the like can be used.

The reaction temperature is 0°C to reflux temperature of the solvent, preferably 20 to 40°C.

The reaction time is 1 to 72 hours, preferably 1 to 8 hours.

As the reaction solvent, methanol, ethyl acetate, tetrahydrofuran, dioxane and the like are exemplified. The reaction solvent can be used alone or in combination.

The compound represented by Formula (I) can be prepared from the compound represented by Formula (X-13) in the same manner as the step of preparing the compound represented by formula (I) from the compound represented by formula (X-3) described above.

[Chemical Formula 55]

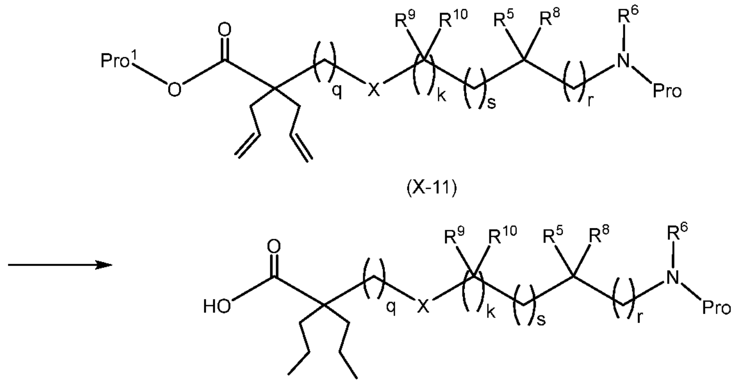

(X-11)

(X-14)

wherein symbols each are the same as defined above.

A compound represented by Formula (X-14) can be prepared by reacting the compound represented by Formula (X-11) with hydrogen gas in the presence of a metal catalyst.

This step can be performed in the same manner as the step of preparing the compound represented by Formula (X-13) from the compound represented by Formula (X-12) described above.

The compound represented by Formula (I) can be prepared from the compound represented by Formula (X-14) in the same manner as the step of preparing the compound represented by formula (I) from the compound represented by formula (X-3) described above.

A compound represented by Formula (I) wherein Z is -C(=O)O- can be, for example, prepared as follows.

[Chemical Formula 56]

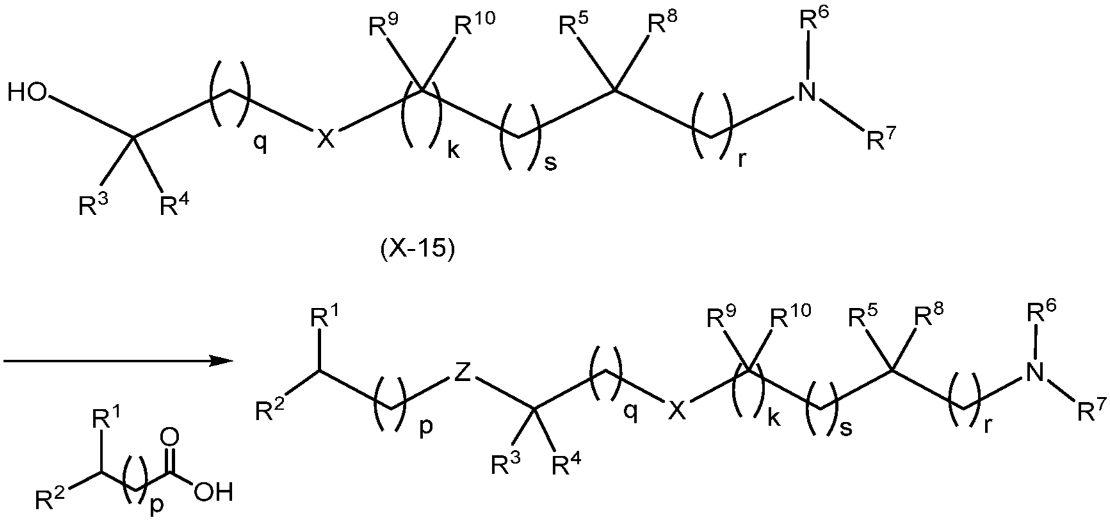

(X-15)

(I)

(X-16)

wherein symbols each are the same as defined above.

The compound represented by Formula (I) can be prepared by reacting a compound represented by Formula (X-15) with the compound represented by Formula (X-16) in the presence or absence of a base and in the presence or absence of a condensing agent.

This step can be performed in the same manner as the step of preparing the compound represented by Formula (X-5) from the compound represented by Formula (X-3) described above.

A compound represented by Formula (I) wherein Z is -N(R)-C(=O)- can be, for example, prepared as follows.

[Chemical Formula 57]

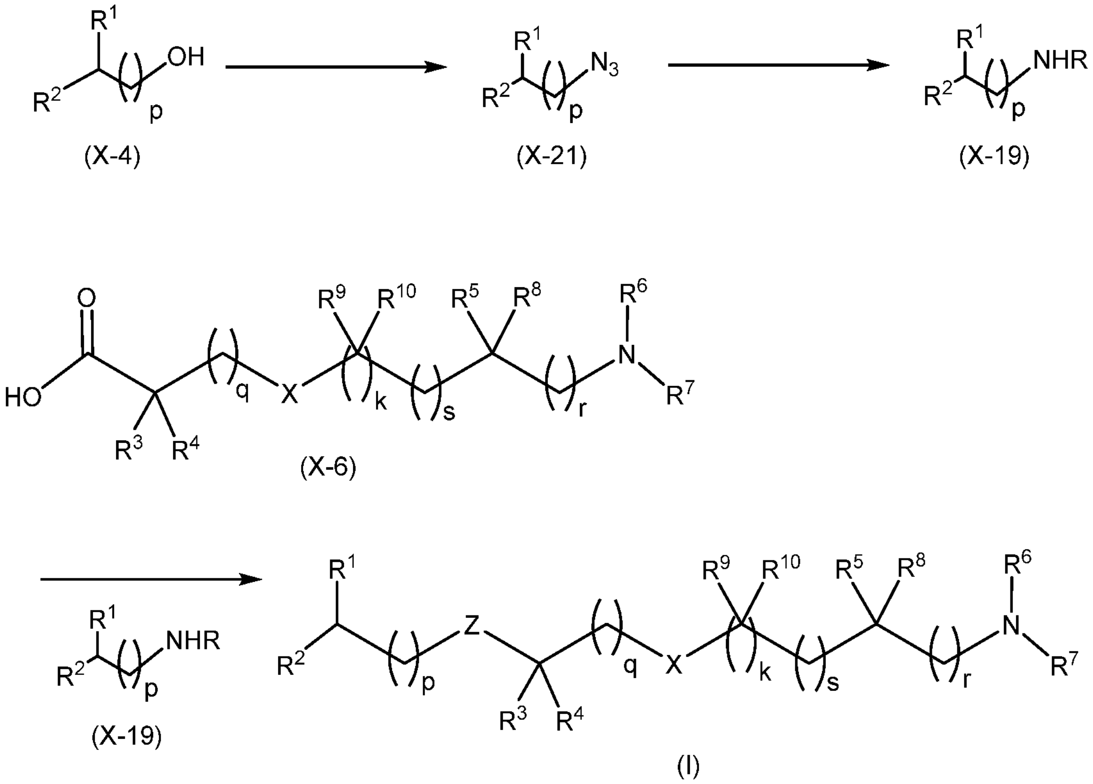

(X-4)

(X-21)

(X-19)

(X-6)

(X-19)

(I)

wherein symbols each are the same as defined above.

A compound represented by Formula (X-21) can be prepared by protecting the hydroxyl group of the compound represented by Formula (X-4) with mesyl, tosyl or the like, and followed by reacting with sodium azide or the like.

A compound represented by Formula (X-19) can be prepared by reacting the compound represented by Formula (X-21) with hydrogen gas in the presence of a metal catalyst, followed by introducing R if necessary.

This step can be performed in the same manner as the step of preparing the compound represented by Formula (I) from the compound represented by Formula (X-8) described above.

The compound represented by Formula (I) can be prepared by reacting the compound represented by Formula (X-19) with a compound represented by Formula (X-6) in the presence or absence of a base and in the presence or absence of a condensing agent.

As the base, triethylamine, DIEA and the like are exemplified.

As the condensing agent, HATU, HBTU, COMU (registered trademark) (Sigma-Aldrich Brand Chemicals and the like), PyBOP (registered trademark) (EMD Biosciences, Inc., Merck and the like) and the like are exemplified.

The reaction temperature is 0°C to reflux temperature of the solvent, preferably 20 to 50°C.

The reaction time is 1 to 48 hours, preferably 1 to 12 hours.

As the reaction solvent, dichloromethane, tetrahydrofuran, dioxane, dimethylformamide, N-methylpyrrolidone and the like are exemplified. The reaction solvent can be used alone or in combination.

A compound represented by Formula (I) wherein Z is -O-C(=O)O- or - N(R)-C(=O)O- can be, for example, prepared as follows.

[Chemical Formula 58]

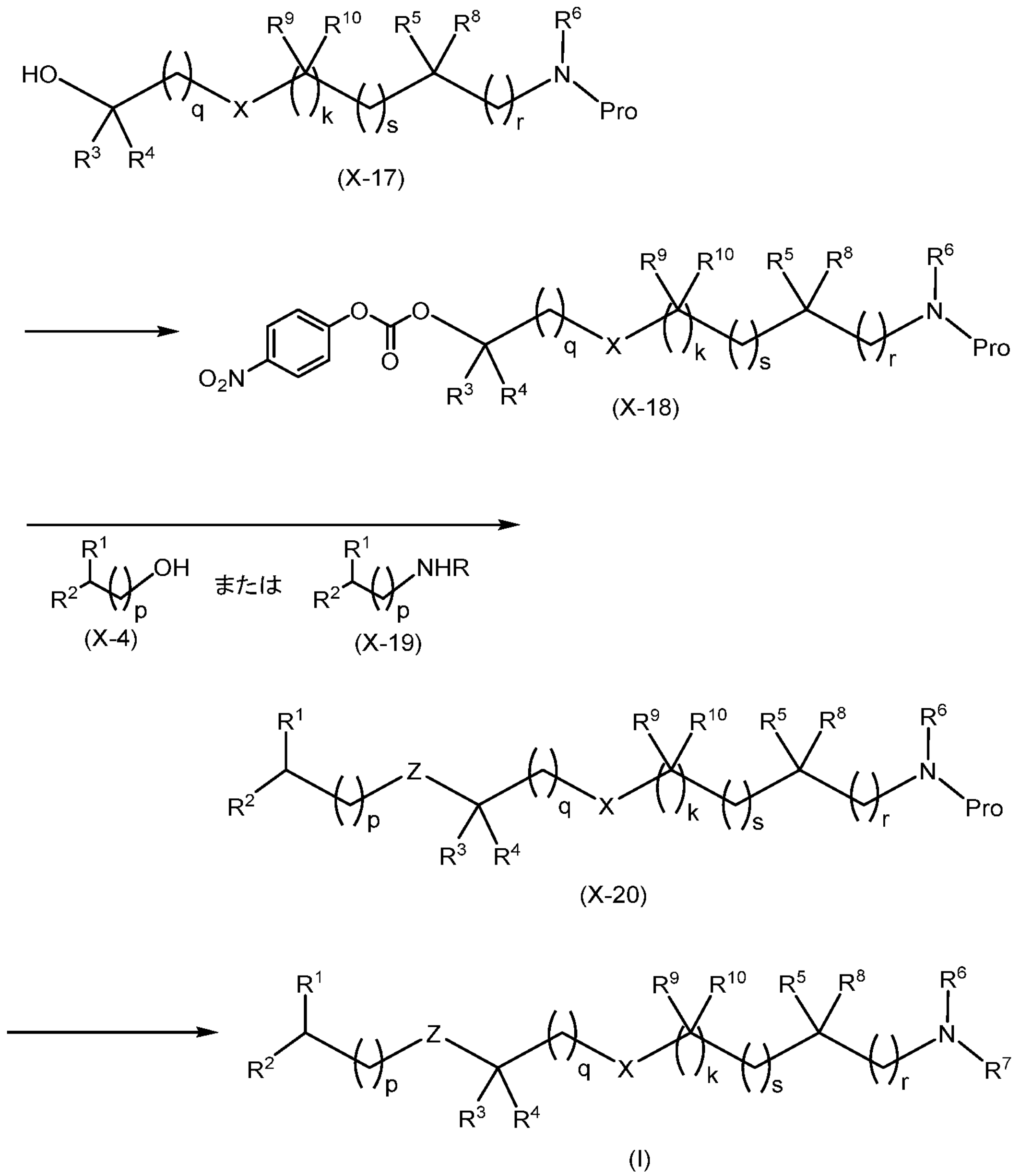


(X-17)

(X-18)

(X-4)

(X-19)

(X-20)

(I)

wherein symbols each are the same as defined above.

A compound represented by Formula (X-18) can be prepared by reacting a compound represented by Formula (X-17) with 4-nitrophenyl chloroformate or bis(4-nitrophenyl) carbonate in the presence of a base.

As the base, triethylamine, DIEA, pyridine and the like are exemplified.

The reaction temperature is 0 to 50°C, preferably 0 to 30°C.

The reaction time is 1 to 24 hours, preferably 1 to 4 hours.

As the reaction solvent, dichloromethane, dichloroethane and the like are exemplified. The reaction solvent can be used alone or in combination.

A compound represented by Formula (X-20) can be prepared by reacting the compound represented by Formula (X-17) or (X-18) with a compound represented by Formula (X-4) or (X-19) in the presence or absence of a base and in the presence or absence of a condensing agent.

As the base, triethylamine, DIEA, DMAP, pyridine and the like are exemplified.

As the condensing agent, triphosgene and the like are exemplified.

The reaction temperature is 0°C to reflux temperature of the solvent, preferably 20°C to reflux temperature of the solvent.

The reaction time is 1 to 48 hours, preferably 1 to 12 hours.

As the reaction solvent, dichloromethane, dichloroethane and the like are exemplified. The reaction solvent can be used alone or in combination.

The compound represented by Formula (I) can be prepared by deprotecting the amino protecting group of the compound represented by Formula (X-20), followed by introducing $R^7$.

This step can be performed in the same manner as the step of preparing the compound represented by Formula (I) from the compound represented by Formula (X-5) described above.

The present invention includes a pharmaceutical composition comprising the cationic lipid of the present invention (the compound represented by Formula (I) or a pharmacologically acceptable salt thereof) and nucleic acid (nucleic acid drug). For example, the pharmaceutical composition of the present invention can be prepared by using the method shown below.

A lipid solution is obtained by dissolving the cationic lipid of the present invention, neutral lipid, sterol and polyethylene glycol-modified lipid in a polar organic solvent. A mixed solution of nucleic acid and lipid is obtained by mixing a nucleic acid solution prepared with a buffer, and the lipid solution. The pharmaceutical composition of the present invention can be synthesized by replacing the polar organic solvent in the polar organic solvent with an aqueous solution such as a buffer.

In the LNP encapsulating the nucleic acid of this embodiment, the content of the cationic lipid of the present invention is preferably 10 to 100 mol%, more preferably 20 to 90 mol%content, further preferably 40 to 70 mol%.

Examples of the nucleic acid include siRNA, mRNA, miRNA, shRNA, antisense oligonucleotide, immunostimulated oligonucleotide, guide RNA (CRISPR-cas), ribozyme, expression vector and the like, preferably siRNA, mRNA and antisense oligonucleotide, more preferably siRNA or mRNA.

In the LNP of this embodiment, the nucleic acid content is preferably 0.01 to 50% by weight, more preferably 0.1 to 30 % by weight, further preferably 1 to 10% by weight.

Examples of the neutral lipid include dioleoyl phosphatidylethanolamine, palmitoyl oleoyl phosphatidylcholine, egg-yolk phosphatidylcholine, dimyristoyl phosphatidylcholine, dipalmitoyl phosphatidylcholine, distearoyl phosphatidylcholine, diarachidoyl phosphatidylcholine, dibehenoyl phosphatidylcholine, dilignoceroyl phosphatidylcholine, dioleoyl phosphatidylcholine, sphingomyelin, ceramide, dioleoyl phosphatidylglycerol, dipalmitoyl phosphatidylglycerol, phosphatidylethanolamine, dioleoyl-phosphatidylethanolamine 4-(N-maleimidomethyl)-cyclohexane-1-carboxylate and the like.

The neutral lipid content in the LNP of the present embodiment is preferably 0 to 50 mol%, more preferably 0 to 40mol%, further preferably 0 to 30mol%.

Examples of the polyethylene glycol-modified lipid include PEG2000-dimyristyl glycerol, PEG2000-dipalmitoyl glycerol, PEG2000-distearoyl glycerol, PEG5000-dimyristyl glycerol, PEG5000-dipalmitoyl glycerol, PEG5000-distearoyl glycerol, N-[(methoxypoly(ethylene glycol) 2000) carbamoyl]-1,2-dimyristyloxylpropyl-3-amine, R-3-[(ω-methoxy-poly(ethylene glycol)2000)carbamoyl]-1,2-dimyristyloxylpropyl-3-amine, PEG-diacyl glycerol, PEG-dialkyloxypropyl, PEG-phospholipid, PEG-ceramide and the like.

The content of the polyethylene glycol-modified lipid in the LNP of this embodiment is preferably 0 to 30 mol%, more preferably 0 to 20mol%, further preferably 0 to 10mol%.

Examples of the sterol include cholesterol, dihydrocholesterol, lanosterol, β-sitosterol, campesterol, stigmasterol, brassicasterol, ergocasterol, fucosterol, 3β-[N-(N',N'-dimethylaminoethyl)carbamoyl]cholesterol (DC-Chol) and the like.

The sterol content in the LNP of the present embodiment is preferably 0 to 90 mol%, more preferably 10 to 80mol%, further preferably 20 to 50mol%.

Examples of aqueous solution containing polar organic solvent include ethanol and the like.

The combination of lipid compositions in the LNP of this embodiment is not particularly limited. For example, a combination of the cationic lipid of the present invention, the neutral lipid and the sterol, or a combination of the cationic lipid of the present invention, the neutral lipid, the polyethylene glycol-modified lipid and the sterol is preferred.

The pharmaceutical composition of the present invention has any or all of the following superior characteristics:

a) The inhibitory activity for CYP enzymes (e.g., CYP1A2, CYP2C9, CYP2C19, CYP2D6, CYP3A4 and the like) is weak.
b) The pharmaceutical composition demonstrates good pharmacokinetics, such as a high bioavailability, moderate clearance and the like.
c) The pharmaceutical composition has a high metabolic stability.
d) The pharmaceutical composition has no irreversible inhibitory action against CYP enzymes (e.g., CYP3A4) when the concentration is within the range described in the present description as the measurement conditions.
e) The pharmaceutical composition has no mutagenicity.
f) The pharmaceutical composition is associated with a low cardiovascular risk.
g) The pharmaceutical composition has high encapsulation rate of the nucleic acid.

h) The stability of the particle encapsulating the nucleic acid is high.
i) The transferability to target tissue is high.
j) The pharmaceutical composition can reduce dosage.
k) The pharmaceutical composition can encapsulate less soluble API and improve solubility.
l) The pharmaceutical composition can efficiently transport the encapsulated nucleic acid to the cytoplasm.
m) The amount of constituent lipids remaining in the body is small.

In the nucleic acid contained in the pharmaceutical composition of the present invention, nucleoside(s) and internucleoside linkage(s) may be modified. The appropriately modified nucleic acid has any or all of the following characteristics compared to an unmodified nucleic acid.

a) The nucleic acid contained in the pharmaceutical composition of the present invention has high affinity for the target gene.
b) The nucleic acid contained in the pharmaceutical composition of the present invention has high resistance to nuclease.
c) The nucleic acid contained in the pharmaceutical composition of the present invention improve pharmacokinetics.
d) The nucleic acid contained in the pharmaceutical composition of the present invention makes tissue migration high.

Any administration method and formulation for a pharmaceutical composition of the present invention can be used if it is a well-known administration method and formulation in this field.
A pharmaceutical composition of the present invention may be administered in a number of ways depending upon whether local or systemic treatment is desired and upon the area to be treated. Examples of an administration method include topical (including ophthalmic, intravaginal, intrarectal, intranasal, transdermal, intraaural, intraocular, intraventricular, direct administration to organs such as intratympanic or intravesical and intratumoral), oral or parenteral. Examples of parenteral administration include intravenous injection or drip, subdermal, intraperitoneal or intramuscular injection, lung administration by aspiration or inhalation, intrathecal administration, intraventricular administration, intrathecal administration and the like, preferably intravenous injection, subcutaneous administration or intramuscular injection.
When a pharmaceutical composition of the present invention is topically administered, a formulation such as a transdermal patch, ointment, lotion, cream, gel, drop, suppository, spray, liquid, powder and the like can be used.
Examples of the composition for oral administration include powder, granule, suspension or solution dissolved in water or non-aqueous vehicle, capsule, powder,tablet and the like.
Examples of the composition for parenteral, intrathecal or intraventricular administration include sterile aqueous solutions which contain buffers, diluents and other suitable additives, and the like.
A pharmaceutical composition of the present invention can be obtained by mixing an effective amount with various pharmaceutical additives suitable for the administration form, such as excipients, binders, moistening agents, disintegrants, lubricants diluents ant the like as needed. When the composition is an injection, it together with a suitable carrier can be sterilized to obtain a composition.
Examples of the excipients include sucrose, trehalose, mannitol, sodium chloride, amino acids and the like.
Examples of the binders include methylcellulose, carboxymethylcellulose, hydroxypropyl cellulose, gelatin and polyvinylpyrrolidone.
Examples of the disintegrants include carboxymethylcellulose, sodium carboxymethylcellulose, starch, sodium alginate, agar and sodium lauryl sulfate.
Examples of the lubricants include talc, magnesium stearate and macrogol. Cacao oil, macrogol, methylcellulose or the like may be used as base materials of suppositories.
When the composition is prepared as solutions, emulsified injections or suspended injections, solubilizing agents, suspending agents, emulsifiers, stabilizers, preservatives, isotonic agents and the like which are usually used may be added as needed. For oral administration, sweetening agents, flavors or the like may be added.
Dosing of a pharmaceutical composition of the present invention is dependent on severity and responsiveness of the disease state to be treated, with the course of treatment lasting from several days to several months, or until a cure is affected or a diminution of the disease state is achieved. Persons of ordinary skill in the art can easily determine optimal dosages, dosing methodologies and repetition rates.
For example, optimal dosing schedules for the pharmaceutical composition comprising the compound and siRNA described herein can be calculated from relevant biomarkers in vivo. Although optimal dosages vary according to the relative potency of the conjugate using the present invention, they can generally be calculated based on the effects of in vitro and in vivo animal studies. For example, the molecular weight of the nucleic acid drug (derived from the sequence and chemical structure) and the experimentally derived effective dose are provided, doses shown as mg/kg or mg/head are calculated according to the usual method.

In order to improve the promotion of the target protein expression or the suppression of the target gene expression of a nucleic acid drug, the pharmaceutical composition of the present invention can be used together with an appropriate nucleic acid drug for the prevention or treatment of various diseases in which the effect of the nucleic acid drug is expected.

[EXAMPLES]

The present invention will be described in more detail with reference to, but not limited to, the following Examples and Reference Examples.
In this description, meaning of each abbreviation is as follows:

Bn: benzyl
Boc: tert-butoxycarbonyl
DCC: N,N'-Dicyclohexylcarbodiimide
DIC: N,N'-Diisopropylcarbodiimide
DIEA: N, N-diisopropylethylamine
DMAP: 4-dimethylaminopyridine
DMEM: Dulbecco's Modified Eagle's Medium
DMF: N, N-dimethylformamide
DMG-PEG: 1,2-dimyristoyl-rac-glycero-3-methylpolyoxyethylene
DMSO: dimethyl sulfoxide
DSPC: distearoylphosphatidylcholine
EDC: 1-[3-(dimethylamino)propyl]-3-ethylcarbodiimide
GAPDH: glyceraldehyde-3-phosphate dehydrogenase
Hprt1: hypoxanthine-guanine phosphoribosyltransferase
LHMDS: lithium bis(trimethylsilyl)amide
NMP: N-methylpyrrolidone
OVA: ovalbumin
PyBOP: (Benzotriazol-1-yloxy)tripyrrolidinophosphonium hexafluorophosphate SOD1: Cu/Zn superoxide dismutase 1
TBAF: tetrabutylammonium fluoride
TBAI: tetrabutylammonium iodide
TBDPS: tert-butyldiphenylsilyl
TBS: tert-butyldimethylsilyl
TMS: trimethylsilyl

NMR analysis of each example was performed by 400 MHz using DMSO-$d_6$ or $CDCl_3$. Sometimes not all the peaks detected are shown in NMR data.
UPLC analysis was performed under the following conditions.

(Method 1)

Column: ACQUITY UPLC (registered trademark) BEH C18 (1.7 pm, i.d.2.1 x 50 mm) (Waters)
Flow rate: 0.8 mL/min
PDA detection wavelength: 254 nm (range of detection 210-500 nm)
Mobile phase: [A] an aqueous solution containing 0.1% formic acid, [B] an acetonitrile solution containing 0.1% formic acid
Gradient: linear gradient of 5% to 100% solvent [B] was performed for 3.5 minutes, and then 100% solvent [B] was maintained for 0.5 minutes.

(Method 2)

Column: Xbridge Protein BEH C4 (3.5 pm, i.d.2.1 x 50 mm) (Waters)
Flow rate: 0.8 mL/min
PDA detection wavelength: 254 nm (range of detection 210-500 nm)
Mobile phases: [A] 10 mM aqueous ammonium carbonate solution, [B] acetonitrile
Gradient: linear gradient of 60% to 100% solvent [B] was performed for 3.5 minutes, and then 100% solvent [B] was maintained for 0.5 minutes.

(Method 3)

Column: Xbridge Protein BEH C4 (3.5 pm, i.d.2.1 x 50 mm) (Waters)
Flow rate: 0.8 mL/min
PDA detection wavelength: 254 nm (range of detection 210-500 nm)
Mobile phases: [A] 10 mM aqueous ammonium carbonate solution, [B] acetonitrile
Gradient: linear gradient of 5% to 100% solvent [B] was performed for 3.5 minutes, and then 100% solvent [B] was maintained for 0.5 minutes.

(Method 4)

Column: ACQUITY UPLC (registered trademark) BEH C18 (1.7 pm, i.d.2.1 x 50 mm) (Waters)
Flow rate: 0.8 mL/min
PDA detection wavelength: 254 nm (range of detection 210-500 nm)
Mobile phases: [A] 10 mM aqueous ammonium carbonate solution, [B] acetonitrile
Gradient: linear gradient of 5% to 100% solvent [B] was performed for 3.5 minutes, and then 100% solvent [B] was maintained for 0.5 minutes.

In this description, the description of MS (m/z) indicates the value observed by mass spectrometry.

Synthesis of the compounds of the present invention

[Example 1]

Synthesis of Compound I-1

[Chemical Formula 59]

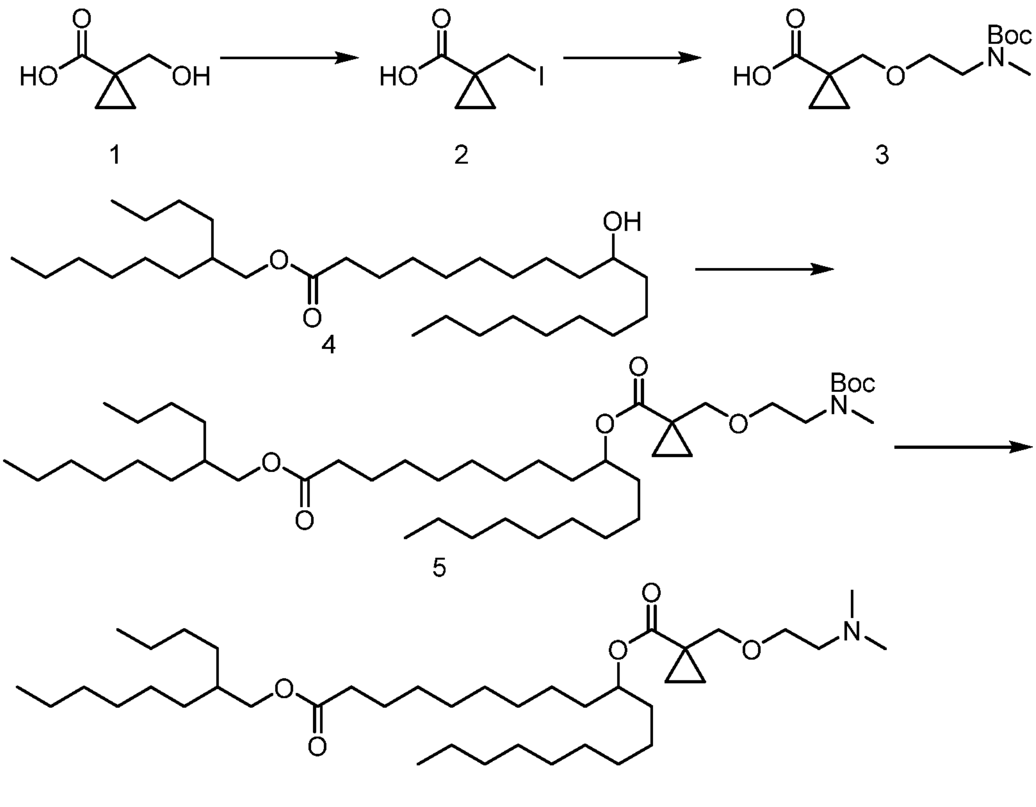

1

2

3

4

5

I-1

Step 1

Hydriodic Acid (10.6 mL, 141 mmol) was added to Compound 1 (3.27 g, 28.2 mmol), and the mixture was stirred at 50 °C for 2 hours. Water was added to the reaction mixture and the mixture was extracted with ethyl acetate. The organic layer was dried over anhydrous magnesium sulfate and the solvent was distilled off under reduced pressure. The obtained residue was purified by silica-gel column chromatography (n-hexane-ethyl acetate) to give Compound 2 (3.56 g, yield 56%).
$^1$H-NMR ($CDCl_3$) δ: 1.07 (t, 2H), 1.73 (t, 2H), 3.41 (s, 2H).

Step 2

To a solution of sodium hydride (531 mg, 13.3 mmol) in tetrahydrofuran (15 mL) were added a solution of N-Boc-N-methyl-2-aminoethanol (1.55 g, 4.42 mmol) in tetrahydrofuran (3.5 mL) and Compound 2 (1.0 g, 4.42 mmol) under ice-cooling and the mixture was stirred at room temperature for 3 hours. Water was added to the reaction mixture and the organic solvent was distilled off under reduced pressure. The resulting aqueous solution was washed with chloroform. A saturated aqueous citric acid solution was added to the aqueous layer to adjust the pH to 2, and then the aqueous layer was extracted with chloroform. The organic layer was washed with water and dried over anhydrous magnesium sulfate. The solvent was distilled off under reduced pressure to give Compound 3 (672 mg, yield 56%).
$^1$H-NMR ($CDCl_3$) δ: 0.96 (t, 2H), 1.33 (t, 2H), 1.44 (s, 9H), 2.90 (s, 3H), 3.40 (brs, 2H), 3.60 (brs, 4H).

Step 3

To a solution of Compound 4 (see WO2016/104580, 50 mg, 0.104 mmol) in dichloromethane (3.5 mL) were added Compound 3 (28 mg, 0.104 mmol), 2-methyl-6-nitrobenzoic anhydride (107 mg, 0.311 mmol), DIEA (0.11 mL, 0.621 mmol) and DMAP (76 mg, 0.621 mmol) and the mixture was heated under reflux for 5 hours. Saturated aqueous solution of sodium hydrogen carbonate was added to the reaction mixture and the mixture was extracted with chloroform. The solvent was distilled off under reduced pressure and the obtained residue was purified by silica-gel column chromatography (n-hexane-ethyl acetate) to give Compound 5 (54 mg, yield 71%).
$^1$H-NMR ($CDCl_3$) δ: 0.88 (m, 11H), 1.25-1.61 (m, 58H), 2.29 (t, 2H), 2.89 (s, 3H), 3.36 (brs, 2H), 3.56 (brs, 2H), 3.62 (s, 2H), 3.96 (d, 2H), 4.85 (m, 1H).
ESI-MS (m/z): 739 (M+1).

Step 4

To Compound 5 (150 mg, 0.203 mmol) was added hydrochloric acid-1,4-dioxane solution (1.52 mL, 6.08 mmol) and the mixture was stirred at room temperature for 2 hours. The solvent was distilled off under reduced pressure. To a solution of the obtained residue in dichloromethane (3.9 mL) were added 36% formaldehyde solution (0.05 mL, 0.711 mmol) and sodium triacetoxyborohydride (301 mg, 1.42 mmol) and the mixture was stirred at room temperature for 1 hour. Saturated aqueous solution of sodium hydrogen carbonate was added to the reaction mixture and the mixture was extracted with chloroform. The solvent was distilled off under reduced pressure and the obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound I-1 (93 mg, yield 70%) as a clear oil.
$^1$H-NMR ($CDCl_3$) δ: 0.88 (m, 11H), 1.17-1.28 (m, 42H), 1.49 (m, 4H), 1.60 (m, 12H), 2.25-2.31 (m, 8H), 2.49 (t, 2H), 3.56 (t, 2H), 3.62 (s, 2H), 3.97 (d, 2H), 4.85 (m, 1H).
ESI-MS (m/z): 653 (M+1).

[Example 2]

Synthesis of Compound I-2

[Chemical Formula 60]

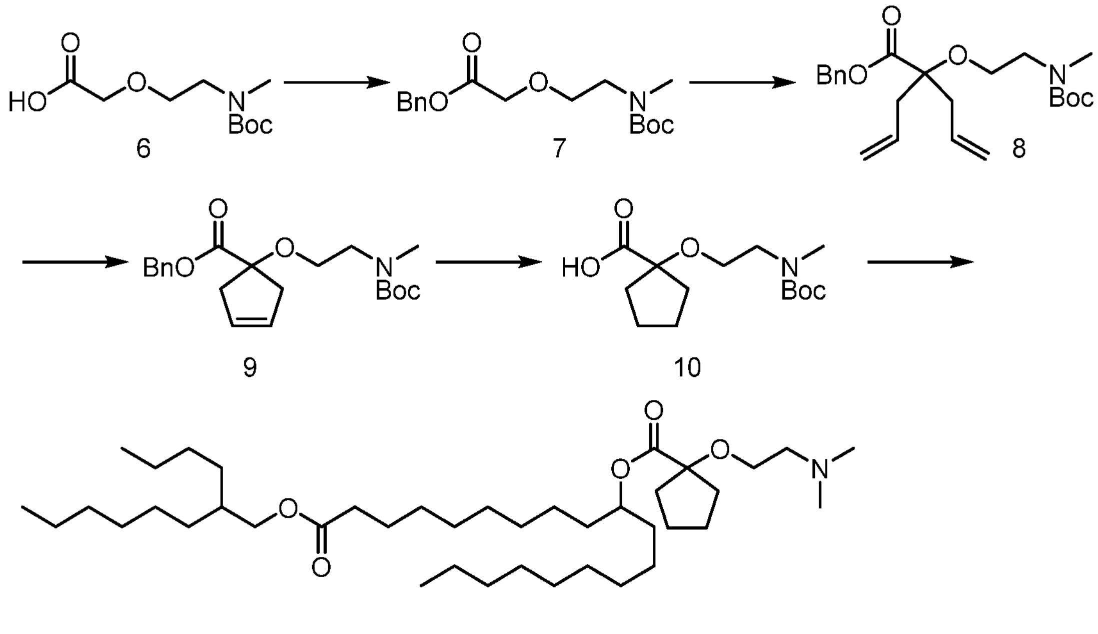

6 7 8 9 10

I-2

Step 5

To a solution of Compound 6 (973 mg, 4.17 mmol) in DMF (20.9 mL) were added Bn bromide (1.04 mL, 8.76 mmol) and sodium hydrogen carbonate (1.12 g, 13.4 mmol) under ice-cooling and the mixture was stirred for 40 hours. To the reaction mixture were added Bn bromide (0.30 mL, 2.53 mmol) and sodium hydrogen carbonate (0.48 g, 5.72 mmol) and the mixture was stirred at 50°C for 5 hours. Water was added to the reaction mixture and the mixture was extracted with ethyl acetate. The organic layer was washed with water and the solvent was distilled off under reduced pressure. The obtained residue was purified by silica-gel column chromatography (n-hexane-ethyl acetate) to give Compound 7 (1.14 g, yield 85%).
$^1$H-NMR ($CDCl_3$) δ: 1.44 (s, 9H), 2.92 (s, 3H), 3.42 (brs, 2H), 3.66 (brs, 2H), 4.13 (s, 2H), 5.19 (s, 2H), 7.31-7.36 (m, 5H).

Step 6

To a solution of Compound 7 (371 mg, 1.15 mmol) in tetrahydrofuran (11.5 mL) were added allyl iodide (2.1 mL, 22.9 mmol) and LHMDS-tetrahydrofuran solution (2.29 mL, 2.29 mmol) at -78°C and the mixture was stirred for 1 hour. To the reaction mixture was added LHMDS-tetrahydrofuran solution (1.15 mL, 1.15 mmol) and the mixture was stirred for 1 hour. Saturated aqueous solution of ammonium chloride was added to the reaction mixture and the mixture was extracted with ethyl acetate. The organic layer was distilled off under reduced pressure. The obtained residue was purified by silica-gel column chromatography (n-hexane-ethyl acetate) to give Compound 8 (197 mg, yield 43%).
$^1$H-NMR ($CDCl_3$) δ: 1.44 (s, 9H), 2.55 (m, 4H), 2.89 (s, 3H), 3.36 (brs, 2H), 3.54 (brs, 2H), 5.05 (m, 4H), 5.14 (s, 2H), 5.69 (m, 2H), 7.31-7.36 (m, 5H).
ESI-MS (m/z): 404 (M+1).

Step 7

To a solution of Compound 8 (96 mg, 0.24 mmol) in dichloromethane (2.4 mL) was added second generation Grubbs catalyst (Registered Trademark) (Sigma-Aldrich Brand Chemicals) (10.1 mg, 0.012 mmol) and the mixture was heated under reflux for 2 hours. The reaction mixture was distilled off under reduced pressure. The obtained residue was purified by silica-gel column chromatography (n-hexane-ethyl acetate) to give Compound 9 (85 mg, yield 95%).
$^1$H-NMR ($CDCl_3$) δ: 1.44 (s, 9H), 2.61 (s, 1H), 2.65 (s, 1H), 2.86-2.92 (m, 5H), 3.33 (brs, 2H), 3.49 (brs, 2H), 5.20 (s, 2H), 5.64 (s, 2H), 7.31-7.36 (m, 5H). ESI-MS (m/z): 376 (M+1).

Step 8

To a solution of Compound 9 (85 mg, 0.23 mmol) in ethyl acetate (4.5 mL) was added Pd-C (12.1 mg, 0.011 mmol) and the mixture was stirred under hydrogen atmosphere at room temperature for 3 hours. The reaction mixture was filtered through Celite (Registered Trademark) (KANTO CHEMICAL). The solvent was distilled off under reduced pressure to give Compound 10 (58 mg, yield 89%).
ESI-MS (m/z): 288 (M+1).

Step 9

Compound I-2 (148 mg, yield 81%) was obtained as a clear oil in the same manner as in Step 3 and 4 using Compound 10 instead of Compound 3 in Step 3.
$^1$H-NMR ($CDCl_3$) δ: 0.88 (m, 9H), 1.20-1.35 (m, 41H), 1.50-1.65 (m, 13H), 1.65-1.80 (m, 4H), 1.90-2.05 (m, 4H), 2.25-2.31 (m, 8H), 2.52 (t, 2H), 3.47 (t, 2H), 3.97 (d, 2H), 4.91 (m, 1H).
ESI-MS (m/z): 667 (M+1).

[Example 3]

Synthesis of Compound I-3

[Chemical Formula 61]

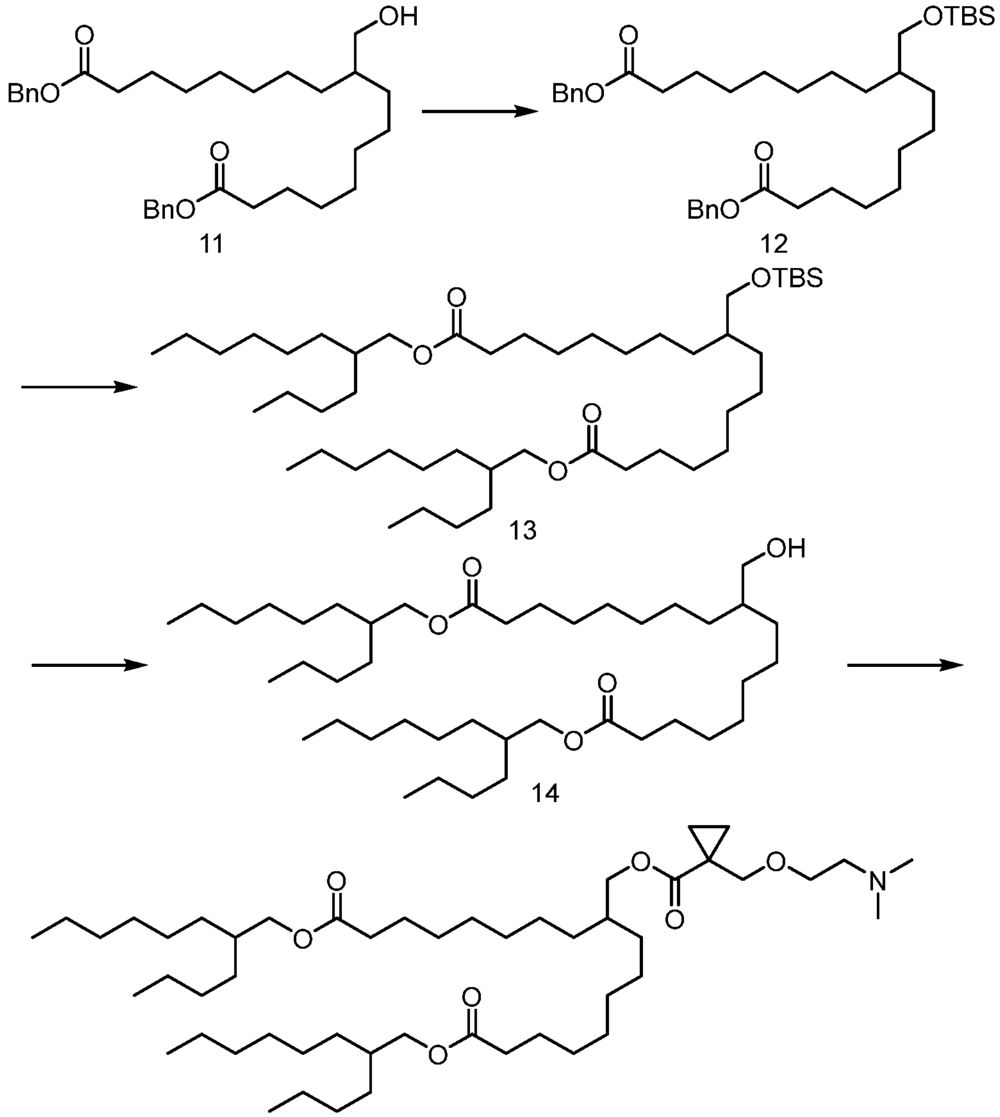

11

12

13

14

I-3

Step 10

To a solution of Compound 11 (see WO2017/222016, 10.7 g, 21.0 mmol) in dichloromethane (42 mL) were added imidazole (3.00 g, 44.0 mmol) and TBS chloride (3.47 g, 23.1 mmol) and the mixture was stirred at room temperature for 1.5 hour. Ethyl acetate was added to the reaction mixture and the mixture was washed sequentially with 2 mol/L hydrochloric acid, water, saturated aqueous solution of sodium hydrogen carbonate, and saturated aqueous solution of sodium chloride The organic layer was dried over anhydrous magnesium sulfate. The solvent was distilled off under reduced pressure to give Compound 12 (13.2 g, yield 100%).

$^1$H-NMR ($CDCl_3$) $\delta$: 0.00 (s, 3H), 0.02 (s, 3H), 0.88 (s, 9H), 1.17-1.38 (m, 21H), 1.64 (t, 4H), 2.35 (t, 4H), 3.44 (d, 2H), 5.11 (s, 4H), 7.30-7.38 (m, 10H). ESI-MS (m/z): 625 (M+1).

Step 11

To a solution of Compound 12 (12.9 g, 20.6 mmol) in tetrahydrofuran (41 mL) was added Pd-C (1.10 g, 1.03 mmol) and the mixture was stirred under hydrogen atmosphere at room temperature for 6 hours. The reaction mixture was filtered through Celite and the solvent was distilled off under reduced pressure. To a solution of the obtained residue in dichloromethane (21 mL) were added 2-butyl-1-octanol (4.6 mL, 20.6 mmol), 2-methyl-6-nitrobenzoic anhydride (10.6 g, 30.9 mmol), DIEA (10.8 mL, 61.8 mmol) and DMAP (252 mg, 2.06 mmol) and the mixture was stirred at room temperature for 17 hours. Saturated aqueous solution of sodium hydrogen carbonate was added to the reaction mixture

and the mixture was extracted with ethyl acetate. The organic layer was washed with water. The solvent was distilled off under reduced pressure and the obtained residue was purified by silica-gel column chromatography (n-hexane-ethyl acetate) to give Compound 13 (2.52 g, yield 31%).

$^1$H-NMR ($CDCl_3$) $\delta$: 0.00 (s, 3H), 0.02 (s, 3H), 0.87 (m, 21H), 1.17-1.39 (m, 55H), 1.63 (m, 6H), 2.29 (t, 4H), 3.45 (d, 2H), 3.97 (d, 4H).

ESI-MS (m/z): 782 (M+1).

Step 12

To a solution of Compound 13 (2.52 g, 3.23 mmol) in tetrahydrofuran (16 mL) were added acetic acid (1.84 mL, 32.3 mmol) and TBAF in tetrahydrofuran (12.9 mL, 12.9 mmol) and the mixture was stirred at room temperature for 17 hours. Ethyl acetate was added to the reaction mixture and the mixture was washed sequentially with saturated aqueous solution of sodium hydrogen carbonate and water. The organic layer was distilled off under reduced pressure and the obtained residue was purified by silica-gel column chromatography (n-hexane-ethyl acetate) to give Compound 14 (1.87 g, yield 87%).

$^1$H-NMR ($CDCl_3$) $\delta$: 0.88 (m, 12H), 1.11-1.44 (m, 55H), 1.62 (m, 6H), 2.30 (t, 4H), 3.53 (t, 2H), 3.97 (d, 4H).

ESI-MS (m/z): 668 (M+1).

Step 13

Compound I-3 (105 mg, total yield 56%) was obtained as a clear oil in the same manner as in Step 3 and 4 using Compound 14 instead of Compound 4 in Step 3.

$^1$H-NMR ($CDCl_3$) $\delta$: 0.88 (m, 14H), 1.22-1.27 (m, 55H), 1.61 (m, 6H), 2.25-2.31 (m, 10H), 2.50 (t, 2H), 3.56 (t, 2H), 3.61 (s, 2H), 3.96 (m, 6H).

ESI-MS (m/z): 837 (M+1).

[Example 4]

Synthesis of Compound I-4

[Chemical Formula 62]

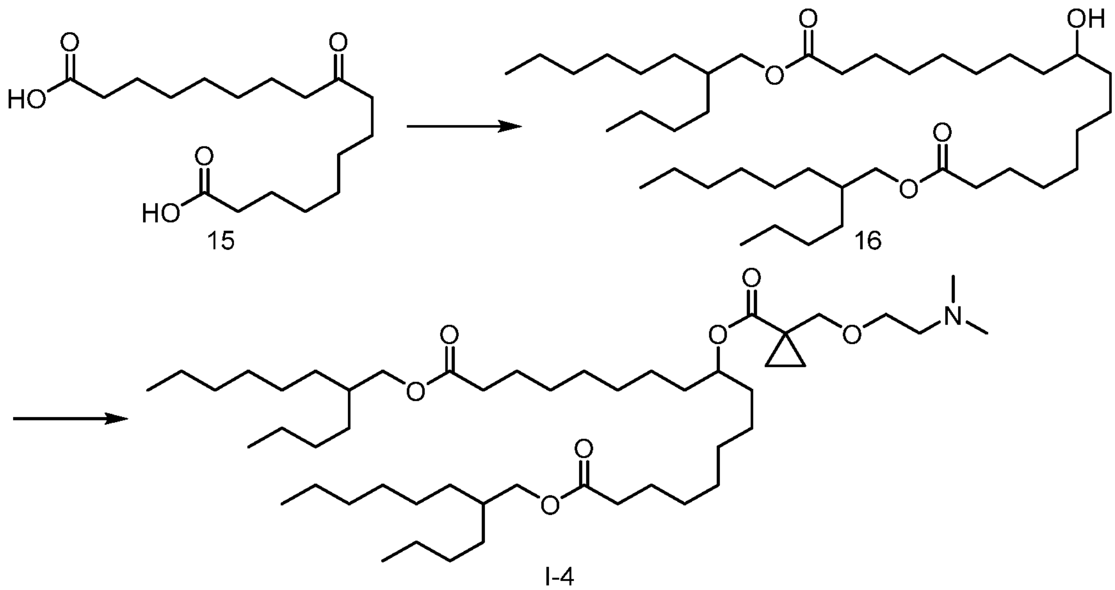

15

16

I-4

Step 14

To a solution of Compound 15 (300 mg, 0.95 mmol) in dichloromethane (1.9 mL) were added 2-butyl-1-octanol (0.64 mL, 2.86 mmol), 2-methyl-6-nitrobenzoic anhydride (985 mg, 2.86 mmol), DIEA (1.3 mL, 7.63 mmol) and DMAP (23 mg, 0.19 mmol) and the mixture was stirred at room temperature for 6 hours. Saturated aqueous solution of sodium

hydrogen carbonate was added to the reaction mixture and the mixture was extracted with ethyl acetate. The organic layer was washed with water. The solvent was distilled off under reduced pressure and the obtained residue was purified by silica-gel column chromatography (n-hexane-ethyl acetate). The solvent in the fractions containing the desired product was distilled off under reduced pressure. To a solution of the obtained residue in 50%tetrahydrofuran-methanol (9.6 mL) was added sodium borohydride (72 mg, 1.91 mmol) and the mixture was stirred at 0°C for 2 hours. Saturated aqueous solution of sodium hydrogen carbonate was added to the reaction mixture and the mixture was extracted with ethyl acetate. The organic layer was distilled off under reduced pressure and the obtained residue was purified by silica-gel column chromatography (n-hexane-ethyl acetate) to give Compound 16 (530 mg, yield 85%).
$^1$H-NMR ($CDCl_3$) $\delta$: 0.88 (m, 12H), 1.27-1.31 (m, 46H), 1.37-1.42 (m, 6H), 1.61 (m, 6H), 2.30 (t, 4H), 3.57 (brs, 1H), 3.97 (d, 4H).
ESI-MS (m/z): 654 (M+1).

Step 15

Compound I-4 (131 mg, total yield 69%) was obtained as a clear oil in the same manner as in Step 3 and 4 using Compound 16 instead of Compound 4 in Step 3.
$^1$H-NMR ($CDCl_3$) $\delta$: 0.88 (m, 14H), 1.19-1.28 (m, 50H), 1.49 (m, 4H), 1.59 (m, 6H), 2.25-2.31 (m, 10H), 2.49 (t, 2H), 3.58 (t, 2H), 3.62 (s, 2H), 3.96 (d, 4H), 4.84 (m, 1H).
ESI-MS (m/z): 823 (M+1).

[Example 5]

Synthesis of Compound I-5

[Chemical Formula 63]

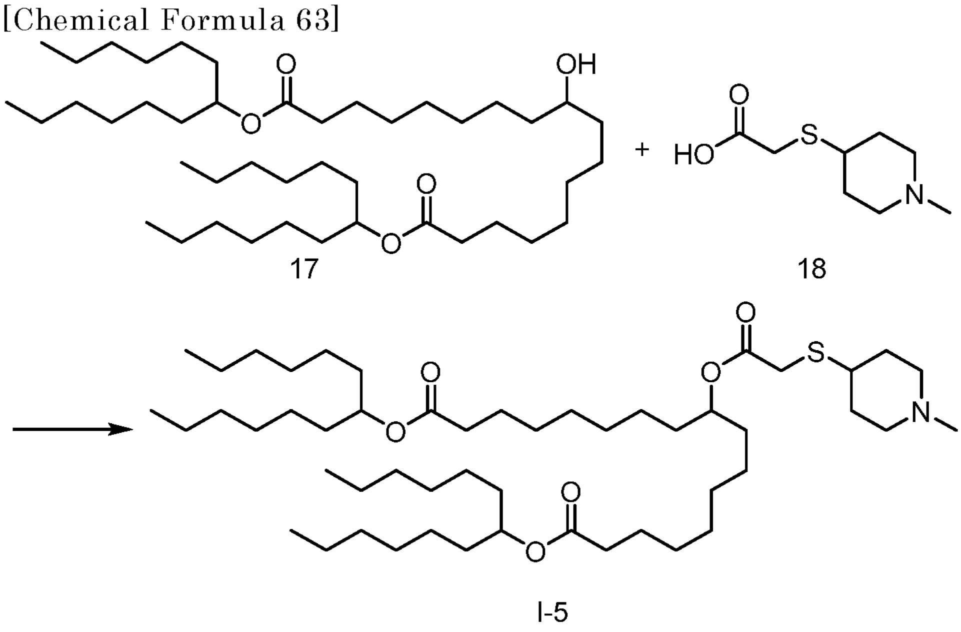

17

18

I-5

Step 16

Compound 17 (485 mg, yield 75%) was obtained in the same manner as in Step 14 using 7-tridecanol instead of 2-butyl-1-octanol in Step 14.
$^1$H-NMR ($CDCl_3$) $\delta$: 0.88 (m, 12H), 1.20-1.35 (m, 49H), 1.35-1.53 (m, 14H), 1.62 (m, 4H), 2.28 (t, 4H), 3.58 (brs, 1H), 4.87 (m, 2H).
ESI-MS (m/z): 682 (M+1).

Step 17

To a solution of Compound 17 (160 mg, 0.235 mmol) in NMP (1.2 mL) were added Compound 18 (67 mg,

0.352 mmol), 2-methyl-6-nitrobenzoic anhydride (162 mg, 0.470 mmol), DIEA (0.16 mL, 0.940 mmol) and DMAP (5.7 mg, 0.047 mmol) and the mixture was stirred at room temperature for 18 hours. To the reaction mixture were added Compound 18 (33 mg, 0.176 mmol), 2-methyl-6-nitrobenzoic anhydride (81 mg, 0.235 mmol) and DIEA (0.08 mL, 0.470 mmol) and the mixture was stirred at room temperature for 3 hours. Saturated aqueous solution of sodium hydrogen carbonate was added to the reaction mixture and the mixture was extracted with ethyl acetate. The organic layer was washed with water and distilled off under reduced pressure The obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound I-5 (86 mg, yield 43%) as a clear oil.
$^1$H-NMR ($CDCl_3$) δ: 0.88 (t, 12H), 1.20-1.35 (m, 49H), 1.45-1.70 (m, 25H), 2.03 (m, 4H), 2.27 (m, 7H), 2.79 (d, 3H), 3.23 (s, 2H), 4.86 (m, 3H).
ESI-MS (m/z): 853 (M+1).

[Example 6]

Synthesis of Compound I-6

[Chemical Formula 64]

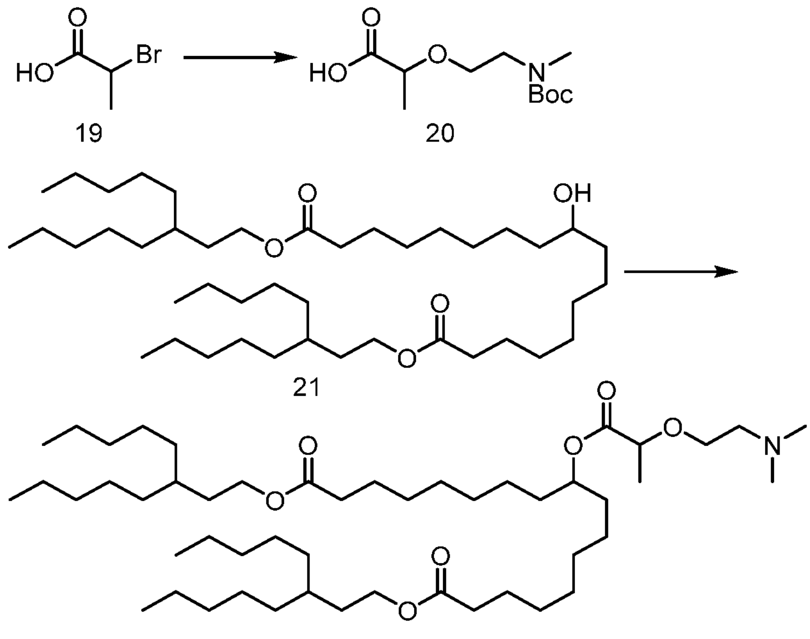

19

20

21

I-6

Step 18

Compound 20 (3.24 g, yield 100%) was obtained in the same manner as in Step 2 using Compound 19 instead of Compound 2 in Step 2.
$^1$H-NMR ($CDCl_3$) δ: 1.44 (m, 12H), 2.92 (s, 3H), 3.38 (brs, 2H), 3.63 (brs, 2H), 4.03 (q, 1H).

Step 19

Compound 21 (0.541 g) was obtained as a clear oil in the same manner as in Step 14 using 3-pentyl-1-octanol instead of 2-butyl-1-octanol in Step 14. $^1$H-NMR ($CDCl_3$) δ: 0.87 (t, 12H), 1.25-1.63 (m, 63H), 2.26 (t, 4H), 3.57 (brs, 1H), 4.06 (t, 4H).
ESI-MS (m/z): 682 (M+1).

Step 20

Compound I-6 (460 mg, total yield 75%) was obtained as a clear oil in the same manner as in Step 3 and 4 using Compound 21 instead of Compound 4 and Compound 20 instead of Compound 3 in Step 3.
$^1$H-NMR ($CDCl_3$) δ: 0.88 (t, 12H), 1.20-1.35 (m, 48H), 1.41 (d, 5H), 1.50-1.65 (m, 23H), 2.27 (m, 10H), 2.54 (t, 2H), 3.46 (m, 1H), 3.68 (m, 1H), 3.96 (q, 1H), 4.08 (t, 4H), 4.92 (m, 1H).
ESI-MS (m/z): 825 (M+1).

[Example 7]

Synthesis of Compound I-7

[Chemical Formula 65]

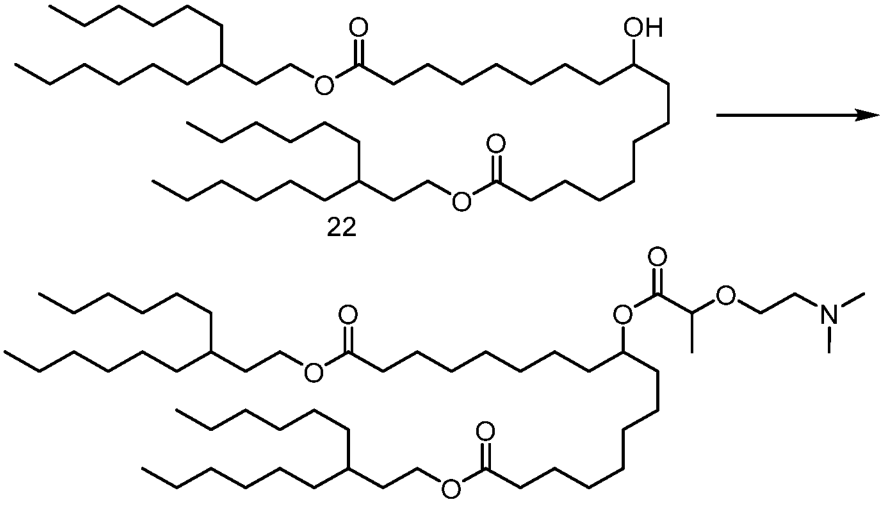

22

I-7

Step 21

Compound 22 (1.44 g, yield 82%) was obtained in the same manner as in Step 14 using 3-hexyl-1-nonanol instead of 2-butyl-1-octanol in Step 14. $^1$H-NMR ($CDCl_3$) δ: 0.88 (m, 12H), 1.20-1.50 (m, 66H), 1.52-1.68 (m, 10H), 2.28 (t, 4H), 3.57 (brs, 1H), 4.08 (t, 4H).

Step 22

Compound I-7 (351 mg, total yield 59%) was obtained as a clear oil in the same manner as in Step 20 using Compound 22 instead of Compound 21 in Step 20.
$^1$H-NMR ($CDCl_3$) δ: 0.88 (t, 14H), 1.20-1.45 (m, 66H), 1.48-1.66 (m, 15H), 2.27 (m, 10H), 2.53 (t, 2H), 3.46 (m, 1H), 3.69 (m, 1H), 3.96 (q, 1H), 4.08 (t, 4H), 4.91 (m, 1H).

[Example 8]

Synthesis of Compound I-8

[Chemical Formula 66]

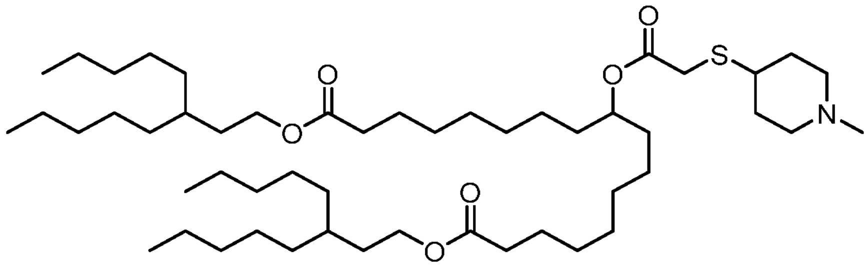

I-8

Step 23

Compound I-8 (539 mg, yield 86%) was obtained as a clear oil in the same manner as in Step 17 using Compound 21 instead of Compound 17 in Step 17.
$^1$H-NMR ($CDCl_3$) δ: 0.88 (t, 12H), 1.20-1.42 (m, 52H), 1.48-1.68 (m, 22H), 2.00 (m, 4H), 2.28 (m, 7H), 2.79 (brs, 3H), 3.23 (s, 2H), 4.08 (t, 4H), 4.88 (m, 1H). ESI-MS (m/z): 853 (M+1).

[Example 9]

Synthesis of Compound I-9

[Chemical Formula 67]

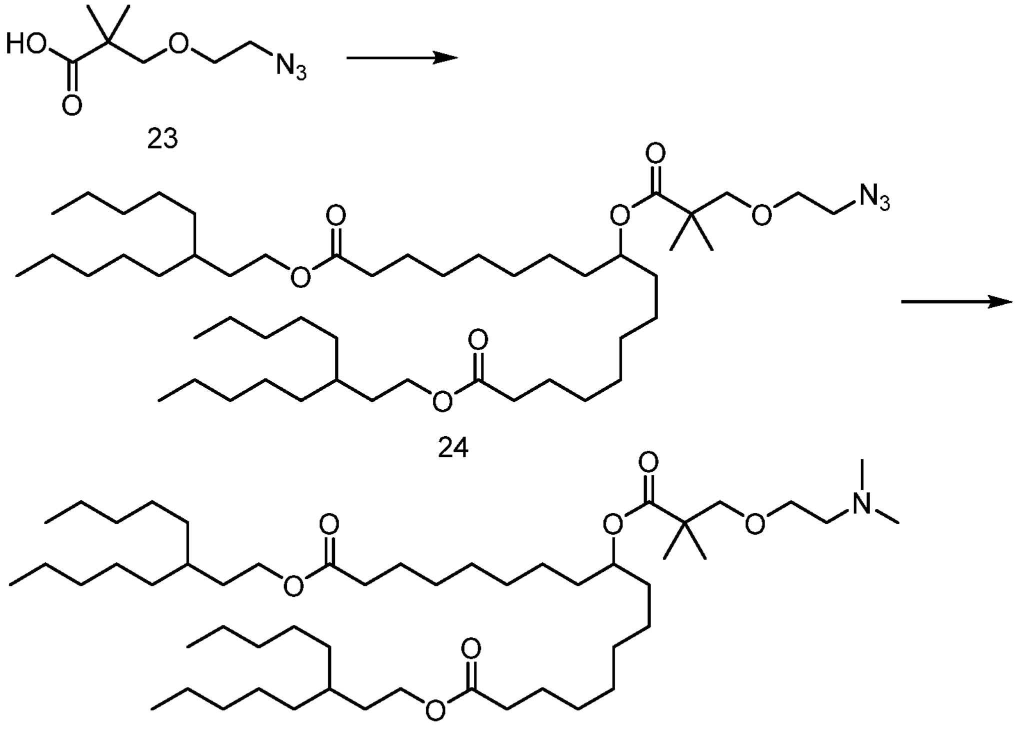

23

24

I-9

Step 24

Compound 23 (856 mg, yield 16%) was obtained in the same manner as in Step 2 using 2, 2-dimethyl-3-iodopropionic acid instead of Compound 2 and 2-azidoethanol instead of N-Boc-N-methyl-2-aminoethanol in Step 2.

$^1$H-NMR ($CDCl_3$) δ: 1.24 (s, 6H), 3.35 (t, 2H), 3.52 (s, 2H), 3.66 (t, 2H). ESI-MS (m/z): 188 (M+H).

Step 25

Compound 24 (188 mg, yield 89%) was obtained in the same manner as in Step 3 using Compound 21 instead of Compound 4 and Compound 23 instead of Compound 3 in Step 3.
$^1$H-NMR ($CDCl_3$) δ: 0.88 (t, 14H), 1.15-1.42 (m, 61H), 1.48-1.65 (m, 23H), 2.27 (t, 4H), 3.31 (t, 2H), 3.49 (s, 2H), 3.61 (t, 2H), 4.08 (t, 4H), 4.85 (m, 1H). ESI-MS (m/z): 868 (M+18).

Step 26

To a solution of Compound 24 (188 mg, 0.22 mmol) in tetrahydrofuran (5.4 mL) were added Pd-C (235 mg, 0.22 mmol) and hydrochloric acid-1,4-dioxane solution (0.28 mL, 1.11 mmol) and the mixture was stirred under hydrogen atmosphere at room temperature for 3 hours. The reaction mixture was filtered through Celite and the solvent was distilled off under reduced pressure. To a solution of the obtained residue in tetrahydrofuran (5.4 mL) were added 36% formaldehyde solution (0.09 mL, 1.11 mmol) and sodium triacetoxyborohydride (469 mg, 2.21 mmol) and the mixture was stirred at room temperature for 1 hour. Saturated aqueous solution of sodium hydrogen carbonate was added to the reaction mixture and the mixture was extracted with chloroform. The solvent was distilled off under reduced pressure and the obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound I-9 (71 mg, yield 38%) as a clear oil.
$^1$H-NMR ($CDCl_3$) δ: 0.88 (t, 12H), 1.17 (s, 6H), 1.20-1.45 (m, 51H), 1.45-1.65 (m, 42H), 2.27 (m, 10H), 2.48 (t, 2H), 3.42 (s, 2H), 3.52 (t, 2H), 4.08 (t, 4H), 4.84 (m, 1H).
ESI-MS (m/z): 853 (M+1).

[Example 10]

Synthesis of Compound I-10

[Chemical Formula 68]

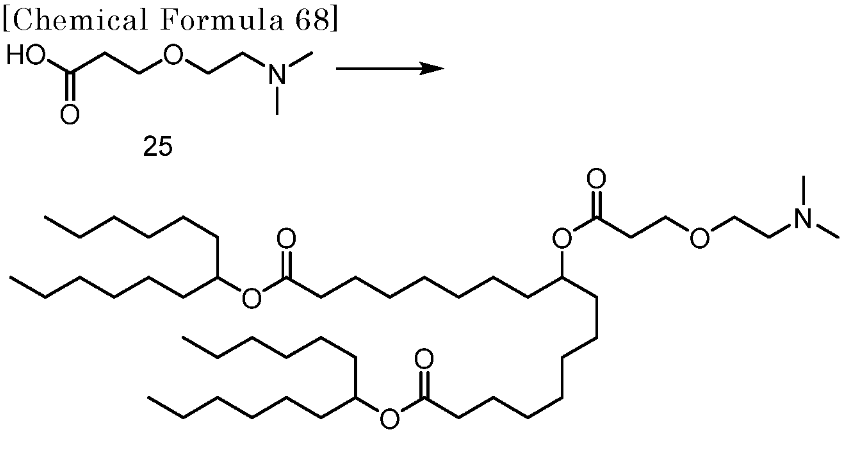

25

I-10

Step 27

Compound I-10 (162 mg, yield 67%) was obtained as a clear oil in the same manner as in Step 17 using Compound 25 instead of Compound 18 in Step 17.
$^1$H-NMR ($CDCl_3$) δ: 0.88 (t, 12H), 1.25-1.35 (m, 50H), 1.45-1.55 (m, 12H), 1.55-1.65 (m, 8H), 2.27 (m, 10H), 2.48 (t, 2H), 2.58 (t, 2H), 3.54 (t, 2H), 3.72 (t, 2H), 4.86 (m, 3H).

[Example 11]

Synthesis of Compound I-11

[Chemical Formula 69]

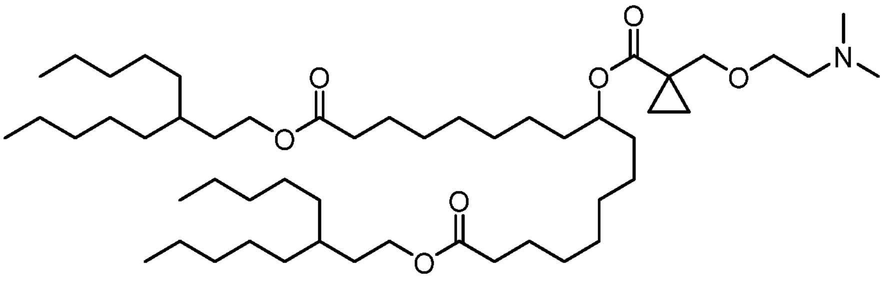

I-11

Step 28

Compound I-11 (123 mg, total yield 61%) was obtained as a clear oil in the same manner as in Step 3 and 4 using Compound 21 instead of Compound 4 in Step 3.
$^1$H-NMR ($CDCl_3$) δ: 0.88 (t, 14H), 1.19-1.35 (m, 50H), 1.35-1.65 (m, 28H), 2.28 (m, 10H), 2.49 (t, 2H), 3.56 (t, 2H), 3.62 (s, 2H), 4.08 (t, 4H), 4.84 (m, 1H). ESI-MS (m/z): 851 (M+1).

[Example 12]

Synthesis of Compound I-12

[Chemical Formula 70]

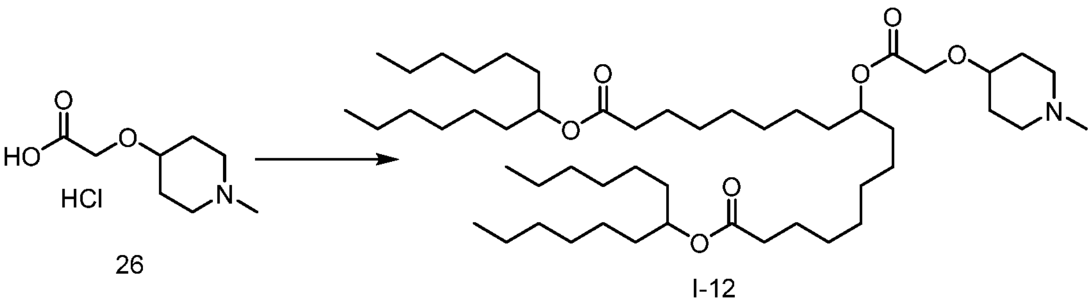

26

I-12

Step 29

Compound I-12 (169 mg, yield 87%) was obtained as a clear oil in the same manner as in Step 17 using Compound 26 instead of Compound 18 in Step 17.
$^1$H-NMR ($CDCl_3$) δ: 0.88 (t, 12H), 1.26-1.29 (m, 45H), 1.50-1.74 (m, 21H), 1.90-1.95 (m, 2H), 2.08-2.13 (m, 2H), 2.25-2.29 (m, 7H), 2.70-2.74 (m, 2H), 3.38-3.44 (m, 1H), 4.08 (s, 2H), 4.79-4.97 (m, 3H).
ESI-MS (m/z): 837 (M+1).

[Example 13]

Synthesis of Compound I-13

[Chemical Formula 71]

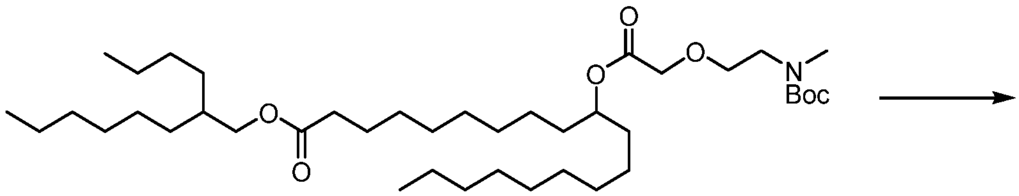

27

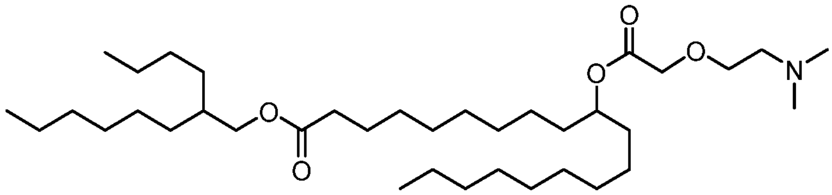

I-13

Step 30

To a solution of Compound 6 in dichloromethane (1.3 mL) were added Compound 4 (497 mg, 1.0 mmol), DMAP (277 mg, 2.26 mmol) and EDC (217 mg, 1.13 mmol) and the mixture was stirred at room temperature for 10 hours. Silica-gel was added to the reaction mixture and the solvent was distilled off under reduced pressure. The obtained residue was purified by silica-gel column chromatography (n-hexane-ethyl acetate) to give Compound 27 (601 mg, yield 84%).

$^1$H-NMR ($CDCl_3$) $\delta$: 0.88 (m, 9H), 1.28-1.43 (m, 41H), 1.45-1.61 (m, 23H), 2.30 (t, 2H), 2.93 (s, 3H), 3.44 (brs, 2H), 3.66 (brs, 2H), 3.97 (d, 2H), 4.06 (s, 2H), 4.95 (m, 1H).

ESI-MS (m/z): 699 (M+H).

Step 31

Compound I-13 (250 mg, yield 95%) was obtained as a clear oil in the same manner as in Step 4 using Compound 27 instead of Compound 5 in Step 4.

$^1$H-NMR ($CDCl_3$) $\delta$: 0.88 (m, 9H), 1.26 (m, 39H), 1.59 (m, 21H), 2.27-2.31 (m, 8H), 2.56 (t, 2H), 3.65 (t, 2H), 3.96 (d, 2H), 4.09 (s, 2H), 4.95 (t, 1H). ESI-MS(m/z): 613 (M+H).

[Example 14]

Synthesis of Compound I-14

[Chemical Formula 72]

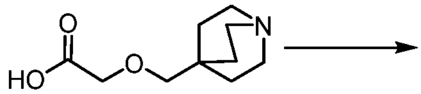

28

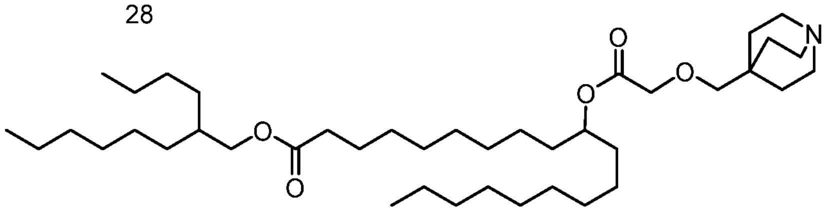

I-14

Step 32

Compound I-14 (21 mg, yield 13%) was obtained as a clear oil in the same manner as in Step 17 using Compound 4 instead of Compound 17 and Compound 28 instead of Compound 18 in Step 17.
$^1$H-NMR(CDCl3)δ: 0.88 (t, 9H), 1.25-1.28 (m, 43H), 1.42-1.58 (m, 54H), 1.85 (t, 6H), 2.29 (t, 2H), 3.36 (s, 2H), 3.41 (t, 6H), 3.97 (d, 2H), 4.02 (s, 2H), 4.94 (m, 1H).
ESI-MS(m/z): 665 (M+H).

[Example 15]

Synthesis of Compound I-15

[Chemical Formula 73]

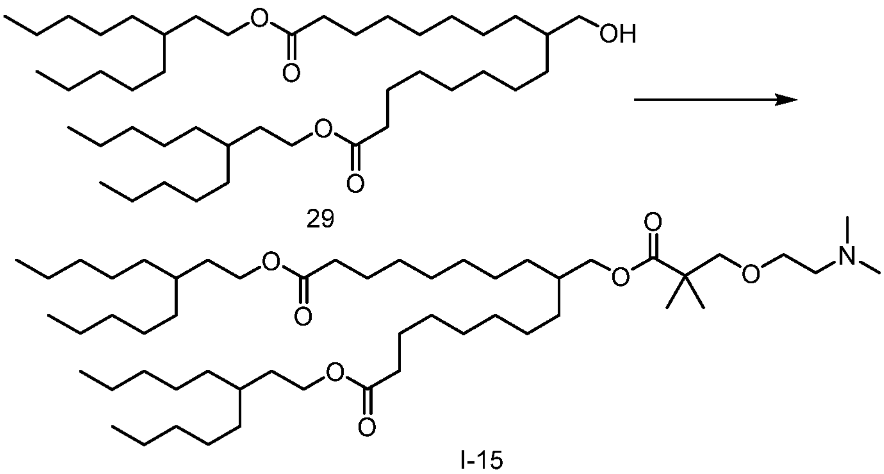

29

I-15

Step 33

Compound 29 (2.52 g) was obtained in the same manner as in Step 11 and 12 using 3-pentyl-1-octanol instead of 2-butyl-1-octanol in Step 11. $^1$H-NMR ($CDCl_3$) δ: 0.87 (m, 12H), 1.26-1.63 (m, 64H), 2.26 (t, 4H), 3.53 (brs, 2H), 4.06 (t, 4H).
ESI-MS (m/z): 696 (M+1).

Step 34

Compound I-15 (100 mg, total yield 33%) was obtained as a clear oil in the same manner as in Step 25 and 26 using Compound 29 instead of Compound 21 in Step 25.
$^1$H-NMR ($CDCl_3$) δ: 0.88 (t, 14H), 1.18 (s, 6H), 1.25-1.42 (m, 56H), 1.57 (m, 26H), 2.26 (m, 10H), 2.48 (t, 2H), 3.43 (s, 2H), 3.53 (t, 2H), 3.96 (d, 2H), 4.08 (t, 4H).
ESI-MS (m/z): 867 (M+H).

[Example 16]

Synthesis of Compound I-16

[Chemical Formula 74]

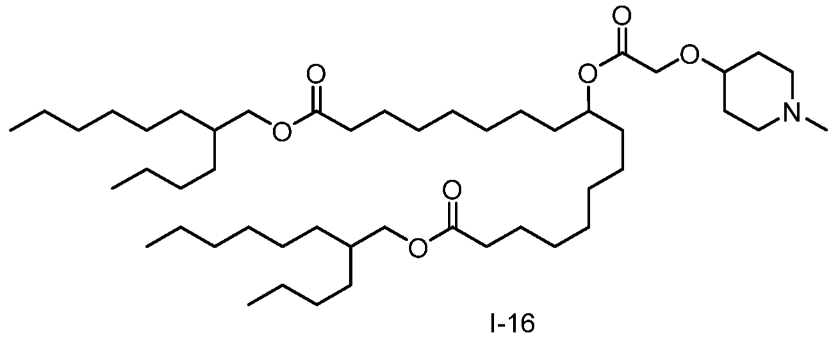

I-16

Step 35

Compound I-16 (115 mg, total yield 60%) was obtained as a clear oil in the same manner as in Step 17 using Compound 16 instead of Compound 17 and Compound 26 instead of Compound 18 in Step 17.
$^1$H-NMR ($CDCl_3$) δ: 0.88 (m, 14H), 1.28 (m, 40H), 1.51-1.71 (m, 12H), 1.91 (m, 2H), 2.11 (m, 2H), 2.29 (m, 7H), 2.70 (m, 2H), 3.41 (m, 1H), 3.96 (d, 4H), 4.08 (s, 2H), 4.94 (t, 1H).
ESI-MS (m/z): 809 (M+1).

[Example 17]

Synthesis of Compound I-17

[Chemical Formula 75]

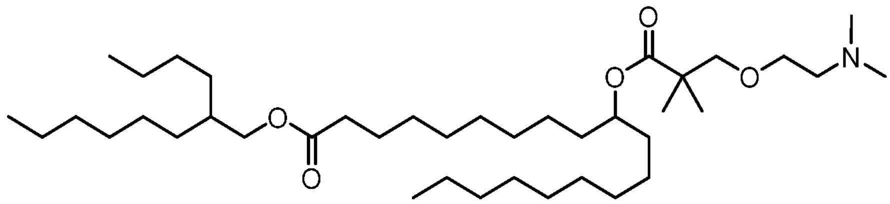

I-17

Step 36

Compound I-17 (20 mg, total yield 9%) was obtained as a clear oil in the same manner as in Step 25 and 26 using Compound 4 instead of Compound 21 in Step 25.
$^1$H-NMR ($CDCl_3$) δ: 0.88 (t, 9H), 1.17 (s, 6H), 1.25-1.28 (m, 40H), 1.49-1.61 (m, 23H), 2.26 (s, 6H), 2.29 (t, 2H), 2.48 (t, 2H), 3.42 (s, 2H), 3.53 (t, 2H), 3.96 (d, 2H), 4.84 (t, 1H).
ESI-MS (m/z): 655 (M+H).

[Example 18]

Synthesis of Compound I-18

[Chemical Formula 76]

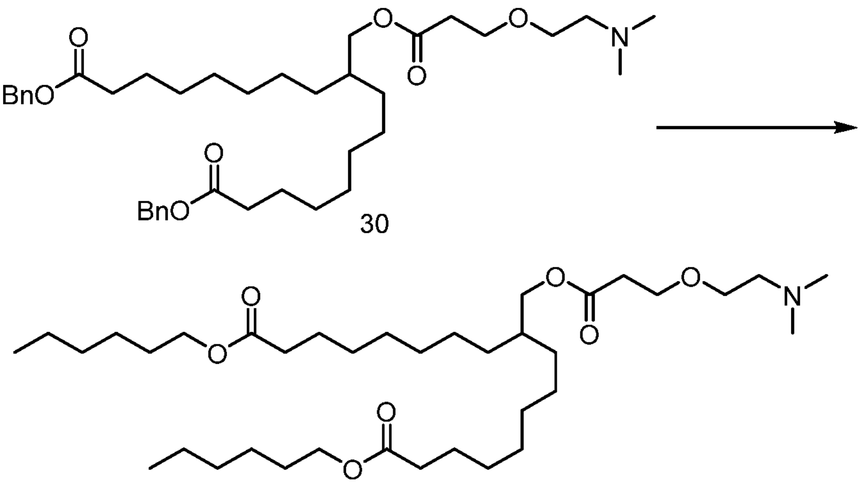

30

I-18

Step 37

Compound 30 was obtained in the same manner as in Step 17 using Compound 11 instead of Compound 17 and Compound 25 instead of Compound 18 in Step 17, and then Compound I-18 (120 mg, total yield 57%) was obtained as a clear oil in the same manner as in Step 11 using Compound 30 instead of Compound 12 and 1-hexanol instead of 2-butyl-1-octanol in Step 11.
$^1$H-NMR ($CDCl_3$) δ: 0.89 (t, 6H), 1.22-1.40 (m, 33H), 1.55-1.65 (m, 18H), 2.25-2.31 (m, 10H), 2.49 (t, 2H), 2.60 (t, 2H), 3.54 (t, 2H), 3.72 (t, 2H), 3.98 (d, 2H), 4.06 (t, 4H).
ESI-MS (m/z): 643 (M+1).

[Example 19]

Synthesis of Compound I-19

[Chemical Formula 77]

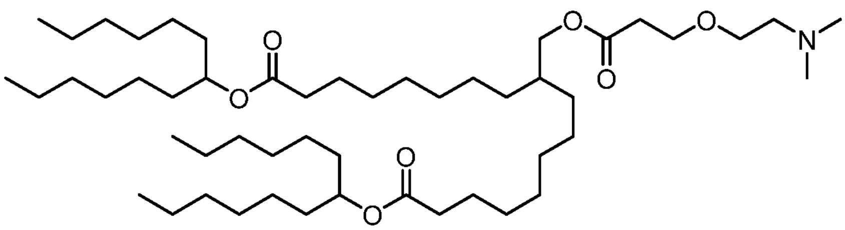

I-19

Step 38

Compound 1-19 (111 mg, total yield 50%) was obtained as a clear oil in the same manner as in Step 37 using 7-tridecanol instead of 1-hexanol in Step 37.
$^1$H-NMR ($CDCl_3$) δ: 0.88 (t, 12H), 1.20-1.35 (m, 51H), 1.45-1.65 (m, 15H), 2.25-2.31 (m, 10H), 2.49 (t, 2H), 2.60 (t, 2H), 3.54 (t, 2H), 3.72 (t, 2H), 3.98 (d, 2H), 4.87 (m, 2H).
ESI-MS (m/z): 839 (M+1).

[Example 20]

Synthesis of Compound I-20

[Chemical Formula 78]

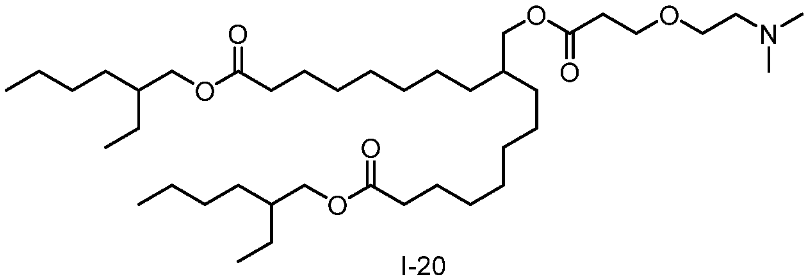

I-20

Step 39

Compound I-20 (116 mg, total yield 51%) was obtained as a clear oil in the same manner as in Step 37 using 2-ethyl-1-hexanol instead of 1-hexanol in Step 37.

$^1$H-NMR ($CDCl_3$) δ: 0.89 (t, 12H), 1.20-1.40 (m, 36H), 1.51-1.67 (m, 9H), 2.25-2.32 (m, 10H), 2.49 (t, 2H), 2.60 (t, 2H), 3.54 (t, 2H), 3.72 (t, 2H), 3.98 (m, 6H). ESI-MS (m/z): 699 (M+1).

[Example 21]

Synthesis of Compound I-21

[Chemical Formula 79]

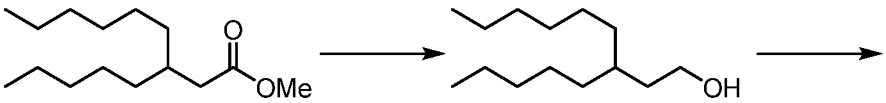

31

32

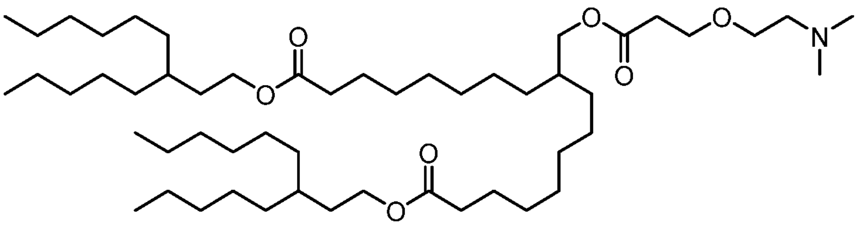

I-21

Step 40

A solution of copper bromide (735 mg, 5.12 mmol) and lithium chloride (434 mg, 10.2 mmol) in tetrahydrofuran (60 mL) was stirred at room temperature for 5 minutes. To the reaction mixture were added methyl trans-2-octenoate (8.0 g, 51.2 mmol) and TMS chloride (7.2 mL, 56.3 mmol) under ice-cooling and the mixture was stirred for 15 minutes. To the reaction mixture was added dropwise hexylmagnesium bromide-tetrahydrofuran solution (77 mL, 77 mmol) under ice-cooling and the mixture was stirred for 2 hours. Saturated aqueous solution of ammonium chloride and water were added to the reaction mixture and the mixture was extracted with diethyl ether. The organic layer was washed with saturated aqueous solution of sodium chloride and dried over anhydrous magnesium sulfate. The solvent was distilled off under reduced pressure and the obtained residue was purified by silica-gel column chromatography (n-hexane-ethyl

acetate) to give Compound 31 (9.80 g, yield 79%).
$^1$H-NMR ($CDCl_3$) δ: 0.88 (t, 6H), 1.26 (brs, 19H), 1.84 (brs, 1H), 2.23 (d, 2H), 3.66 (s, 3H).

Step 41

To a solution of lithium aluminium hydride (3.07 g, 81 mmol) in tetrahydrofuran (100 mL) was added dropwise a solution of Compound 31 (9.80 g, 40.4 mmol) in tetrahydrofuran (27 mL) and the mixture was heated under reflux for 3 hours. To the reaction mixture was added sodium sulfate decahydrate under ice-cooling and the mixture was stirred at room temperature for 2 hours. The reaction mixture was filtered, and the filtrate was distilled off under reduced pressure. Diethyl ether was added to the obtained residue and the aqueous layer was removed. The organic layer was dried over anhydrous magnesium sulfate. The solvent was distilled off under reduced pressure and the obtained residue was purified by silica-gel column chromatography (n-hexane-ethyl acetate) to give Compound 32 (8.26 g, yield 95%).
$^1$H-NMR ($CDCl_3$) δ: 0.88 (t, 6H), 1.12 (t, 1H), 1.26 (brs, 18H), 1.41 (brs, 1H), 1.53 (m, 2H), 3.66 (m, 2H).

Step 42

Compound I-21 (137 mg, total yield 54%) was obtained as a clear oil in the same manner as in Step 37 using Compound 32 instead of 1-hexanol in Step 37.
$^1$H-NMR ($CDCl_3$) δ: 0.89 (t, 12H), 1.20-1.45 (m, 58H), 1.54-1.66 (m, 11H), 2.25-2.32 (m, 10H), 2.49 (t, 2H), 2.60 (t, 2H), 3.55 (t, 2H), 3.72 (t, 2H), 3.98 (d, 2H), 4.08 (t, 4H).
ESI-MS (m/z): 867 (M+1).

[Example 22]

Synthesis of Compound I-22

[Chemical Formula 80]

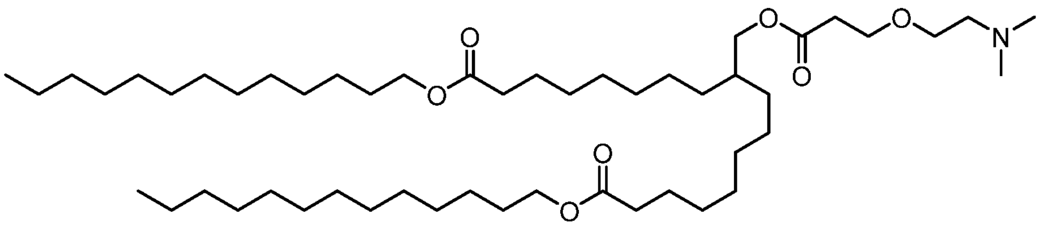

I-22

Step 43

Compound I-22 (134 mg, total yield 54%) was obtained as a clear oil in the same manner as in Step 37 using 1-tridecanol instead of 1-hexanol in Step 37.
$^1$H-NMR ($CDCl_3$) δ: 0.88 (t, 6H), 1.20-1.35 (m, 59H), 1.55-1.65 (m, 12H), 2.25-2.31 (m, 10H), 2.48 (t, 2H), 2.60 (t, 2H), 3.54 (t, 2H), 3.72 (t, 2H), 3.98 (d, 2H), 4.05 (t, 4H).
ESI-MS (m/z): 839 (M+1).

[Example 23]

Synthesis of Compound I-23

[Chemical Formula 81]

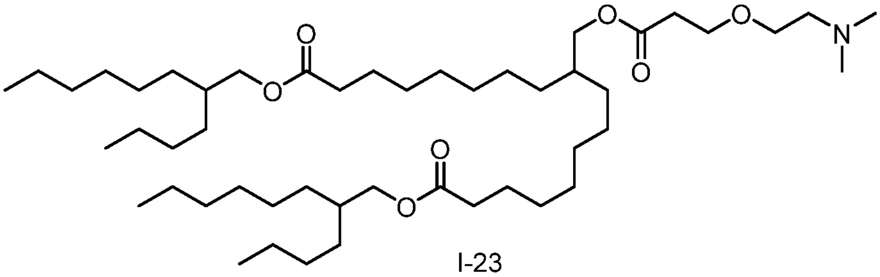

I-23

Step 44

Compound I-23 (183 mg, yield 66%) was obtained as a clear oil in the same manner as in Step 17 using Compound 25 instead of Compound 18 and Compound 14 instead of Compound 17 in Step 17.
$^{1}$H-NMR ($CDCl_3$) δ: 0.88 (m, 12H), 1.20-1.35 (m, 53H), 1.55-1.65 (m, 16H), 2.25-2.31 (m, 10H), 2.49 (t, 2H), 2.60 (t, 2H), 3.54 (t, 2H), 3.72 (t, 2H), 3.98 (m, 6H). ESI-MS (m/z): 811 (M+1).

[Example 24]

Synthesis of Compound I-24

[Chemical Formula 82]

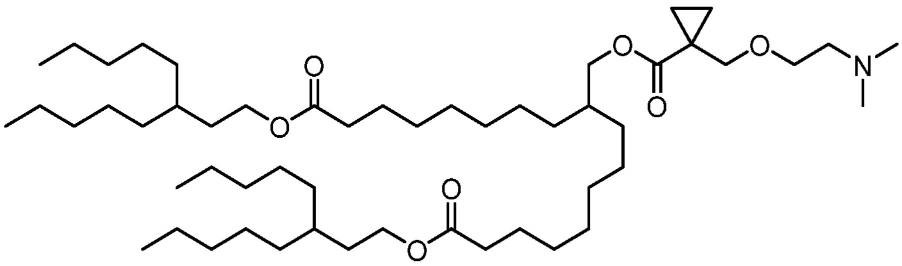

I-24

Step 45

Compound I-24 (102 mg, total yield 55%) was obtained as a clear oil in the same manner as in Step 3 and 4 using Compound 29 instead of Compound 4 in Step 3.
$^{1}$H-NMR ($CDCl_3$) δ: 0.88 (m, 14H), 1.20-1.45 (m, 57H), 1.52-1.65 (m, 15H), 2.25-2.31 (m, 10H), 2.50 (t, 2H), 3.56 (t, 2H), 3.61 (s, 2H), 3.95 (d, 2H), 4.08 (t, 4H). ESI-MS (m/z): 865 (M+1).

[Example 25]

Synthesis of Compound I-25

[Chemical Formula 83]

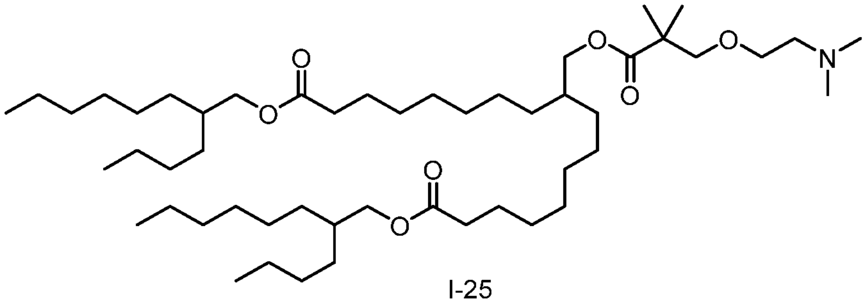

I-25

Step 46

Compound I-25 (46 mg, total yield 37%) was obtained as a clear oil in the same manner as in Step 25 and 26 using Compound 14 instead of Compound 21 in Step 25.
$^1$H-NMR ($CDCl_3$) δ: 0.88 (m, 12H), 1.18 (s, 6H), 1.20-1.35 (m, 54H), 1.52-1.65 (m, 24H), 2.25-2.31 (m, 10H), 2.48 (t, 2H), 3.42 (s, 2H), 3.53 (t, 2H), 3.97 (m, 6H).
ESI-MS (m/z): 839 (M+1).

[Example 26]

Synthesis of Compound I-26

[Chemical Formula 84]

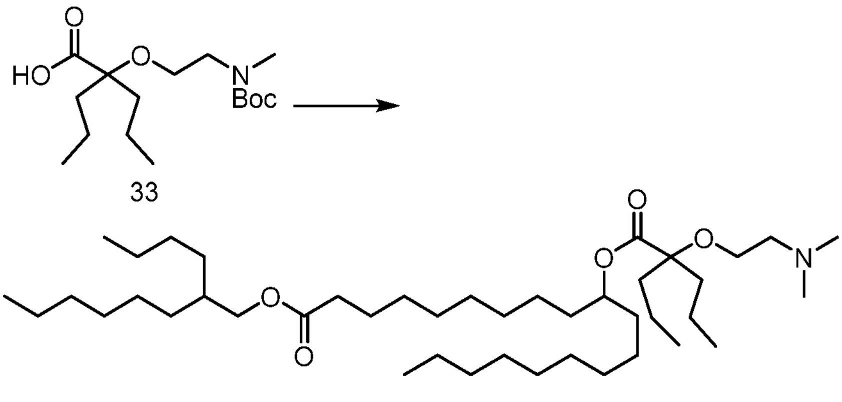

I-26

Step 47

Compound 33 (50 mg, yield 64%) was obtained in the same manner as in Step 8 using Compound 8 instead of Compound 9 in Step 8.
ESI-MS (m/z): 318 (M+1).

Step 48

Compound I-26 (54 mg, total yield 50%) was obtained as a clear oil in the same manner as in Step 3 and 4 using Compound 33 instead of Compound 3 in Step 3.
$^1$H-NMR ($CDCl_3$) δ:0.89 (m, 15H), 1.20-1.40 (m, 45H), 1.45-1.65 (m, 17H), 1.65-1.80 (m, 4H), 2.25-2.31 (m, 8H), 2.53 (t, 2H), 3.44 (t, 2H), 3.97 (d, 2H), 4.88 (m, 1H).
ESI-MS (m/z): 697 (M+1).

[Example 27]

Synthesis of Compound I-27

[Chemical Formula 85]

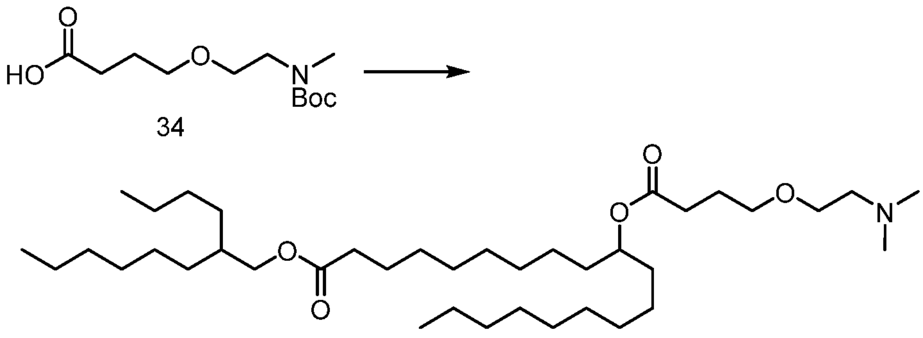

I-27

Step 49

Compound 34 (29 mg, yield 4%) was obtained in the same manner as in Step 2 using 4-bromobutyric acid instead of Compound 2 in Step 2.
ESI-MS(m/z): 262 (M+1).

Step 50

Compound I-27 (40 mg, total yield 57%) was obtained as a clear oil in the same manner as in Step 3 and 4 using Compound 34 instead of Compound 3 in Step 3.
$^1$H-NMR ($CDCl_3$) $\delta$: 0.88 (m, 9H), 1.20-1.35 (m, 40H), 1.45-1.65 (m, 13H), 1.90 (m, 2H), 2.25-2.31 (m, 8H), 2.37 (t, 2H), 2.49 (t, 2H), 3.46 (t, 2H), 3.51 (t, 2H), 3.97 (d, 2H), 4.86 (m, 1H).
ESI-MS (m/z): 641 (M+1).

[Example 28]

Synthesis of Compound I-28

[Chemical Formula 86]

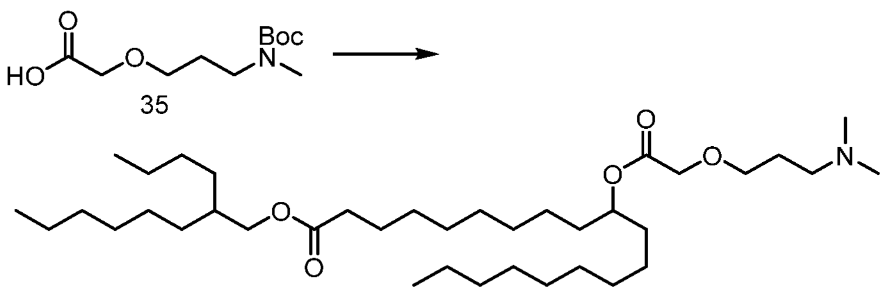

I-28

Step 51

Compound 35 (247 mg, yield 69%) was obtained in the same manner as in Step 2 using 2-bromoacetic acid

instead of Compound 2 and N-Boc-3-methylamino-1-propanol instead of N-Boc-N-methyl-2-aminoethanol in Step 2. ESI-MS (m/z): 248 (M+1).

Step 52

Compound I-28 (99 mg, total yield 76%) was obtained as a clear oil in the same manner as in Step 3 and 4 using Compound 35 instead of Compound 3 in Step 3.
$^1$H-NMR ($CDCl_3$) $\delta$: 0.88 (m, 9H), 1.20-1.35 (m, 41H), 1.45-1.65 (m, 17H), 1.80 (m, 2H), 2.23 (s, 6H), 2.29 (t, 2H), 2.38 (t, 2H), 3.58 (t, 2H), 3.97 (d, 2H), 4.05 (s, 2H), 4.95 (m, 1H).
ESI-MS (m/z): 627 (M+1).

[Example 29]

Synthesis of Compound I-29

[Chemical Formula 87]

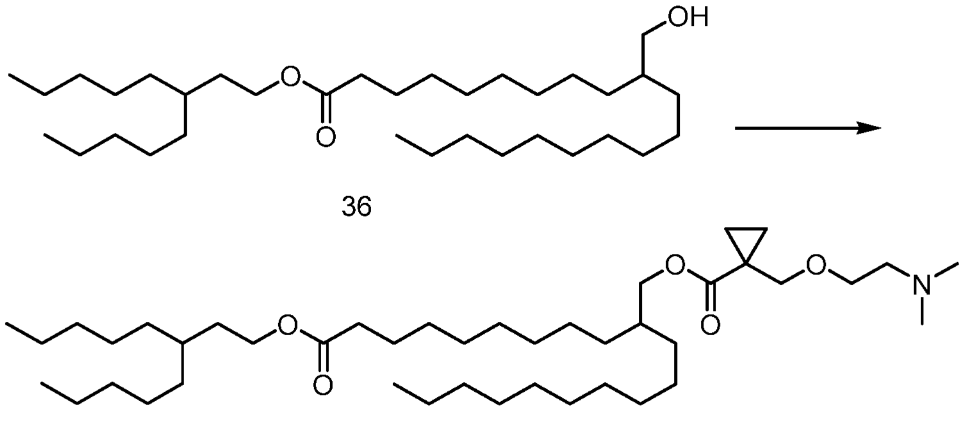

36

I-29

Step 53

Compound I-29 (87 mg, total yield 66%) was obtained as a clear oil in the same manner as in Step 3 and 4 using Compound 36 (see WO2019/131580) instead of Compound 4 in Step 3.
$^1$H-NMR ($CDCl_3$) $\delta$: 0.88 (m, 11H), 1.20-1.45 (m, 49H), 1.50-1.65 (m, 10H), 2.25-2.31 (m, 8H), 2.50 (t, 2H), 3.56 (t, 2H), 3.61 (s, 2H), 3.96 (d, 2H), 4.08 (t, 2H). ESI-MS(m/z): 695 (M+1).

[Example 30]

Synthesis of Compound I-30

[Chemical Formula 88]

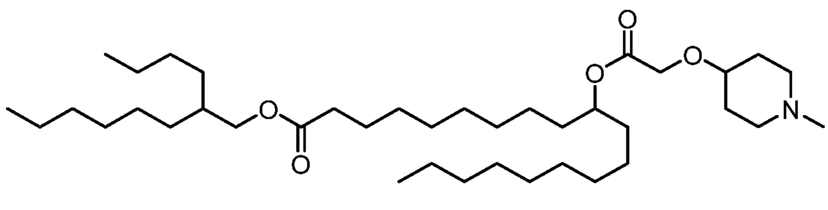

I-30

Step 54

To a solution of Compound 26 (434 mg, 2.07 mmol) in dichloromethane (15 mL) was added DMF (0.12 mL and the mixture was cooled to 0 °C. To the reaction mixture was added oxalyl chloride (0.163 mL, 0.622 mmol) and the mixture was heated under reflux for 90 minutes. The reaction mixture was distilled off under reduced pressure and dichloromethane (1 mL) was added to the obtained residue. This solution was added to a solution of Compound 4 (500 mg, 1.04 mmol) in dichloromethane (50 mL)-triethylamine (0.43 mL) under ice-cooling and the mixture was stirred overnight. Aqueous solution of sodium hydrogen carbonate was added to the reaction mixture and the mixture was extracted with ethyl acetate. The solvent was distilled off under reduced pressure and the obtained residue was purified by aminosilica-gel column chromatography (n-hexane-ethyl acetate) to give Compound I-30 (250 mg, yield 38%) as a clear oil.
$^1$H-NMR ($CDCl_3$) δ: 0.88 (m, 9H), 1.25-1.74 (m, 49H), 1.91-1.93 (m, 2H), 2.10 (t, 2H), 2.26 (s, 3H), 2.29 (t, 2H), 2.70 (m, 2H), 3.41 (m, 1H), 3.96 (d, 2H), 4.08 (s, 2H), 4.95 (m, 1H). ESI-MS (m/z): 639 (M+1).

[Example 31]

Synthesis of Compound I-31

[Chemical Formula 89]

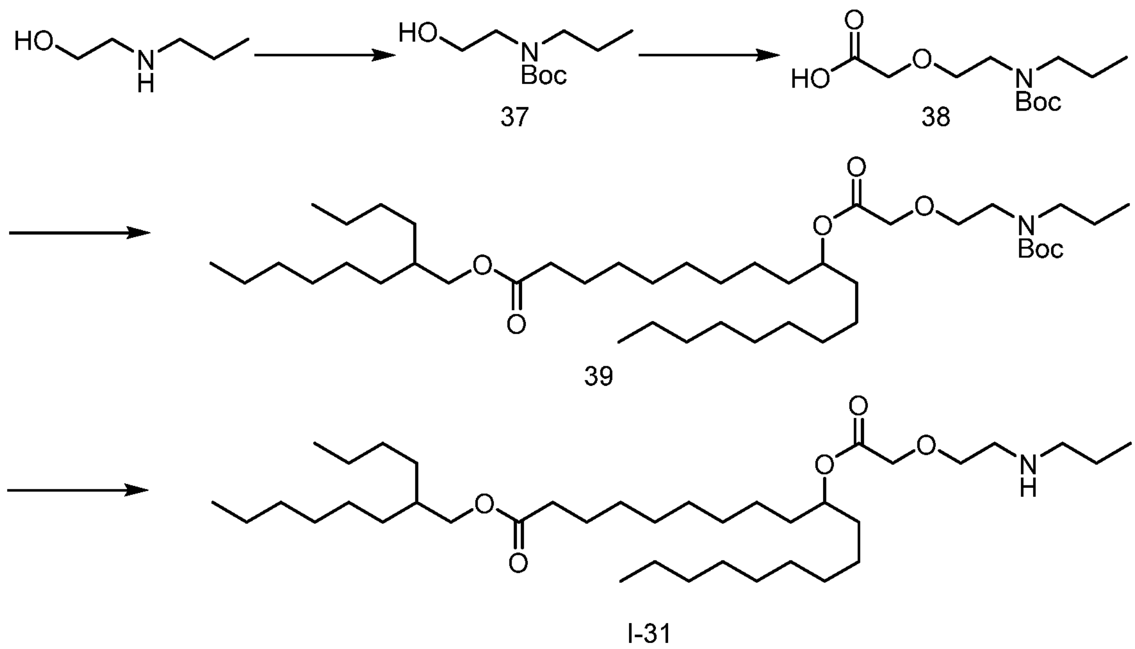

Step 55

A solution of 2-(propylamino)ethan-1-ol (9 g, 87 mmol) in ethyl acetate (60 mL) was cooled with ice and to this solution was added dropwise a solution of di-tert-butyl dicarbonate (17.1 g, 79 mmol) in ethyl acetate (60 mL). The mixture was stirred at room temperature overnight. Water was added to the reaction mixture and the mixture was extracted with ethyl acetate. The organic layer was dried over anhydrous magnesium sulfate and filtered. The solvent was distilled off under reduced pressure to give Compound 37 (15.6 g) as a clear oil.
Compound 38 (4.9 g, mixture with Compound 37) was obtained in the same manner as in Step 2 using Compound 37 (4.39 g) instead of N-Boc-N-methyl-2-aminoethanol and 2-bromoacetic acid instead of Compound 2 in Step 2.
Compound 39 (0.875 g) was obtained in the same manner as in Step 3 using Compound 38 instead of Compound 3 in Step 3.
$^1$H-NMR ($CDCl_3$) δ: 0.86 (m, 12H), 1.25-1.63 (m, 58H), 2.27 (t, 2H), 3.19 (t, 2H), 3.41 (brs, 2H), 3.65 (brs, 2H), 3.96 (d, 2H), 4.06 (s, 2H), 4.93 (m, 1H).
ESI-MS (m/z): 727 (M+1).

Step 56

To Compound 39 (0.875 g, 1.21 mmol) was added hydrochloric acid-1,4-dioxane solution (8.4 mL, 33.6 mmol) and the mixture was stirred at room temperature for 30 minutes. The solvent was distilled off under reduced pressure and the obtained residue was purified by aminosilica-gel column chromatography (n-hexane-ethyl acetate) to give Compound I-31 (0.61 g, yield 80%) as a slightly colored oil.
$^1$H-NMR ($CDCl_3$) $\delta$: 0.88-0.94 (m, 12H), 1.25-1.61 (m, 50H), 2.27 (t, 2H), 2.57 (t, 2H), 2.83 (m, 2H), 3.66(brs, 2H), 3.96 (d, 2H), 4.08 (s, 2H), 4.94 (m, 1H). ESI-MS (m/z): 627 (M+1).

[Example 32]

Synthesis of Compound I-32

[Chemical Formula 90]

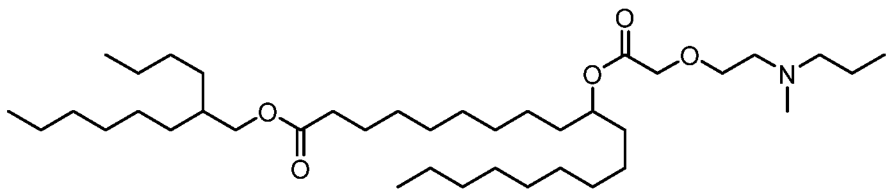

I-32

Step 57

To a solution of Compound I-31 (0.143 g, 0.228 mmol) in dichloromethane (2.1 mL) were added 36% formaldehyde solution (0.061 mL) and sodium triacetoxyborohydride (0.335 g) and the mixture was stirred at room temperature for 3 hours. To the reaction mixture were added sodium triacetoxyborohydride (0.335 g) and hydrochloric acid-1,4-dioxane solution and the mixture was stirred for 1 hour. Saturated aqueous solution of sodium hydrogen carbonate was added to the reaction mixture and the mixture was extracted with ethyl acetate. The solvent was distilled off under reduced pressure and the obtained residue was purified by aminosilica-gel column chromatography (n-hexane-ethyl acetate) to give Compound I-32 (115 mg, yield 79%) as a clear oil.
$^1$H-NMR($CDCl_3$)$\delta$:0.86 (m, 12H), 1.25-1.61 (m, 49H), 2.28 (s, 3H), 2.29-2.37 (m, 4H), 2.60 (t, 2H), 3.63 (t, 2H), 3.96 (d, 2H), 4.09 (s, 2H), 4.92 (m, 1H). ESI-MS(m/z): 641 (M+1).

[Example 33]

Synthesis of Compound I-33

[Chemical Formula 91]

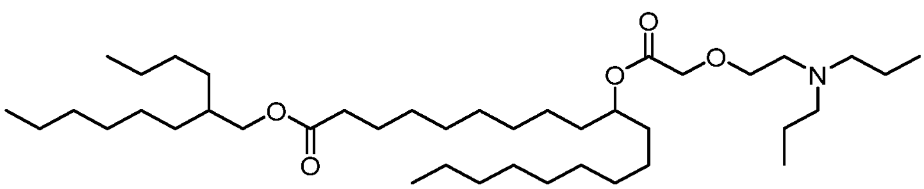

I-33

Step 58

To a solution of Compound I-31 (0.154 g, 0.246 mmol) in dichloromethane (2.1 mL) were added hydrochloric acid-1,4-dioxane solution (1 drop), propionaldehyde (0.058 mL) and sodium triacetoxyborohydride (0.335 g) and the mixture was stirred at room temperature for 3 hours. Saturated aqueous solution of sodium hydrogen carbonate was

added to the reaction mixture and the mixture was extracted with ethyl acetate. The solvent was distilled off under reduced pressure and the obtained residue was purified by aminosilica-gel column chromatography (n-hexane-ethyl acetate) to give Compound I-33 (108 mg, yield 66%) as a clear oil.

$^1$H-NMR ($CDCl_3$) $\delta$: 0.85-0.89 (m, 15H), 1.25-1.63 (m, 51H), 2.27 (t, 2H), 2.41 (t, 4H), 2.68 (t, 2H), 3.59 (t, 2H), 3.96 (d, 2H), 4.09 (s, 2H), 4.93 (m, 1H).

ESI-MS (m/z): 669 (M+1).

[Example 34]

Synthesis of Compound I-34

[Chemical Formula 92]

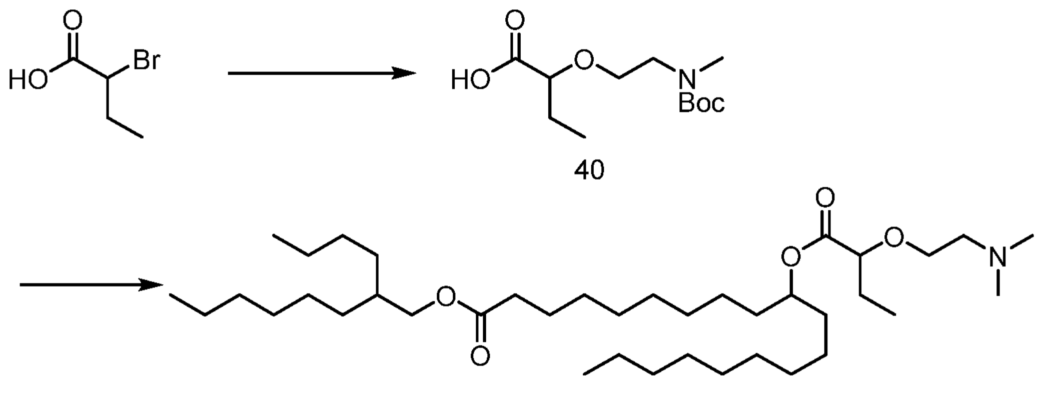

40

I-34

Step 59

Compound 40 was obtained in the same manner as in Step 2 using 2-bromobutyric acid instead of Compound 2 in Step 2, and then Compound I-34 (144 mg) was obtained as a clear oil in the same manner as in Step 3 and 4 using Compound 40 instead of Compound 3 in Step 3.

$^1$H-NMR ($CDCl_3$) $\delta$: 0.82 (m, 9H), 0.95 (t, 3H), 1.25-1.60 (m, 47H), 1.69 (m, 2H), 2.27 (s, 6H), 2.29 (t, 2H), 2.53 (t, 2H), 3.41 (m, 1H), 3.67 (m, 1H), 3.75 (m, 1H), 3.96 (d, 2H), 4.90 (m, 1H).

ESI-MS (m/z): 641 (M+1).

[Example 35]

Synthesis of Compound I-35

[Chemical Formula 93]

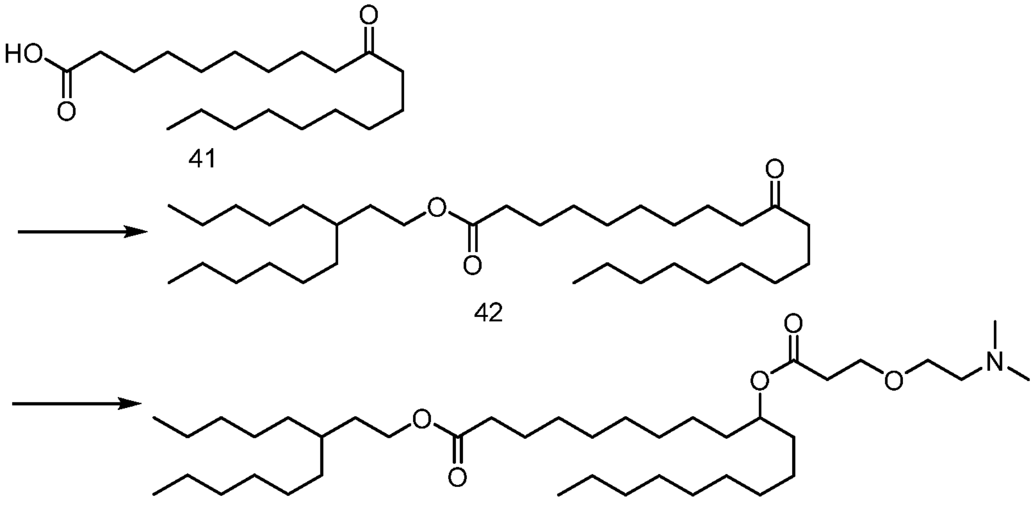

41

42

I-35

Step 60

To a solution of Compound 32 (1.65 g, 7.68 mmol) in tetrahydrofuran (22 mL) were added Compound 41 (see WO2016/104580, 2 g, 6.4 mmol), triphenylphosphine (2.01 g, 7.68 mmol) and azodicarboxylic acid bis(2-methoxyethyl) ester (2.1 g, 8.96 mmol) and the mixture was stirred at room temperature for 2 hours. Water was added to the reaction mixture and the mixture was extracted with ethyl acetate. The solvent was distilled off under reduced pressure and the obtained residue was purified by aminosilica-gel column chromatography (n-hexane-ethyl acetate/chloroform) to give Compound 42 (2.71 g, yield 83%).

$^1$H-NMR ($CDCl_3$) $\delta$: 0.83 (m, 9H), 1.25-1.62 (m, 47H), 2.26 (t, 2H), 2.35 (t, 4H), 4.06 (t, 2H).

ESI-MS (m/z): 510 (M+1).

Step 61

To a solution of Compound 42 (2.71 g, 5.33 mmol) in 50%tetrahydrofuran-methanol (27 mL) was added sodium borohydride (0.4 g, 10.7 mmol) under ice-cooling and the mixture was stirred 40 minutes. Acetone and Saturated aqueous solution of ammonium chloride were added to the reaction mixture and the mixture was extracted with ethyl acetate. The organic layer was dried over anhydrous magnesium sulfate and filtered. The solvent was distilled off under reduced pressure and the obtained residue was purified by silica-gel column chromatography (n-hexane-ethyl acetate). The solvent in the fractions containing the desired product was distilled off under reduced pressure, and then Compound I-35 (0.537 g, total yield 84%) was obtained as a clear oil in the same manner as in Step 17 using Compound 25 instead of Compound 18 and the obtained residue instead of Compound 17 in Step 17.

$^1$H-NMR ($CDCl_3$) $\delta$: 0.87 (m, 9H), 1.26-1.62 (m, 51H), 2.25-2.29 (m, 8H), 2.41 (t, 2H), 2.56 (t, 2H), 3.53 (t, 2H), 3.70 (t, 2H), 4.06 (t, 2H), 4.85 (m, 1H).

ESI-MS (m/z): 654 (M+1).

[Example 36]

Synthesis of Compound I-36

[Chemical Formula 94]

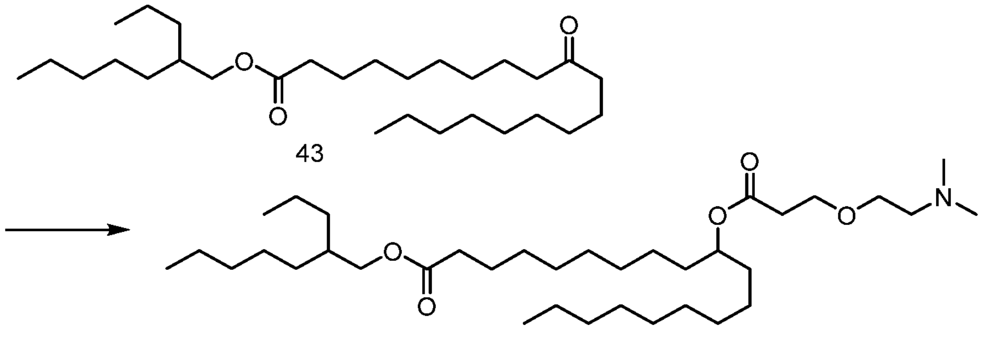

43

I-36

Step 62

Compound 43 (1.6 g, yield 64%) was obtained in the same manner as in Step 60 using 2-propylheptanol instead of Compound 32 in Step 60.
$^1$H-NMR ($CDCl_3$)δ: 0.86-0.91 (m, 9H), 1.26-1.63 (m, 39H), 2.27 (t, 2H), 2.36 (t, 4H), 3.96 (d, 2H).
ESI-MS (m/z): 453 (M+1).

Step 63

Compound I-36 (0.314 g, total yield 72%) was obtained as a clear oil in the same manner as in Step 61 using Compound 43 instead of Compound 42 in Step 61.
$^1$H-NMR ($CDCl_3$) δ: 0.86 (m, 9H), 1.25-1.65 (m, 43H), 2.25-2.31 (m, 8H), 2.47 (t, 2H), 2.56 (t, 2H), 3.53 (t, 2H), 3.70 (t, 2H), 3.96 (d, 2H), 4.85 (m, 1H).
ESI-MS (m/z): 598 (M+1).

[Example 37]

Synthesis of Compound I-37

[Chemical Formula 95]

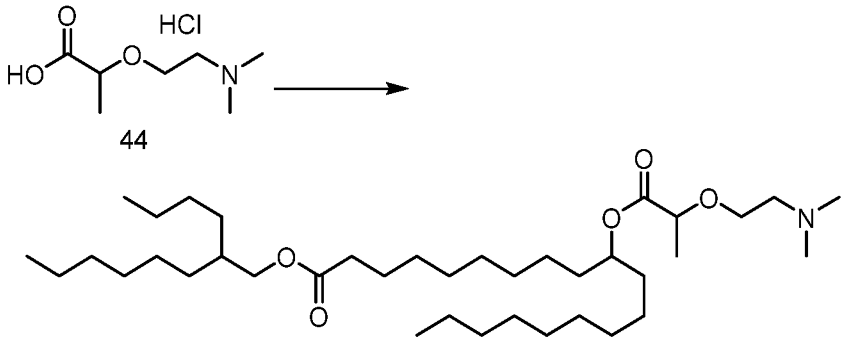

44

I-37

Step 64

Compound I-37 (0.276 g, yield 71%) was obtained as a clear oil in the same manner as in Step 17 using Compound 44 instead of Compound 18 and Compound 4 instead of Compound 17 in Step 17.
$^1$H-NMR ($CDCl_3$) δ: 0.87 (m, 9H), 1.25-1.62 (m, 50H), 2.27-2.31 (m, 8H), 2.53 (t, 2H), 3.43 (m, 1H), 3.65 (m, 1H), 3.93 (m, 3H), 4.90 (m, 1H).
ESI-MS (m/z): 627 (M+1).

[Example 38]

Synthesis of Compound I-38

[Chemical Formula 96]

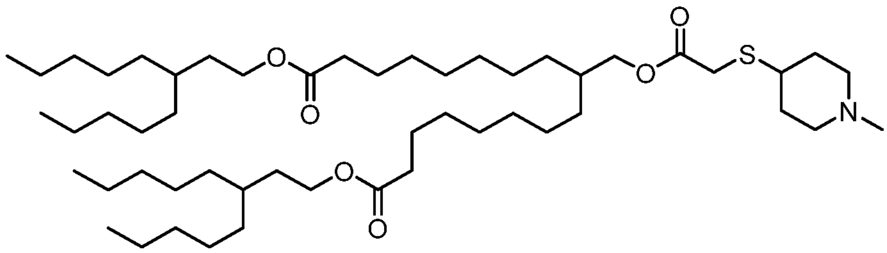

I-38

Step 65

Compound I-38 (0.219 g, yield 44%) was obtained as a clear oil in the same manner as in Step 17 using Compound 29 instead of Compound 17 in Step 17.
$^1$H-NMR ($CDCl_3$) δ: 0.87 (m, 12H), 1.25-1.68 (m, 65H), 1.96 (brs, 4H), 2.26-2.30 (m, 7H), 2.78 (brs, 3H), 3.25 (s, 2H), 4.02 (d, 2H), 4.06 (t, 4H).
ESI-MS (m/z): 867 (M+1).

[Example 39]

Synthesis of Compound I-39

[Chemical Formula 97]

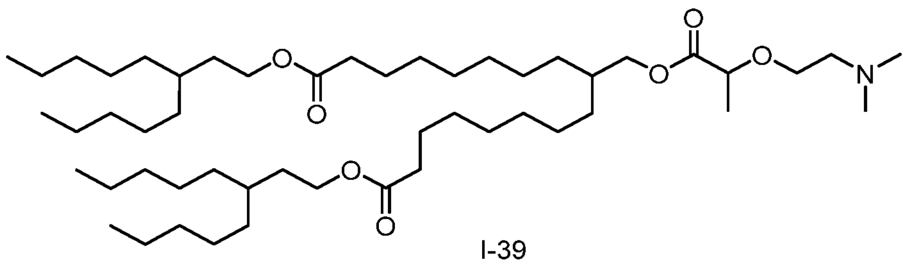

I-39

Step 66

Compound I-39 (0.186 g, yield 51%) was obtained as a clear oil in the same manner as in Step 17 using Compound 44 instead of Compound 18 and Compound 29 instead of Compound 17 in Step 17.
$^1$H-NMR ($CDCl_3$) δ: 0.87 (t, 12H), 1.25-1.68 (m, 66H), 2.26-2.30 (m, 10H), 2.52 (t, 2H), 3.45 (m, 1H), 3.66 (m, 1H), 3.96-4.06 (m, 7H).
ESI-MS (m/z): 839 (M+1)

[Example 40]

Synthesis of Compound I-40

[Chemical Formula 98]

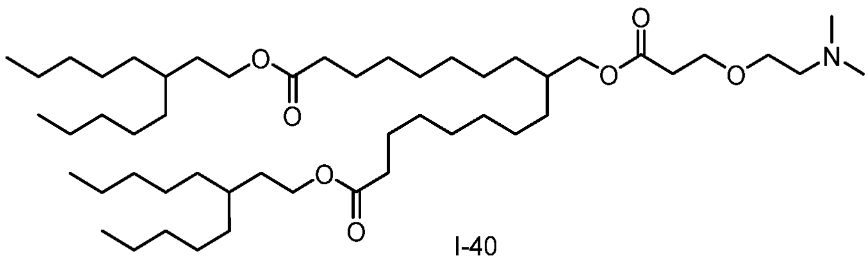

I-40

Step 67

Compound I-40 (0.373 g, yield 77%) was obtained as a slightly colored oil in the same manner as in Step 17 using Compound 25 instead of Compound 18 and Compound 29 instead of Compound 17 in Step 17.
$^{1}$H-NMR ($CDCl_3$) δ: 0.87 (t, 12H), 1.25-1.64 (m, 63H), 2.25-2.30 (m, 10H), 2.47 (t, 2H), 2.58 (t, 2H), 3.53 (t, 2H), 3.70 (t, 2H), 3.97 (d, 2H), 4.06 (t, 4H). ESI-MS (m/z): 839 (M+1)

[Example 41]

Synthesis of Compound I-41

[Chemical Formula 99]

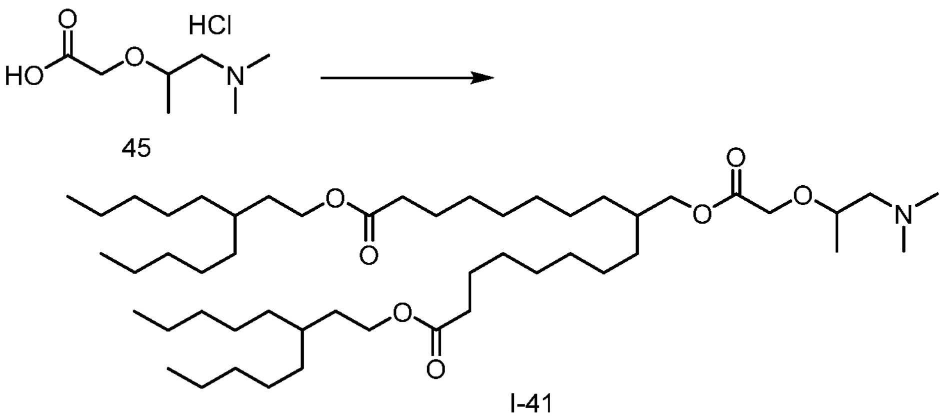

I-41

Step 68

Compound I-41 (0.232 g, yield 64%) was obtained as a clear oil in the same manner as in Step 17 using Compound 45 instead of Compound 18 and Compound 29 instead of Compound 17 in Step 17.
$^{1}$H-NMR ($CDCl_3$) δ: 0.87 (t, 12H), 1.18-1.64 (m, 66H), 2.28 (dd, 1H), 2.25-2.30 (m, 10H), 2.51 (dd, 1H), 3.66 (m, 1H), 4.03 (d, 2H), 4.06 (t, 4H), 4.17 (m, 2H). ESI-MS (m/z): 839 (M+1)

[Example 42]

Synthesis of Compound I-42

[Chemical Formula 100]

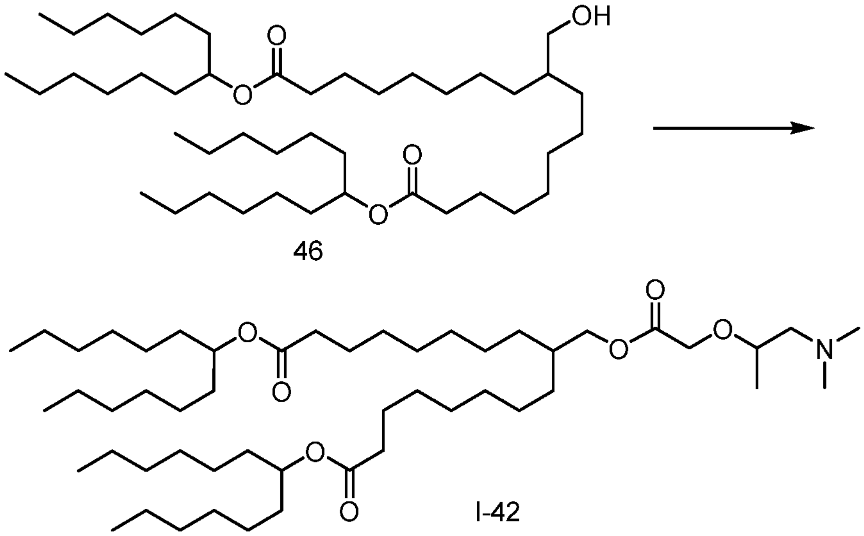

46

I-42

Step 69

Compound 46 (1.69 g, total yield 23%) was obtained in the same manner as in Step 11 and 12 using 7-tridecanol instead of 2-butyl-1-octanol in Step 11.
$^1$H-NMR ($CDCl_3$)δ: 0.88 (t, 14H), 1.20-1.35 (m, 56H), 1.40-1.65 (m, 21H), 2.27 (t, 4H), 3.53 (t, 2H), 4.87 (m, 2H).
ESI-MS (m/z): 696 (M+1).

Step 70

Compound I-42 (0.046 g, yield 32%) was obtained as a clear oil in the same manner as in Step 17 using Compound 45 instead of Compound 18 and Compound 46 instead of Compound 17 in Step 17.
$^1$H-NMR ($CDCl_3$) δ: 0.86 (t, 12H), 1.18-1.63 (m, 68H), 2.19 (dd, 1H), 2.25-2.29 (m, 10H), 2.49 (dd, 1H), 3.66 (m, 1H), 4.00 (d, 2H), 4.13 (m, 2H), 4.84 (m, 2H). ESI-MS (m/z): 839 (M+1)

[Example 43]

Synthesis of Compound I-43

[Chemical Formula 101]

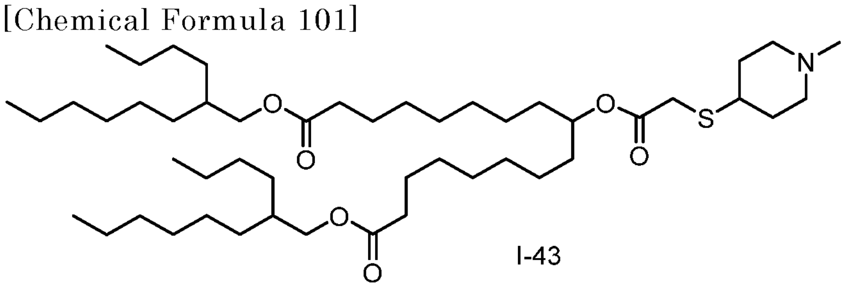

I-43

Step 71

Compound I-43 (0.084 g, yield 67%) was obtained as a clear oil in the same manner as in Step 17 using Compound 16 instead of Compound 17 in Step 17.
$^1$H-NMR ($CDCl_3$) δ: 0.87 (t, 12H), 1.27-1.68 (m, 60H), 1.96-2.05 (br., 4H), 2.26-2.31 (m, 7H), 2.78 (brs, 3H), 3.22 (s, 2H), 3.96 (d, 4H), 4.85 (m, 1H).
ESI-MS (m/z): 825 (M+1)

[Example 44]

Synthesis of Compound I-44

[Chemical Formula 102]

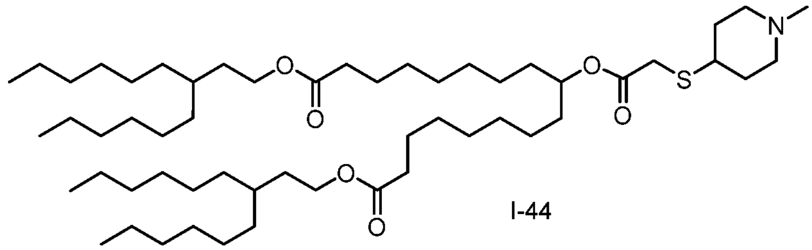

I-44

Step 72

Compound I-44 (0.271 g, yield 55%) was obtained as a clear oil in the same manner as in Step 17 using Compound 22 instead of Compound 17 in Step 17.
$^1$H-NMR ($CDCl_3$) $\delta$: 0.87 (t, 12H), 1.25-1.68 (m, 72H), 1.96-2.05 (br., 4H), 2.26-2.30 (m, 7H), 2.78 (brs, 3H), 3.23 (s, 2H), 4.06 (t, 4H), 4.85 (m, 1H). ESI-MS (m/z): 909 (M+1)

[Example 45]

Synthesis of Compound I-45

[Chemical Formula 103]

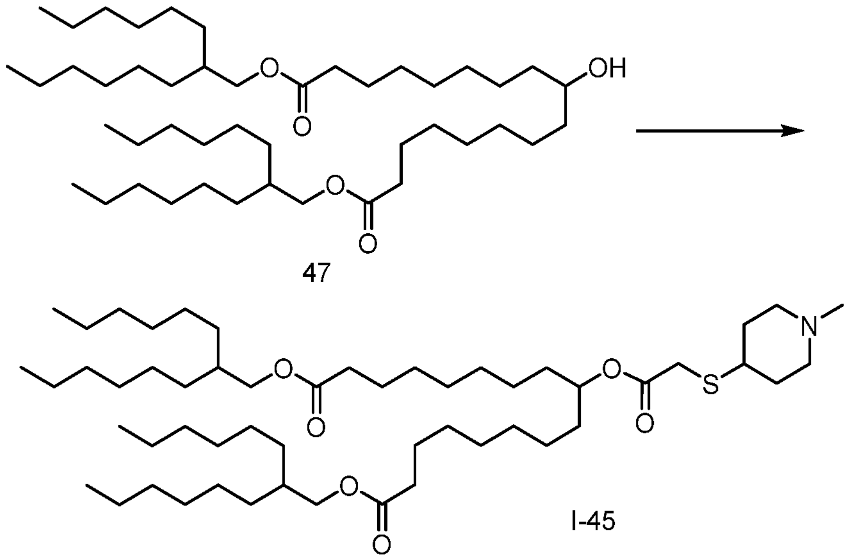

47

I-45

Step 73

Compound 47 (1.46 g, total yield 81%) was obtained in the same manner as in Step 14 using 2-hexyl-1-octanol instead of 2-butyl-1-octanol in Step 14.
$^1$H-NMR ($CDCl_3$) $\delta$: 0.88 (t, 12H), 1.20-1.50 (m, 65H), 1.55-1.70 (m, 8H), 2.30 (t, 4H), 3.57 (brs, 1H), 3.97 (d, 4H).

Step 74

Compound I-45 (0.518 g, yield 83%) was obtained as a slightly colored oil in the same manner as in Step 17 using Compound 47 instead of Compound 17 in Step 17.
$^1$H-NMR ($CDCl_3$) $\delta$: 0.81 (t, 12H), 1.27-1.64 (m, 68H), 1.96-2.06 (brs, 4H), 2.26-2.31 (m, 7H), 2.79 (brs, 3H), 3.23 (s, 2H), 4.06 (d, 4H), 4.85 (m, 1H).
ESI-MS (m/z): 882 (M+1)

[Example 46]

Synthesis of Compound I-46

[Chemical Formula 104]

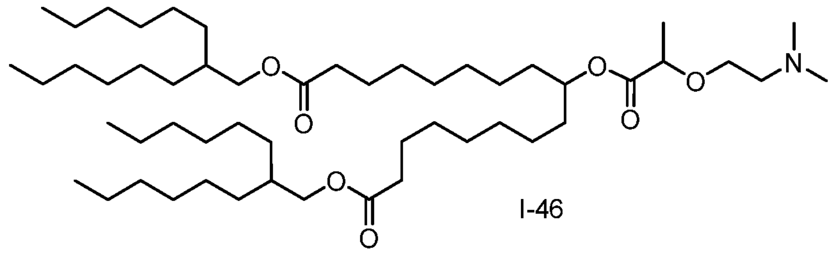

I-46

Step 75

Compound I-46 (0.488 g) was obtained as a clear oil in the same manner as in Step 3 and 4 using Compound 20 instead of Compound 3 and Compound 47 instead of Compound 4 in Step 3.
$^1$H-NMR ($CDCl_3$) $\delta$:0.87 (t, 12H), 1.27-1.61 (m, 69H), 2.27-2.31 (m, 10H), 2.52 (t, 2H), 3.45 (m, 1H), 3.67 (m, 1H), 3.96 (m, 5H), 4.91 (m, 1H).
ESI-MS (m/z): 854 (M+1)

[Example 47]

Synthesis of Compound I-47

[Chemical Formula 105]

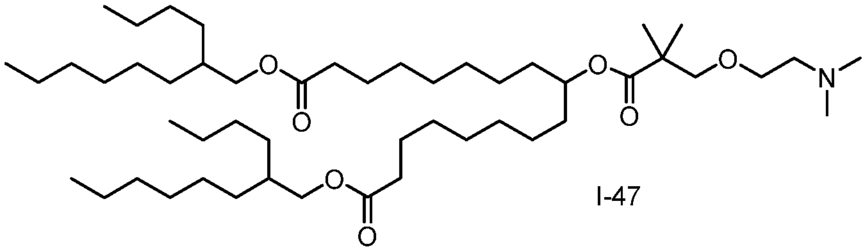

I-47

Step 76

Compound I-47 (80 mg) was obtained as a clear oil in the same manner as in Step 25 and 26 using Compound 16 instead of Compound 21 in Step 25. $^1$H-NMR ($CDCl_3$) $\delta$: 0.87 (m, 12H), 1.17 (s, 6H), 1.27-1.60 (m, 58H), 2.25-2.31 (m, 10H), 2.46 (t, 2H), 3.42 (s, 2H), 3.51 (t, 2H), 3.95 (d, 4H), 4.81 (m, 1H).
ESI-MS (m/z): 825 (M+1)

[Example 48]

Synthesis of Compound I-48

[Chemical Formula 106]

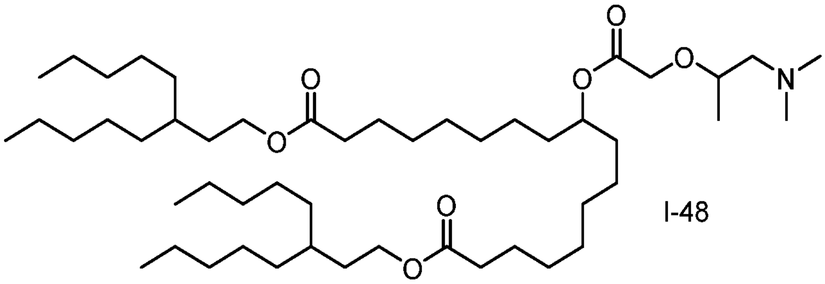

I-48

Step 77

Compound I-48 (0.172 g, yield 85%) was obtained as a clear oil in the same manner as in Step 17 using Compound 45 instead of Compound 18 and Compound 21 instead of Compound 17 in Step 17.
$^1$H-NMR ($CDCl_3$) δ: 0.87 (t, 12H), 1.18 (d, 3H), 1.25-1.62 (m, 62H), 2.19 (dd, 1H), 2.26-2.29 (m, 10H), 2.50 (dd, 1H), 3.66 (m, 1H), 4.06 (t, 4H), 4.11 (m, 2H), 4.91 (m, 1H).
ESI-MS (m/z): 825 (M+1)

[Example 49]

Synthesis of Compound I-49

[Chemical Formula 107]

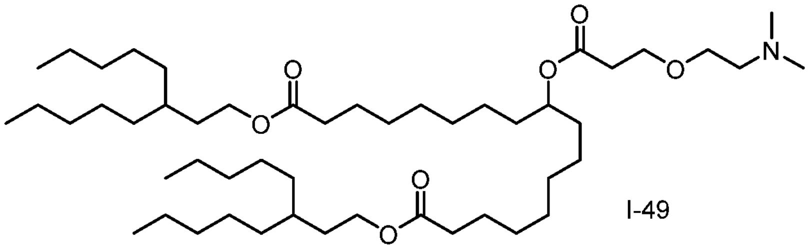

I-49

Step 77 Synthesis of Compound I-49

Compound I-49 (0.042 g, yield 60%) was obtained as a clear oil in the same manner as in Step 17 using Compound 25 instead of Compound 18 and Compound 21 instead of Compound 17 in Step 17.
$^1$H-NMR ($CDCl_3$) δ: 0.87 (t, 12H), 1.25-1.62(m, 62H), 2.25-2.29 (m, 10H), 2.42 (t, 2H), 2.56 (t, 2H), 3.53 (t, 2H), 3.70 (t, 2H), 4.06 (t, 4H), 4.84 (m, 1H). ESI-MS (m/z): 825 (M+1)

[Example 50]

Synthesis of Compound I-50

[Chemical Formula 108]

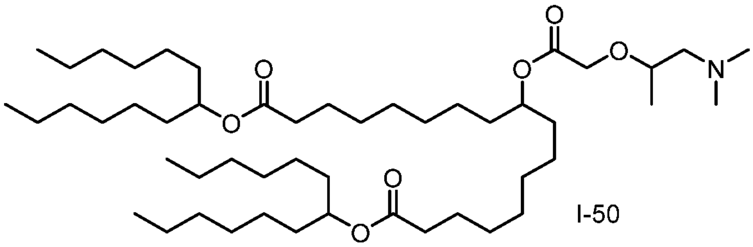

I-50

Step 79 Synthesis of Compound I-50

Compound I-50 (0.128 g, yield 88%) was obtained as a clear oil in the same manner as in Step 17 using Compound 45 instead of Compound 18 in Step 17.
$^1$H-NMR ($CDCl_3$) δ: 0.86 (t, 12H), 1.18 (d, 3H), 1.26-1.62 (m, 64H), 2.19 (dd, 1H), 2.26-2.29 (m, 10H), 2.49 (dd, 1H), 3.66 (m, 1H), 4.13 (m, 2H), 4.83 (m, 2H), 4.91 (m, 1H).
ESI-MS (m/z): 825 (M+1)

[Example 51]

Synthesis of Compound I-51

[Chemical Formula 109]

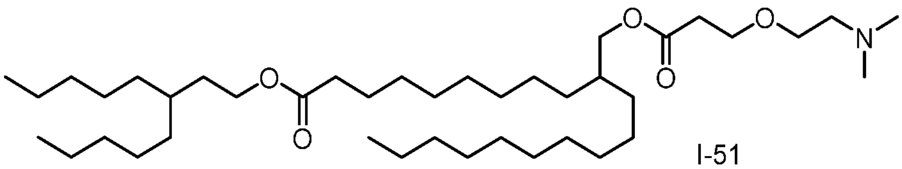

I-51

Step 80 Synthesis of Compound I-51

Compound I-51 (0.406 g, yield 80%) was obtained as a slightly colored clear oil in the same manner as in Step 17 using Compound 25 instead of Compound 18 and Compound 36 instead of Compound 17 in Step 17.
$^1$H-NMR ($CDCl_3$) δ: 0.87 (m, 9H), 1.25-1.62 (m, 52H), 2.25-2.30 (m, 8H), 2.47 (t, 2H), 2.58 (t, 2H), 3.53 (t, 2H), 3.71 (t, 2H), 3.98 (d, 2H), 4.06 (t, 2H).
ESI-MS (m/z): 668 (M+1).

[Example 52]

Synthesis of Compound I-52

[Chemical Formula 110]

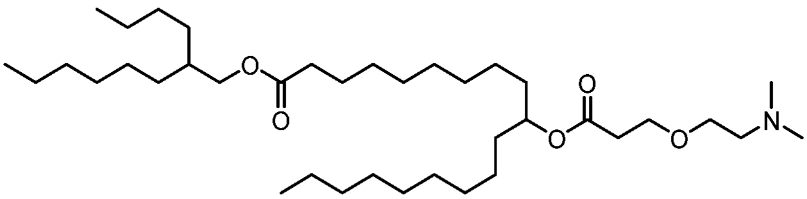

I-52

Step 81 Synthesis of Compound I-52

Compound I-52 (52.5 mg, yield 41%) was obtained in the same manner as in Step 17 using Compound 25 instead of Compound 18 and Compound 4 instead of Compound 17 in Step 17.
$^1$H-NMR ($CDCl_3$) δ: 0.86-0.91 (m, 9H), 1.18-1.35 (m, 42H), 1.45-1.55 (m, 4H), 1.55-1.66 (m, 1H), 2.25 (s, 6H), 2.29 (t, 2H), 2.48 (t, 2H), 2.58 (t, 2H), 3.54 (t, 2H), 3.72 (t, 2H), 3.97 (d, 2H), 4.88 (m, 1H).
ESI-MS (m/z): 627 (M+1)

[Example 53]

Synthesis of Compound I-53

[Chemical Formula 111]

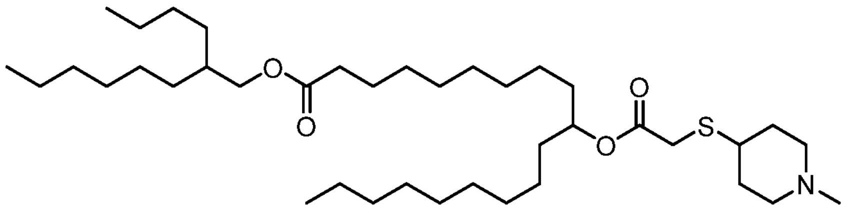

I-53

Step 82 Synthesis of Compound I-53

Compound I-53 (139.8 mg, yield 52%) was obtained in the same manner as in Step 17 using Compound 4 instead of Compound 17 in Step 17. $^1$H-NMR ($CDCl_3$) δ: 0.84-0.93 (m, 9H), 1.20-1.37 (m, 42H), 1.46-1.56 (m, 4H), 1.56-1.69 (m, 3H), 1.93-2.08 (m, 4H), 2.26 (s, 3H), 2.29 (t, 2H), 2.73-2.84 (m, 3H), 3.23 (s, 2H), 3.97 (d, 2H), 4.89 (m, 1H).
ESI-MS (m/z): 655 (M+1).

[Example 54]

Synthesis of Compound I-54

[Chemical Formula 112]

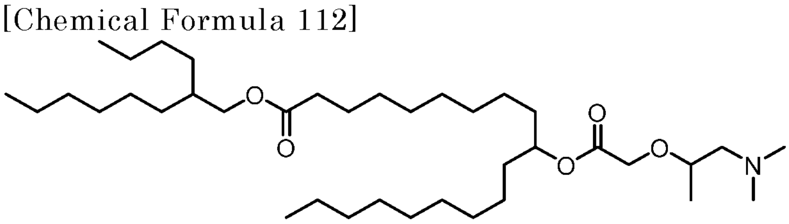

I-54

Step 83 Synthesis of Compound I-54

Compound I-54 (74.3 mg, total yield 14%) was obtained in the same manner as in Step 17 using Compound 45 instead of Compound 18 and Compound 4 instead of Compound 17 in Step 17.
$^1$H-NMR ($CDCl_3$) δ: 0.83-0.94 (m, 9H), 1.19 (d, 3H), 1.21-1.36 (m, 42H), 1.47-1.56 (m, 4H), 1.56-1.66 (m, 1H), 2.18-2.32 (m, 9H), 2.48-2.55 (m, 1 H), 3.65-3.75 (m, 1H), 3.97 (d, 2H), 4.10-4.22 (m, 2H), 4.94 (m, 1H)
ESI-MS (m/z): 627 (M+1).

[Example 55]

Synthesis of Compound I-55

[Chemical Formula 113]

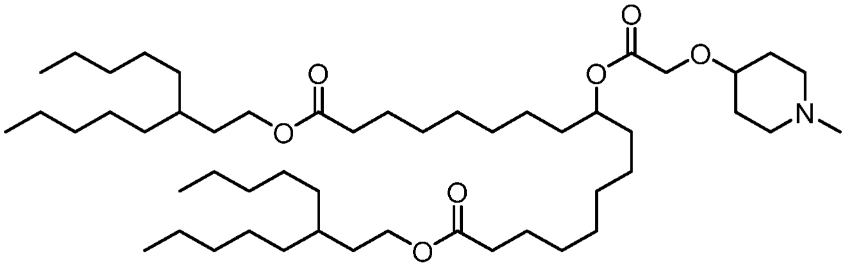

I-55

Step 84

Compound I-55 (188 mg, yield 76%) was obtained as a clear oil in the same manner as in Step 17 using Compound 21 instead of Compound 17 and Compound 26 instead of Compound 18 in Step 17.
$^1$H-NMR ($CDCl_3$) δ: 0.88 (t, 12H), 1.20-1.42 (m, 53H), 1.48-1.75 (m, 32H), 1.92 (m, 2H), 2.10 (m, 2H), 2.28 (m, 7H), 2.70 (brs, 2H), 3.41 (m, 1H), 4.08 (t, 6H), 4.94 (m, 1H).
ESI-MS (m/z): 837 (M+1).

[Example 56]

Synthesis of Compound I-56

[Chemical Formula 114]

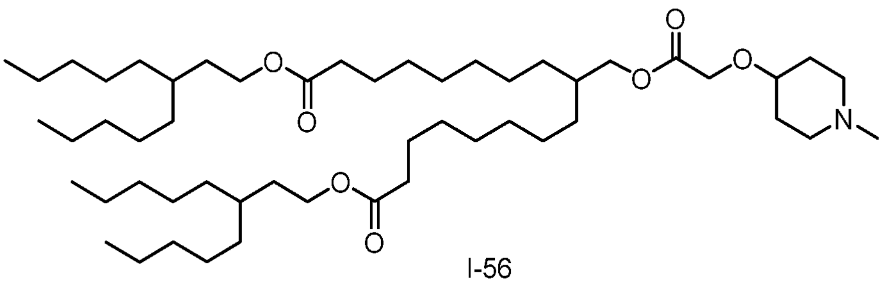

I-56

Step 85

Compound I-56 (162 mg, yield 81%) was obtained as a clear oil in the same manner as in Step 17 using Compound 29 instead of Compound 17 and Compound 26 instead of Compound 18 in Step 17.
$^1$H-NMR ($CDCl_3$) δ: 0.88 (t, 12H), 1.20-1.45 (m, 59H), 1.50-1.75 (m, 34H), 1.92 (m, 2H), 2.10 (m, 2H), 2.28 (m, 7H), 2.70 (brs, 2H), 3.42 (m, 1H), 4.08 (m, 8H). ESI-MS (m/z): 851 (M+1).

[Example 57]

Synthesis of Compound I-80

[Chemical Formula 115]

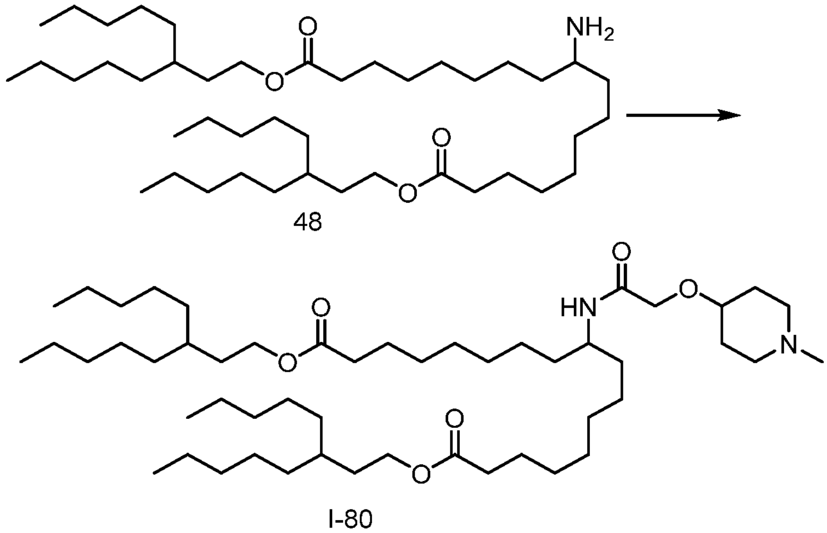

48

I-80

Step 86 Synthesis of Compound 48

To a solution of Compound 21 (510 mg, 0.75 mmol) in dichloromethane (3.7 mL) were added methanesulfonyl chloride (0.070 mL, 0.898 mmol) and triethylamine (0.125 mL, 0.898 mmol) under ice-cooling and the mixture was stirred for 2 hours. To the reaction mixture were added methanesulfonyl chloride (0.023 mL, 0.299 mmol) and triethylamine (0.042 mL, 0.299 mmol) and the mixture was stirred for 15 minutes. Methanol was added to the reaction mixture and the mixture was stirred at room temperature for 15 minutes. Saturated aqueous solution of sodium hydrogen carbonate was added to the reaction mixture and the mixture was extracted with chloroform. The organic layer was distilled off under reduced pressure and the obtained residue was purified by silica-gel column chromatography (n-hexane-ethyl acetate). The solvent in the fractions containing the desired product was distilled off under reduced pressure.

To a solution of the obtained residue in DMF (3.7 mL) was added sodium azide (97 mg, 1.498 mmol) and the mixture was stirred at 80°C for 75 minutes. After cooling the reaction mixture to room temperature, water was added to the reaction mixture and the mixture was extracted with ethyl acetate. The organic layer was distilled off under reduced pressure and the obtained residue was purified by silica-gel column chromatography (n-hexane-ethyl acetate). The solvent in the fractions containing the desired product was distilled off under reduced pressure.

To a solution of the obtained residue in tetrahydrofuran (7.0 mL) were added Pd-C (746 mg, 0.70 mmol) and hydrochloric acid-1,4-dioxane solution (0.88 mL, 3.50 mmol) and the mixture was stirred under hydrogen atmosphere at room temperature for 3 hours. The reaction mixture was filtered through Celite and the solvent was distilled off under reduced pressure. To a solution of the obtained residue in tetrahydrofuran (7.0 mL) were added Pd-C (746 mg, 0.70 mmol) and hydrochloric acid-1,4-dioxane solution (0.88 mL, 3.50 mmol) and the mixture was stirred under hydrogen atmosphere at room temperature for 2 hours. The reaction mixture was filtered through Celite and the solvent was distilled off under reduced pressure. The obtained residue was purified by aminosilica-gel column chromatography (n-hexane-ethyl acetate) to give Compound 48 (185 mg, total yield 37%) as a clear oil.

$^1$H-NMR ($CDCl_3$) δ: 0.87 (t, 12H), 1.18-1.42 (m, 58H), 1.50-1.63 (m, 10H), 2.28 (t, 4H), 2.66 (brs, 1H), 4.08 (t, 4H).

ESI-MS (m/z): 681 (M+1).

Step 87 Synthesis of Compound I-80

To a solution of Compound 48 (92 mg, 0.135 mmol) in NMP (1.4 mL) were added Compound 26 (43 mg, 0.203 mmol), PyBOP (106 mg, 0.203 mmol) and DIEA (0.11 mL, 0.609 mmol) and the mixture was stirred at room temperature for 2.5 hours. Saturated aqueous solution of sodium hydrogen carbonate was added to the reaction mixture and the mixture was extracted with ethyl acetate. The organic layer was washed with saturated aqueous solution of sodium chloride and distilled off under reduced pressure. The obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound I-80 (94 mg, yield 83%) as a clear oil.

$^1$H-NMR ($CDCl_3$) δ: 0.88 (t, 12H), 1.20-1.42 (m, 55H), 1.48-1.70 (m, 28H), 1.92 (m, 2H), 2.12 (m, 2H), 2.28 (m, 7H), 2.68 (brs, 2H), 3.37 (m, 1H), 3.94 (m, 3H), 4.08 (t, 4H), 6.28 (d, 1H).

ESI-MS (m/z): 836 (M+1).

[Example 58]

Synthesis of Compound I-65

[Chemical Formula 116]

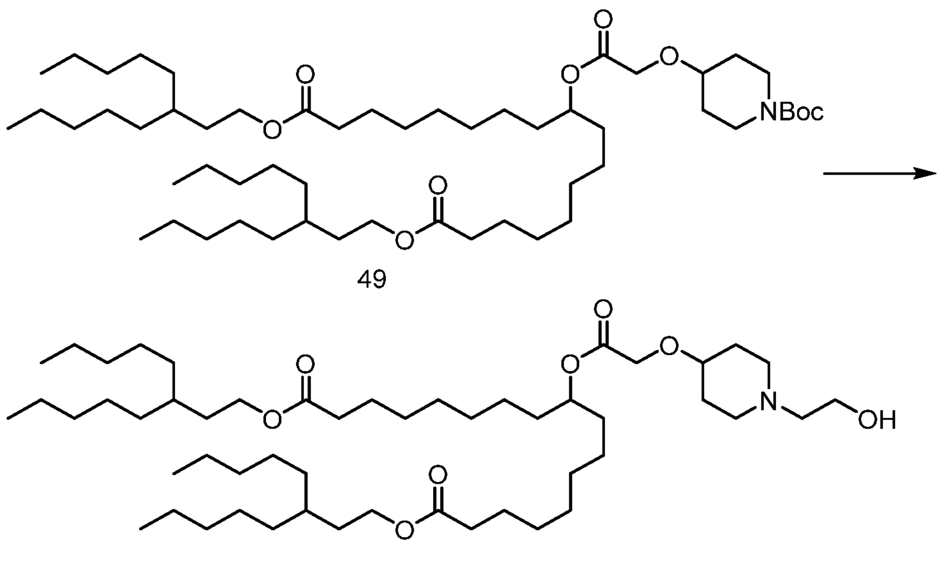

49

I-65

Step 88 Synthesis of Compound 49

To a solution of Compound 21 (300 mg, 0.440 mmol) in dichloromethane (6 mL) were added N-Boc-4-carboxymethoxypiperidine (171 mg, 0.661 mmol), 2-methyl-6-nitrobenzoic anhydride (455 mg, 1.321 mmol), DIPA (0.49 mL, 2.64 mmol) and DMAP (11 mg, 0.088 mmol) and the mixture was stirred at room temperature for 15 hours. Saturated aqueous solution of sodium hydrogen carbonate was added to the reaction mixture and the mixture was extracted with ethyl acetate. The organic layer was washed with saturated aqueous solution of sodium chloride and distilled off under reduced pressure. The obtained residue was purified by silica-gel column chromatography (n-hexane-ethyl acetate) to give Compound 49 (390 mg, yield 96%) as a clear oil.
$^1$H-NMR ($CDCl_3$ )δ: 0.88(t, 14H), 1.20-1.42 (m, 58H), 1.45 (s, 11H), 1.48-1.70 (m, 26H), 1.85 (brs, 2H), 2.26 (t, 4H), 3.07 (m, 2H),3.55 (m, 1H), 3.76 (brs, 2H), 4.11 (m, 6H), 4.95 (m, 1H)
ESI-MS (m/z): 923(M+1).

Step 89 Synthesis of Compound I-65

To Compound 49 (190 mg, 0.206 mmol) was added 4mol/L hydrochloric acid-1,4-dioxane solution (1 mL). The mixture was stirred at room temperature for 4 hours and distilled off under reduced pressure. To a solution of the obtained residue in tetrahydrofuran (3 mL) were added TBAI (16 mg, 0.044 mmol), triethylamine (0.243 mL, 1.75 mmol) and 2-bromoethanol (0.062 mL, 0.876 mmol) and the mixture was stirred at room temperature for 72 hours. Saturated aqueous solution of sodium hydrogen carbonate was added to the reaction mixture and the mixture was extracted with ethyl acetate. The organic layer was washed with saturated aqueous solution of sodium chloride and distilled off under reduced pressure. The obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound I-65 (74 mg, yield 39%) as a clear oil.
$^1$H-NMR ($CDCl_3$) δ: 0.89 (t, 12H), 1.20-1.42 (m, 52H), 1.48-1.70 (m, 18H), 1.93 (m, 2H), 2.28 (m, 6H), 2.53 (m, 2H), 2.78 (m, 2H) 3.43 (m, 1H), 3.53 (t, 2H), 4.07 (t, 6H), 4.92 (m, 1H).
ESI-MS (m/z): 867 (M+1).

[Example 59]

Synthesis of Compound I-61

[Chemical Formula 117]

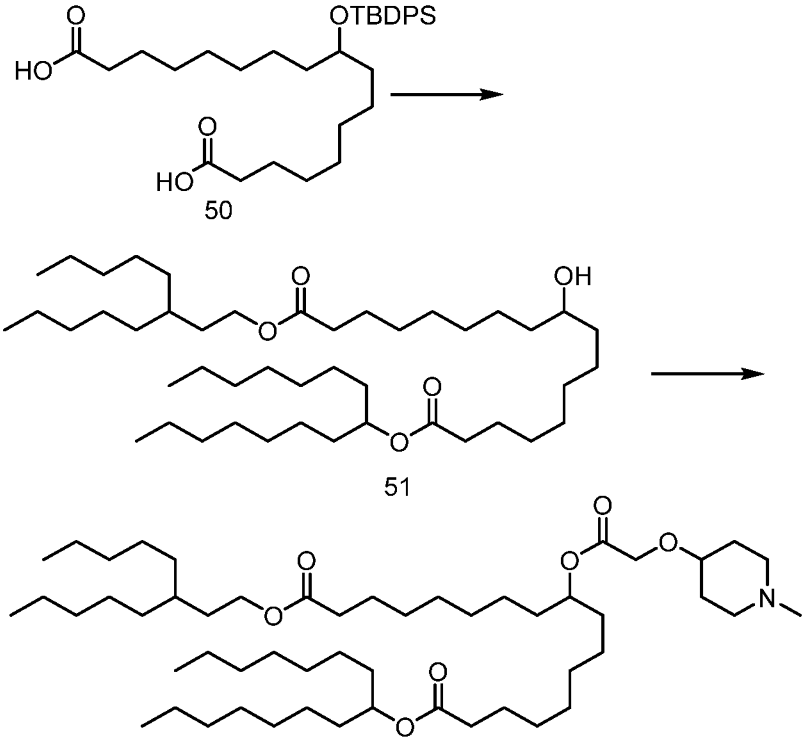

50

51

I-61

Step 90 Synthesis of Compound 51

To a solution of Compound 50 (500 mg, 0.901 mmol) in dichloromethane (10 mL) were added pentadecan-8-ol (103 mg, 0.451 mmol), 2-methyl-6-nitrobenzoic anhydride (155 mg, 0.451 mmol), DIPA (0.472 mL, 2.70 mmol) and DMAP (22 mg, 0.18 mmol) and the mixture was stirred at room temperature for 20 hours. The organic layer was washed with saturated aqueous solution of sodium chloride and distilled off under reduced pressure. The obtained residue was purified by silica-gel column chromatography (n-hexane-ethyl acetate). The solvent in the fractions containing the desired product was distilled off under reduced pressure. To a solution of the obtained residue in dichloromethane (3 mL) were added 3-pentyloctan-1-ol (82 mg, 0.412 mmol), 2-methyl-6-nitrobenzoic anhydride (189 mg, 0.549 mmol), DIPA (0.192 mL, 1.1 mmol) and DMAP (7 mg, 0.055 mmol) and the mixture was stirred at room temperature for 20 hours. The organic layer was washed with saturated aqueous solution of sodium chloride and distilled off under reduced pressure. The obtained residue was purified by silica-gel column chromatography (chloroform-methanol). The solvent in the fractions containing the desired product (205 mg, 0.216 mmol) was distilled off under reduced pressure. To a solution of the obtained residue in tetrahydrofuran (2 mL) was added TBAF-tetrahydrofuran solution (0.649 mL, 0.649 mmol) and the mixture was stirred at room temperature for 20 hours. Ethyl acetate was added to the reaction mixture and the mixture was washed sequentially with saturated aqueous solution of sodium hydrogen carbonate, 2 mol/L hydrochloric acid, saturated aqueous solution of sodium hydrogen carbonate, and saturated aqueous solution of sodium chloride. The organic layer was distilled off under reduced pressure and the obtained residue was purified by silica-gel column chromatography (n-hexane-ethyl acetate) to give Compound 51 (90 mg, yield 59%).
$^1$H-NMR ($CDCl_3$) $\delta$: 0.88 (t, 12H), 1.27-1.64 (m, 73H), 2.28 (t, 4H), 3.57 (brs, 1H), 4.11 (t, 2H), 4.88 (m, 1H).
ESI-MS (m/z): 710 (M+1).

Step 91 Synthesis of Compound I-61

To a solution of Compound 51 (143 mg, 0.202 mmol) in dichloromethane (3 mL) were added Compound 26 (64 mg, 0.302 mmol), 2-methyl-6-nitrobenzoic anhydride (208 mg, 0.605 mmol), DIPA (0.211 mL, 1.21 mmol) and DMAP (5 mg, 0.04 mmol) and the mixture was stirred at room temperature for 16 hours. Saturated aqueous solution of sodium hydrogen carbonate was added to the reaction mixture and the mixture was extracted with ethyl acetate. The organic layer was washed with saturated aqueous solution of sodium chloride and distilled off under reduced pressure. The obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound I-61 (127 mg, yield 73%) as a clear oil.
$^1$H-NMR ($CDCl_3$) $\delta$: 0.88 (t, 13H), 1.20-1.42 (m, 54H), 1.54-1.70 (m, 21H), 1.92 (m, 2H), 2.11 (m, 2H), 2.26(m, 7H),2.70 (brs, 2H), 3.40 (m, 1H), 4.13 (m, 4H), 4.95 (m, 2H)
ESI-MS (m/z): 865 (M+1).

[Example 60]

Synthesis of Compound I-73

[Chemical Formula 118]

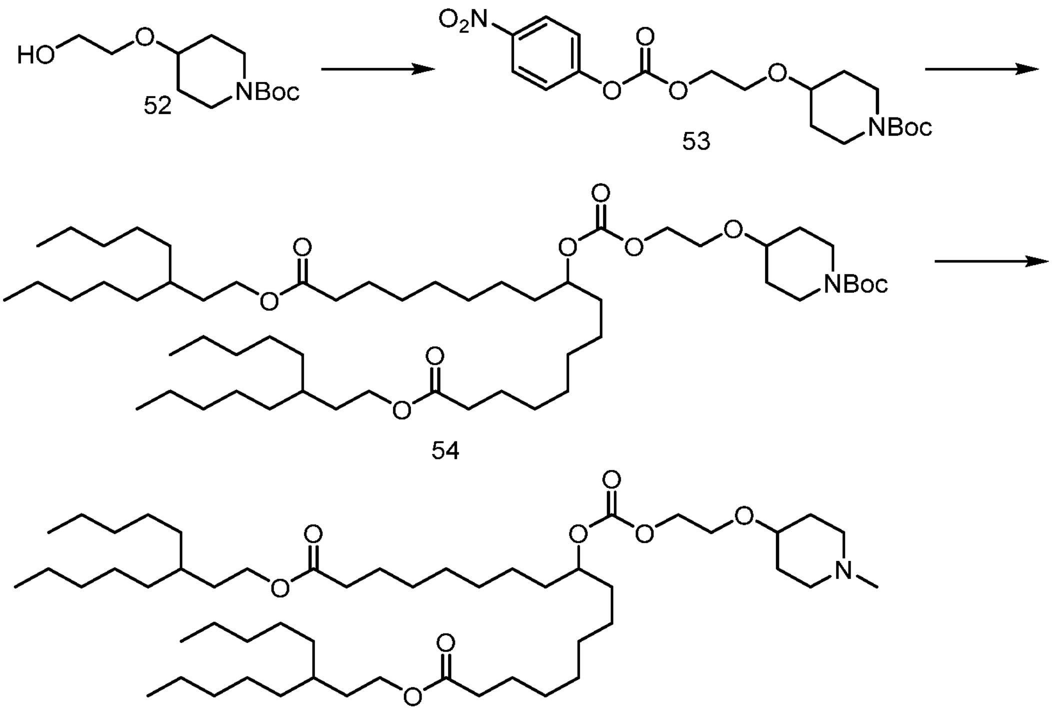

52

53

54

I-73

Step 92 Synthesis of Compound 53

To a solution of Compound 52 (254 mg, 1.04 mmol) in dichloromethane (2.6 mL) were added 4-nitrophenyl chloroformate (250 mg, 1.24 mmol) and triethylamine (0.172 mL, 1.24 mmol) under ice-cooling and the mixture was stirred at room temperature for 90 minutes. To the reaction mixture were added 4-nitrophenyl chloroformate (250 mg, 1.24 mmol), triethylamine (0.172 mL, 1.24 mmol) and dichloromethane (1.6 mL) and the mixture was stirred for 90 minutes. To the reaction mixture were added 4-nitrophenyl chloroformate (125 mg, 0.62 mmol), triethylamine (0.086 mL, 0.62 mmol) and dichloromethane (1.0 mL) and the mixture was stirred for 90 minutes. Water was added to the reaction mixture and the mixture was extracted with chloroform. The organic layer was washed with saturated aqueous solution of sodium hydrogen carbonate and distilled off under reduced pressure. The obtained residue was purified by silica-gel column chromatography (n-hexane-ethyl acetate) to give Compound 53 (386 mg, yield 91%) as a clear oil. $^1$H-

NMR ($CDCl_3$) δ: 1.46 (s, 9H), 1.54-1.57 (m, 8H), 1.85 (brs, 2H), 3.12 (m, 2H), 3.54 (m, 1H), 3.77 (t, 4H), 4.43 (t, 2H), 7.39 (dd, 2H), 8.28 (dd, 2H).
ESI-MS (m/z): 411 (M+1).

Step 93 Synthesis of Compound 54

To a solution of Compound 21 (61 mg, 0.089 mmol) in dichloromethane (1.8 mL) were added Compound 53 (147 mg, 0.358 mmol), DIEA (0.063 mL, 0.358 mmol) and DMAP (44 mg, 0.358 mmol) and the mixture was heated under reflux for 13 hours. After cooling the reaction mixture to room temperature, the mixture was washed with water. The organic layer was distilled off under reduced pressure and the obtained residue was purified by silica-gel column chromatography (n-hexane-ethyl acetate) to give Compound 54 (76 mg, yield 89%) as a clear oil.
$^1$H-NMR ($CDCl_3$) δ: 0.88 (t, 12H), 1.25-1.45 (m, 63H), 1.51-1.60 (m, 47H), 1.80 (brs, 2H), 2.28 (t, 4H), 3.07 (m, 2H), 3.49 (m, 1H), 3.69 (t, 2H), 3.73 (brs, 2H), 4.08 (t, 4H), 4.26 (t, 2H), 4.67 (m, 1H).
ESI-MS (m/z): 953 (M+1).

Step 94 Synthesis of Compound I-73

Compound I-73 (57 mg, total yield 82%) was obtained as a clear oil in the same manner as in Step 4 using Compound 54 instead of Compound 5 in Step 4.
$^1$H-NMR ($CDCl_3$) δ: 0.88 (t, 12H), 1.22-1.42 (m, 52H), 1.54-1.68 (m, 32H), 1.88 (d, 2H), 2.10 (t, 2H), 2.28 (m, 7H), 2.68 (brs, 2H), 3.34 (m, 1H), 3.67 (t, 2H), 4.08 (t, 4H), 4.26 (t, 2H), 4.67 (m, 1H).
ESI-MS (m/z): 867 (M+1).

[Example 61]

Synthesis of Compound II-8

[Chemical Formula 119]

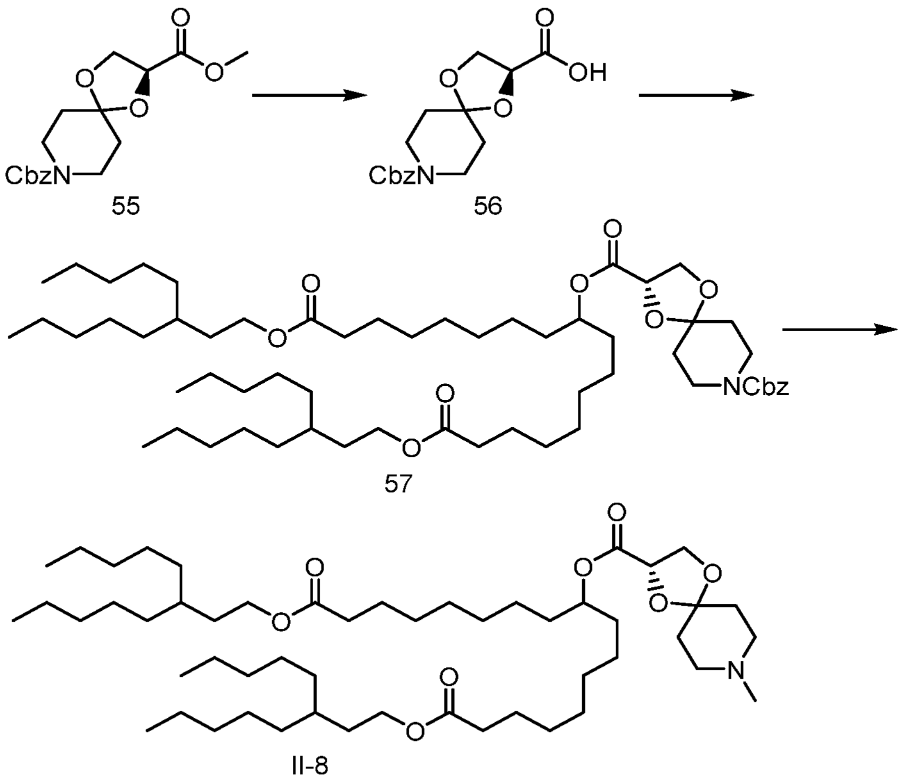

55

56

57

II-8

Step 95 Synthesis of Compound 56

To a solution of L-glyceric acid methyl ester (300 mg, 2.50 mmol) in toluene (5.0 mL) were added 1-carbobenzoxy-4-piperidone (0.50 mL, 2.50 mmol) and p-toluenesulfonic acid monohydrate (47.5 mg, 0.25 mmol) and the mixture was heated under reflux using a Dean-Stark apparatus for 12 hours. After cooling the reaction mixture to room temperature, saturated aqueous solution of sodium hydrogen carbonate was added to the reaction mixture and the mixture was extracted with ethyl acetate. The organic layer was washed with saturated aqueous solution of sodium chloride and distilled off under reduced pressure. The obtained residue was purified by silica-gel column chromatography (n-hexane-ethyl acetate) and the solvent in the fractions containing Compound 55 was distilled off under reduced pressure. To a mixture of Compound 55 in tetrahydrofuran/methanol/water (8.4 mL, 1:2:1) was added lithium hydroxide monohydrate (352 mg, 8.39 mmol) and the mixture was stirred at room temperature for 17 hours. After the reaction mixture was distilled off under reduced pressure to remove the organic solvent, water was added to the mixture and the mixture was washed with chloroform. Saturated aqueous solution of citric acid was added to the aqueous layer to adjust the pH to 3, and the aqueous layer was extracted with chloroform. The organic layer was washed with saturated aqueous solution of sodium chloride, dried over anhydrous magnesium sulfate and filtered. The solvent was distilled off under reduced pressure to give Compound 56 (359 mg, total yield 45%).
$^1$H-NMR ($CDCl_3$) $\delta$: 1.72-1.86 (brs, 4H), 3.55 (m, 2H), 3.72 (m, 2H), 4.22 (t, 1H), 4.30 (t, 1H), 4.66 (q, 1H), 5.14 (s, 2H), 7.31-7.39 (m, 5H).
ESI-MS (m/z): 322 (M+1).

Step 96 Synthesis of Compound 57

Compound 57 (280 mg, yield 99%) was obtained as a clear oil in the same manner as in Step 3 using Compound 21 instead of Compound 4 and Compound 56 instead of Compound 3 in Step 3.
$^1$H-NMR ($CDCl_3$) $\delta$: 0.88 (t, 12H), 1.25-1.40 (m, 52H), 1.53-1.71 (m, 30H), 2.28 (t, 4H), 3.63 (m, 4H), 4.09 (m, 5H), 4.25 (t, 1H), 4.59 (t, 1H), 4.92 (m, 1H), 5.13 (s, 2H), 7.31-7.36 (m, 5H).
ESI-MS (m/z): 985 (M+1).

Step 97 Synthesis of Compound II-8

To a solution of Compound 57 (280 mg, 0.28 mmol) in ethyl acetate (2.8 mL) was added Pd-C (15.1 mg, 0.014 mmol) and the mixture was stirred under hydrogen atmosphere at room temperature for 5 hours. The reaction mixture was filtered through Celite and the solvent was distilled off under reduced pressure. To a solution of the obtained residue in dichloromethane (5.5 mL) were added 36% formaldehyde solution (0.076 mL, 0.99 mmol) and sodium cyanoborohydride (53 mg, 0.85 mmol) and the mixture was stirred at room temperature for 4 hours. To the reaction mixture were added 36% formaldehyde solution (0.076 mL, 0.99 mmol) and sodium cyanoborohydride (53 mg, 0.85 mmol) and the mixture was stirred at room temperature for 39 hours. Saturated aqueous solution of sodium hydrogen carbonate was added to the reaction mixture and the mixture was extracted with chloroform. The organic layer was distilled off under reduced pressure and the obtained residue was purified by silica-gel column chromatography (chloroform-methanol). The solvent in the fractions containing the desired product was distilled off under reduced pressure and the obtained residue was purified by aminosilica-gel column chromatography (n-hexane-ethyl acetate) to give Compound II-8 (110 mg, total yield 45%) as a clear oil.
$^1$H-NMR ($CDCl_3$) $\delta$: 0.88 (t, 12H), 1.25-1.42 (m, 52H), 1.54-1.59 (m, 39H), 1.83 (m,2H), 1.96 (m, 1H), 2.28 (m, 7H), 2.53 (brs, 4H), 4.09 (m, 5H), 4.24 (t, 1H), 4.58 (dd, 1H), 4.92 (t, 1H).
ESI-MS (m/z): 865 (M+1).
Compounds other than those described above can also be synthesized in the same manner as the synthesis method described above.

[Chemical Formula 120]

I-74

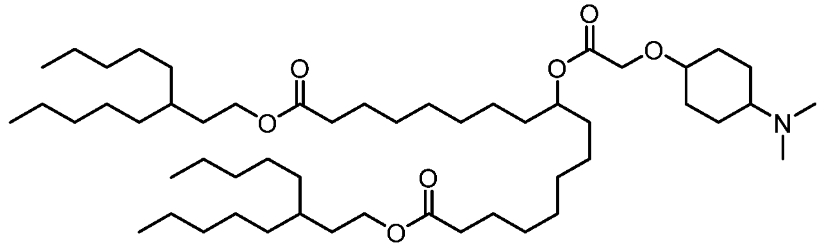

[Chemical Formula 121]

I-76

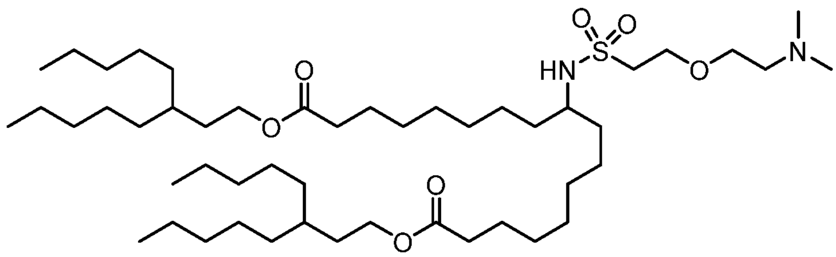

I-79

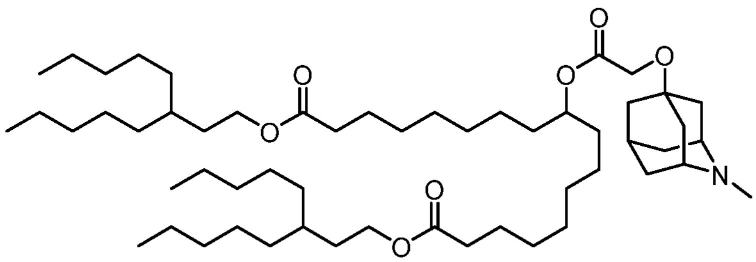

[Chemical Formula 122]

I-83

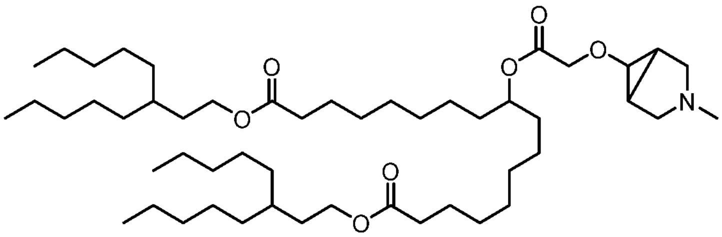

I-84

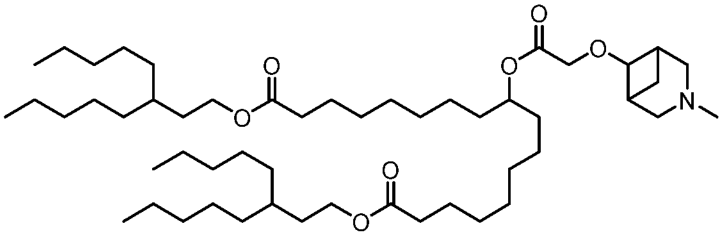

I-85

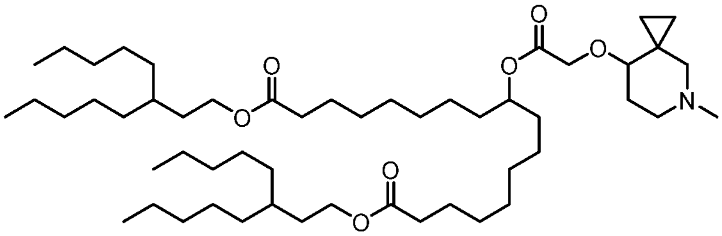

[Chemical Formula 123]

I-86

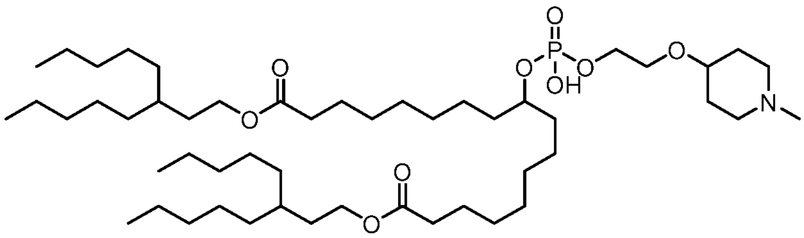

I-89

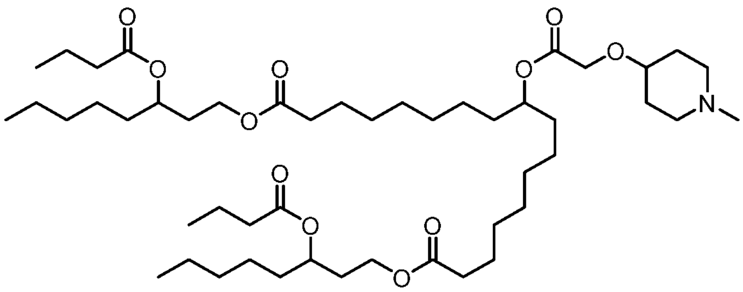

The following compounds were synthesized according to the general synthetic procedures and the method described in the examples described above. The structures and physical properties (LC/MS data, NMR spectrum) are shown in the following tables.

In the structural formula, "wedged bond" and "hashed wedged bond" indicate the steric configuration. In particular, among the compounds whose steric configurations are described, compound with "a" in item of "cfg" (configuration) means racemic compound whose relative steric configuration is determined. Compound with "c" in item of "cfg" (configuration) means that the stereo is determined as shown in the chemical structure.

In addition, among the compounds in which the bond that forms the asymmetric carbon is indicated by a solid line, compound with "d" in item of "cfg" (configuration) means racemic compound.

[Table 1]

| Compound No. | Structure | LCMS Method | Retention Time (min) | [M+H] | cfg |
|---|---|---|---|---|---|
| I-57 | 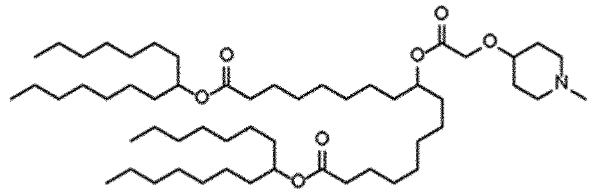 | 2 | 1.07 | 893 | |
| I-58 | 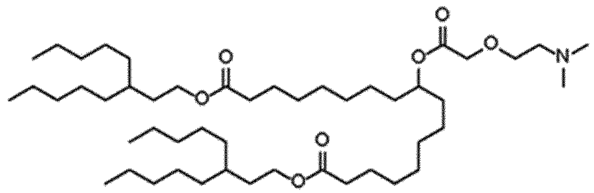 | 2 | 1.97 | 811 | |
| I-59 | 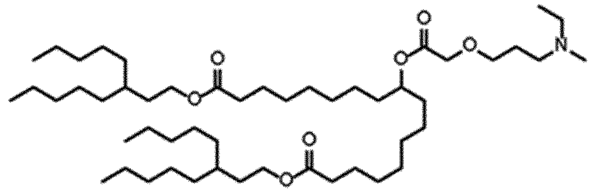 | 2 | 2.34 | 840 | |
| I-60 | 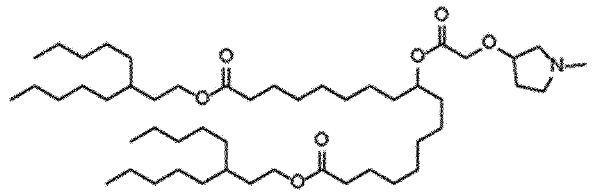 | 2 | 2.20 | 823 | |
| I-61 | 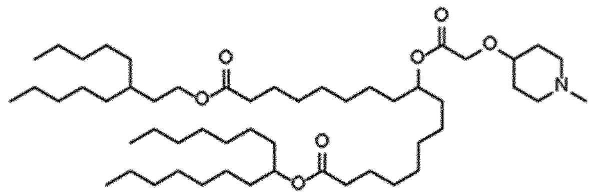 | 2 | 1.03 | 865 | d |

(continued)

| Compound No. | Structure | LCMS Method | Retention Time (min) | [M+H] | cfg |
|---|---|---|---|---|---|
| I-82 | 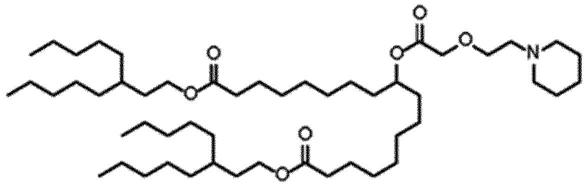 | 2 | 2.25 | 851 | |
| I-63 | 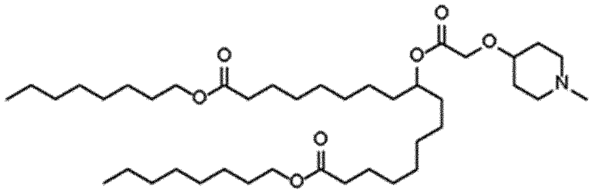 | 2 | 0.72 | 697 | |

Table 2]

| | | | | | |
|---|---|---|---|---|---|
| I-64 | 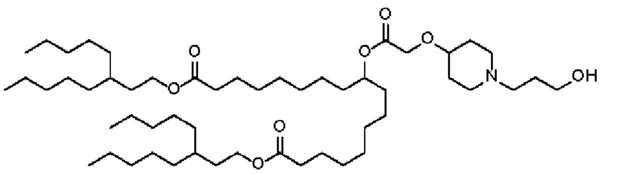 | 2 | 0.58 | 881 | |
| I-65 | 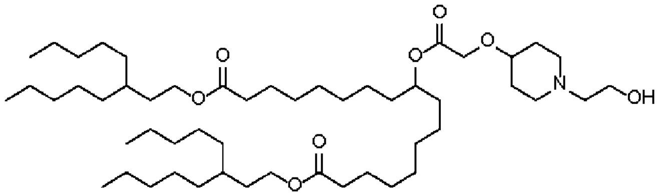 | 2 | 0.62 | 867 | |
| I-66 | 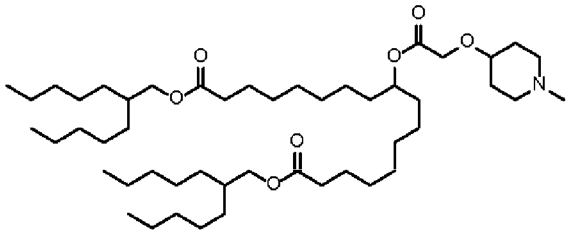 | 2 | 2.13 | 809 | |
| I-67 | 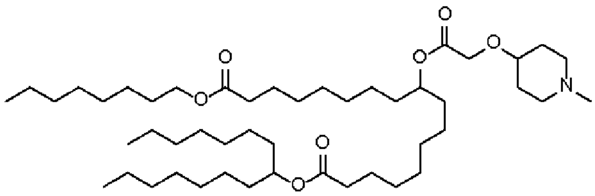 | 2 | 0.89 | 795 | d |
| I-68 | 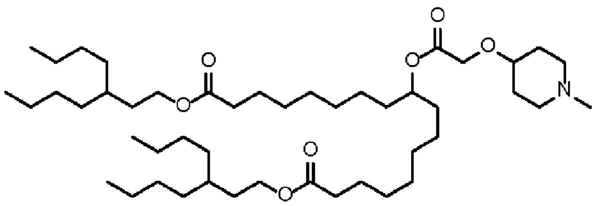 | 2 | 0.86 | 781 | |
| I-69 | 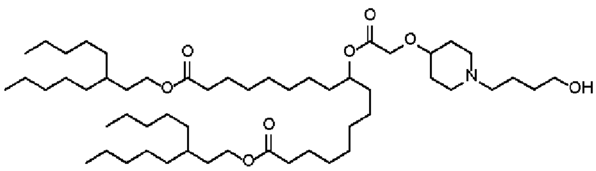 | 2 | 2.08 | 895 | |

(continued)

| | | | | | |
|---|---|---|---|---|---|
| I-70 | 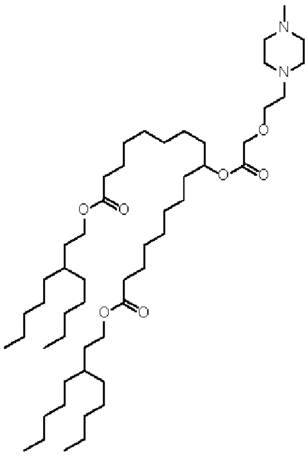 | 2 | 1.99 | 866 | |

[Table 3]

| | | | | | |
|---|---|---|---|---|---|
| I-71 | 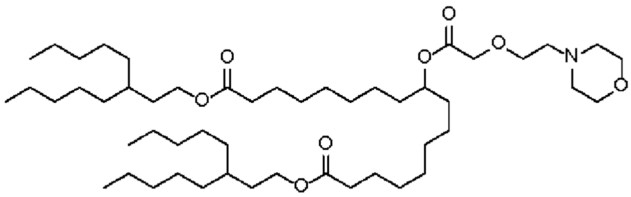 | 2 | 1.28 | 853 | |
| I-72 | 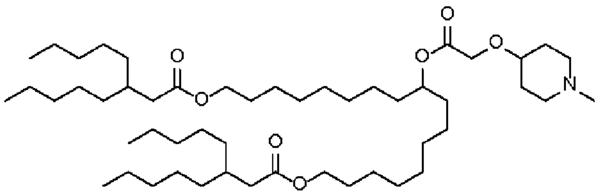 | 2 | 2.18 | 837 | |
| I-73 | 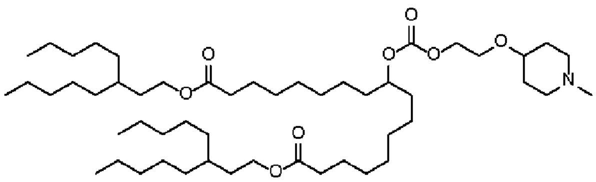 | 2 | 2.23 | 867 | |
| I-75 | 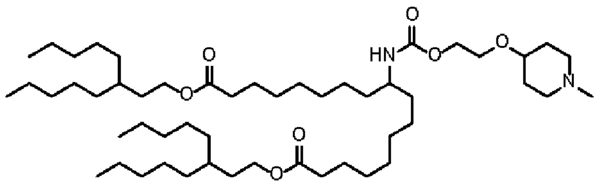 | 2 | 2.13 | 866 | |

(continued)

| | | | | | |
|---|---|---|---|---|---|
| I-77 | 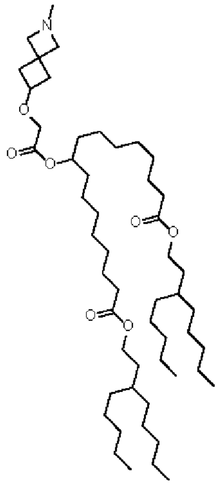 | 2 | 2.44 | 849 | |
| I-78 | 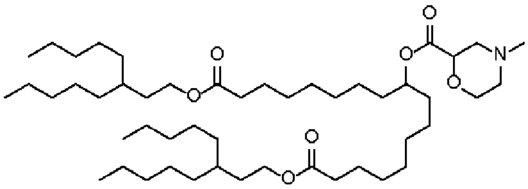 | 2 | 1.33 | 809 | |
| I-80 | 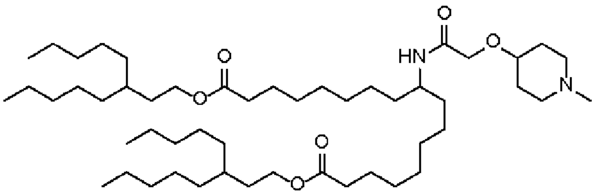 | 2 | 2.02 | 836 | |

Table 4]

| | | | | | |
|---|---|---|---|---|---|
| I-81 | 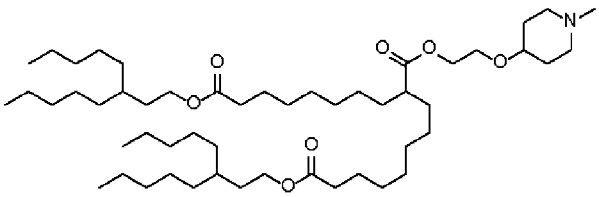 | 2 | 2.20 | 851 | |
| I-82 | 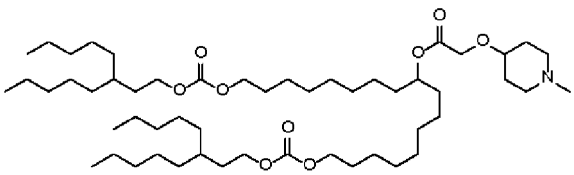 | 2 | 2.35 | 897 | |

(continued)

| | | | | | |
|---|---|---|---|---|---|
| I-87 | 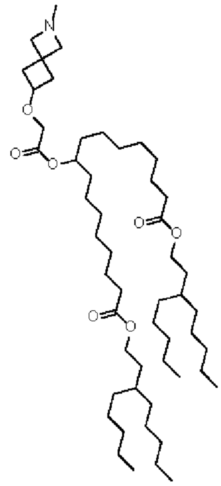 | 2 | 2.70 | 863 | |
| I-88 | 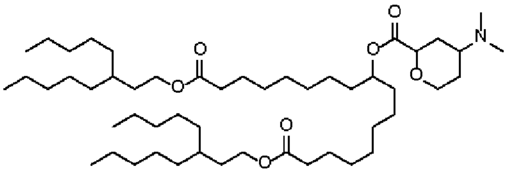 | 2 | 1.78 | 837 | |
| II-1 | 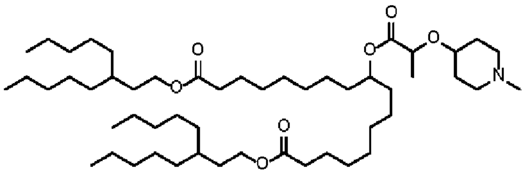 | 2 | 2.09 | 851 | d |
| II-2 | 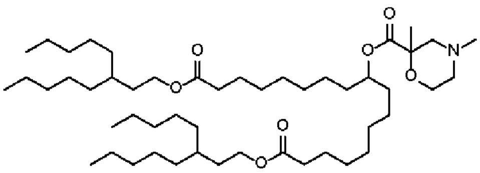 | 2 | 1.42 | 823 | |
| II-3 | 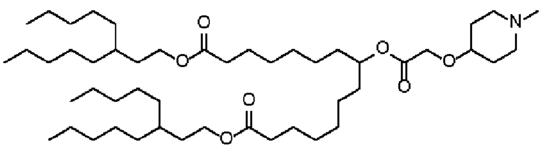 | 3 | 3.38 | 809 | |
| II-4 | 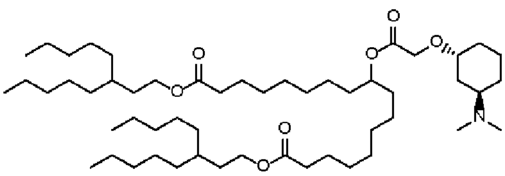 | 2 | 2.56 | 865 | a |

[Table 5]

| | | | | | |
|---|---|---|---|---|---|
| II-5 | 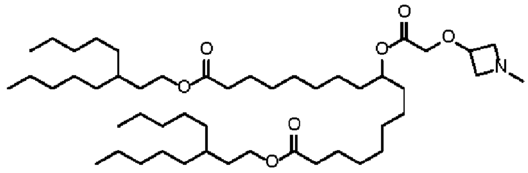 | 2 | 2.11 | 809 | |
| II-6 | 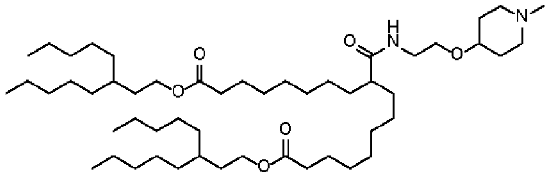 | 2 | 2.09 | 850 | |
| II-7 | 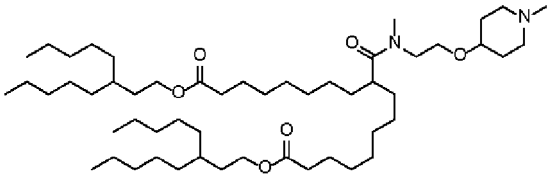 | 2 | 2.21 | 864 | |
| II-8 | 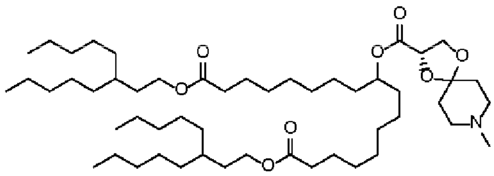 | 2 | 1.90 | 865 | c |
| II-9 | 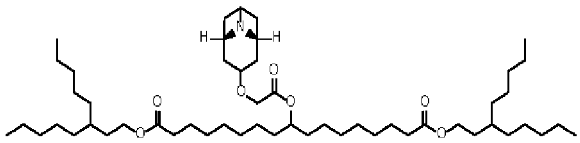 | 2 | 2.79 | 878 | c |
| II-10 | 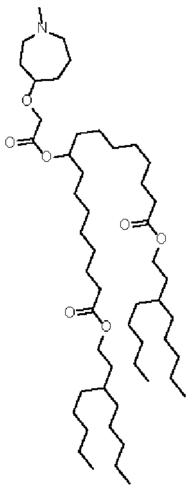 | 2 | 2.84 | 851 | d |

(continued)

| | | | | | |
|---|---|---|---|---|---|
| II-11 | 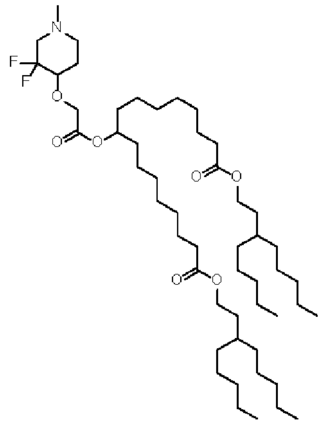 | 2 | 1.28 | 873 | d |

[Table 6]

| | | | | | |
|---|---|---|---|---|---|
| II-12 | 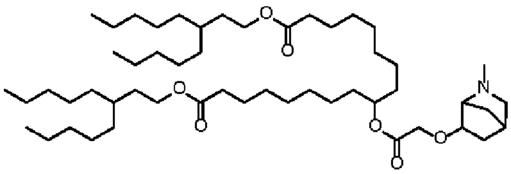 | 2 | 2.28 | 849 | d |
| II-13 | 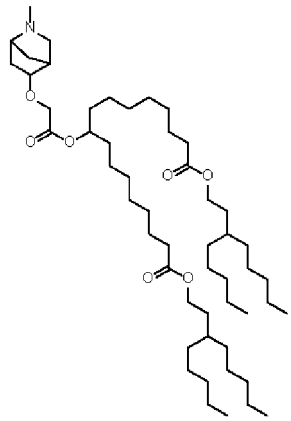 | 2 | 2.94 | 849 | d |
| II-14 | 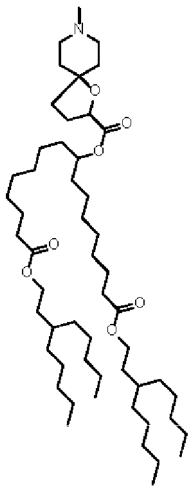 | 2 | 2.67 | 863 | d |

(continued)

| | | | | | |
|---|---|---|---|---|---|
| II-15 | 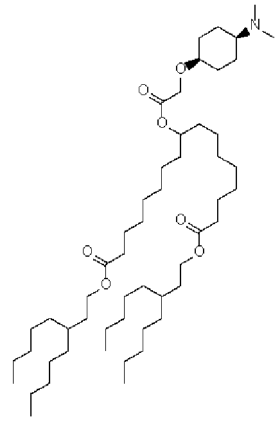 | 2 | 2.76 | 865 | c |

[Table 7]

| | | | | | |
|---|---|---|---|---|---|
| II-16 | 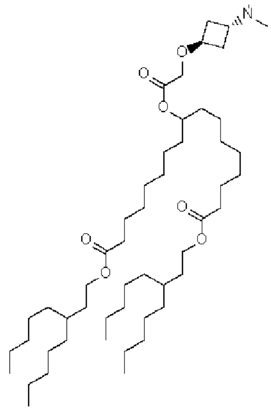 | 2 | 1.98 | 837 | a |
| II-17 | 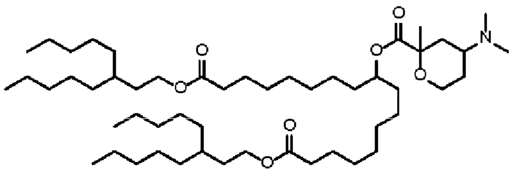 | 2 | 1.80 | 851 | d |
| II-18 | 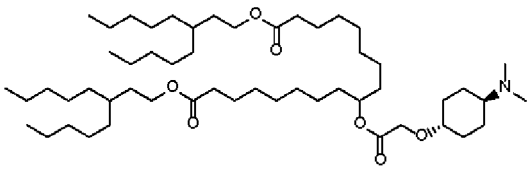 | 2 | 2.67 | 865 | a |
| II-19 | 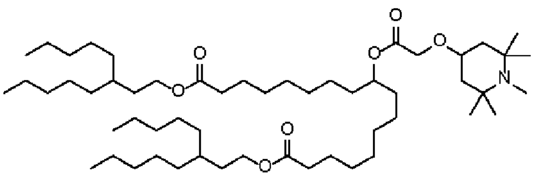 | 2 | 2.13 | 894 | |

(continued)

| | | | | | |
|---|---|---|---|---|---|
| II-20 | 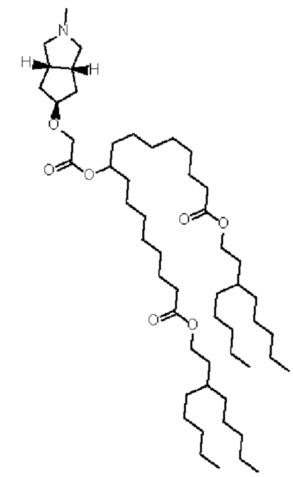 | 2 | 2.32 | 863 | a |
| II-21 | 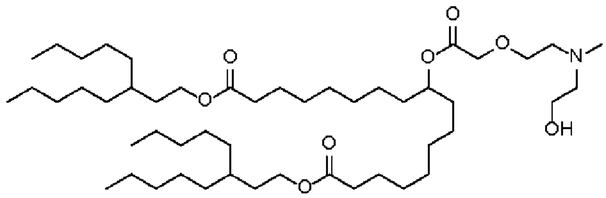 | 2 | 1.59 | 841 | |

[Table 8]

| | | | | | |
|---|---|---|---|---|---|
| II-22 | 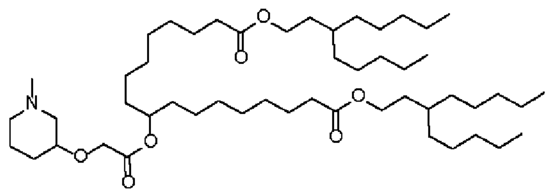 | 2 | 1.83 | 837 | d |
| II-23 | 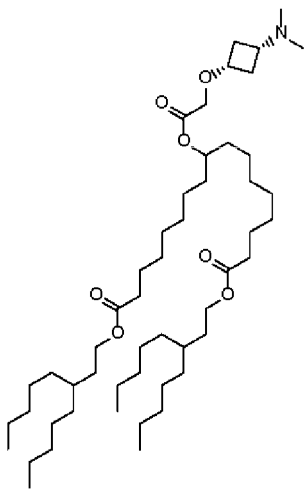 | 2 | 1.78 | 837 | c |

[Table 9]

| Compound No. | NMR |
|---|---|
| I-57 | δ : 0.88 (t, 12H), 1.25 (m, 56H), 1.51 (m, 12H), 1.60 (m, 6H), 1.70 (m, 2H), 1.93 (m, 2H), 2.10 (m, 2H), 2.27 (m, 7H), 2.71 (brs, 2H), 3.40 (m, 1H), 4.09 (s, 2H), 4.90 (m, 3H) |
| 1-58 | δ : 0.88 (t, 13H), 1.25-1.33 (m, 49H), 1.40 (t, 2H), 1.51-1.62 (m, 32H), 2.28 (m, 10H), 2.56 (t, 2H), 3.65 (t, 2H), 4.08 (m, 6H), 4.95 (t, 1H). |
| I-59 | δ : 0.88 (t, 12H), 1.05 (t, 3H), 1.22-1.42 (m, 52H), 1.52-1.61 (m, 22H), 1.81 (m, 2H), 2.21 (s, 3H), 2.28 (t, 4H), 2.42(m, 4H), 3.57 (t, 2H), 4.08 (m, 6H), 4.95 (t, 1H). |
| I-60 | δ : 0.88 (t, 12H), 1.25-1.40 (m, 56H), 1.52-1.57 (m, 24H), 1.89 (m, 1H), 2.12 (m, 1H), 2.28 (t, 4H), 2.35 (s, 3H), 2.42 (m, 1H), 2.66 (m, 3H), 4.03 (s, 2H), 4.08 (t, 4H), 4.15 (m, 1H), 4.93 (m, 1H). |
| I-62 | δ : 0.88 (t, 12H), 1.22-1.44 (m, 55H), 1.52-1.62 (m, 44H), 2.28 (t, 4H), 2.44 (brs, 4H), 2.59 (t, 2H), 3.67 (t, 2H), 4.08 (t, 6H), 4.94 (t, 1H). |
| I-63 | δ : 0.88 (t, 6H), 1.31 (m, 38H), 1.52-1.71 (m, 18H), 1.93 (m, 2H), 2.11 (m, 2H), 2.28 (m, 7H), 2.72 (brs, 2H), 3.40 (m, 1H), 4.07 (m, 6H), 4.94 (m, 1H). |
| I-64 | δ : 0.89 (t, 14H), 1.20-1.41 (m, 54H), 1.51-1.68 (m, 22H), 1.91 (brs, 2H), 2.27 (m, 6H), 2.61 (m, 2H), 2.83 (brs, 2H) 3.47 (brs, 1H), 3.80 (m, 2H), 4.09 (t, 6H), 4.94 (m, 1H). |
| I-66 | δ: 0.88 (t, 13H), 1.28-1.37 (m, 49H), 1.53-1.74 (m, 30H), 1.91 (brs, 2H), 2.11 (brs, 2H), 2.27 (m, 7H), 2.70 (brs, 2H), 3.38 (m, 1H), 3.96 (d, 4H), 4.08 (s, 2H), 4.93 (t, 1H). |
| I-67 | δ : 0.88 (m, 10H), 1.27 (m, 49H), 1.50-1.69 (m, 19H), 1.93 (m, 2H), 2.11 (m, 2H), 2.28 (m, 7H), 2.72 (m, 2H), 3.41 (m, 1H), 4.05 (m, 4H), 4.86 (m, 1H), 4.95 (m, 1H) |
| 1-68 | δ : 0.89 (m, 14H), 1.22-1.46 (m, 49H), 1.52-1.72 (m, 26H), 1.93 (m, 2H), 2.27 (m, 2H), 2.28 (m, 7H), 2.72 (brs, 2H), 3.41 (m, 1H), 4.09 (m, 6H), 4.95 (m, 1H). |
| I-69 | δ : 0.88 (t, 13H), 1.20-1.40 (m, 58H), 1.56-1.75 (m, 24H), 1.90 (m, 2H), 2.29 (m, 8H), 2.74 (brs, 2H), 3.50 (m, 4H), 4.08 (m, 7H), 4.92 (m, 1H). |
| I-70 | δ : 0.88 (t, 12H), 1.25-1.40 (m, 55H), 1.52-1.59 (m, 26H), 2.28 (t, 7H), 2.48 (brs, 6H), 2.64 (t, 2H), 3.68 (t, 2H), 4.08 (m, 6H), 4.94 (m, 1H). |
| I-71 | δ : 0.88 (t, 13H), 1.25-1.62 (m, 81H), 2.28 (t, 4H), 2.53 (s, 4H), 2.63 (t, 2H), 3.71 (m, 6H), 4.08 (t, 6H), 4.94 (t, 1H). |
| I-72 | δ : 0.88 (t, 14H), 1.26-1.42 (m, 55H), 1.54-1.74 (m, 37H), 1.84-1.91 (m, 4H), 2.11 (m, 2H), 2.22 (d, 4H), 2.26 (s, 3H), 2.70 (brs, 2H), 3.41 (m, 1H), 4.04 (t, 4H), 4.08 (s, 2H), 4.95 (m, 1H). |
| I-75 | δ : 0.88 (t, 14H), 1.25-1.42 (m, 67H), 1.55-1.62 (m, 100H), 1.89 (m, 2H), 2.09 (m, 2H), 2.27 (m, 7H), 2.71 (m, 2H), 3.33 (m, 1H), 3.55 (m, 1H), 3.64 (t, 2H), 4.08 (t, 4H), 4.19 (t, 2H), 4.45 (m, 1H). |
| I-77 | δ : 0.88 (t, 13H), 1.25-1.40 (m, 54H), 1.52-1.59 (m, 37H), 2.13 (m, 2H), 2.28 (m, 7H), 2.43 (m, 2H), 3.16 (d, 4H), 3.94 (m, 3H), 4.08 (t, 4H), 4.93 (m, 1H). |
| I-78 | δ : 0.88 (t, 13H), 1.25-1.42 (m, 54H), 1.54-1.60 (m, 37H), 2.25 (m, 9H), 2.56(d, 1H), 2.91 (d, 1H), 3.68 (m, 1H), 4.08 (m, 5H), 4.18 (d, 1H), 4.97 (t, 1H). |
| I-81 | δ : 0.88 (t, 13H), 1.25-1.33 (m, 51H), 1.43 (m, 5H), 1.54-1.67 (m, 28H), 1.88 (m, 2H), 2.11 (m, 2H), 2.28 (m, 7H), 2.33 (m, 1H), 2.68 (brs, 2H), 3.33 (m, 1H), 3.64 (t, 2H), 4.08 (t, 4H), 4.22 (t, 2H). |
| I-82 | δ : 0.88 (t, 14H), 1.25-1.37 (m, 54H), 1.42-1.53 (m, 6H), 1.60-1.74 (m, 10H), 1.92 (m, 2H), 2.11 (m, 2H), 2.26 (s, 3H), 2.70 (brs, 2H), 3.41 (m, 1H), 4.12 (m, 10H), 4.95 (m, 1H). |
| I-87 | δ : 0.88 (t, 13H), 1.25-1.40 (m, 53H), 1.53-1.58 (m, 29H), 2.10 (t, 2H), 2.28 (m, 7H), 2.40 (t, 2H), 2.50 (s, 4H), 3.89 (m, 1H), 4.04 (s, 2H), 4.08 (t, 4H), 4.93 (t, 1H). |
| 1-88 | δ : 0.88 (t, 14H), 1.25-1.45 (m, 55H), 1.54-1.63 (m, 54H), 1.82 (m, 3H), 2.23 (m, 12H), 3.90 (m, 2H), 4.08 (t, 4H), 4.50 (t, 1H), 4.97 (t, 1H). |

(continued)

| Compound No. | NMR |
|---|---|
| II-1 | δ : 0.88 (t, 13H), 1.25-1.31 (m, 50H), 1.40 (m, 6H), 1.54-1.76 (m, 35H), 1.88 (m, 2H), 2.08 (m, 2H), 2.27 (m, 7H), 2.70 (brs, 2H), 3.33 (m, 1H), 4.08 (m, 5H), 4.92 (m, 1H). |

[Table 10]

| | |
|---|---|
| II-2 | δ : 0.88 (t, 13H), 1.25-1.42 (m, 57H), 1.54-1.60 (m, 38H), 1.91 (d, 1H), 2.11 (m, 1H), 2.21 (s, 3H), 2.27 (t, 4H), 2.55 (d, 1H), 3.18 (d, 1H), 3.75 (d, 1H), 3.96 (t, 1H), 4.08 (t, 4H), 4.95 (t, 1H). |
| II-3 | δ: 0.88 (t, 12H), 1.32 (dd, 52H), 1.63 (ddt, 21H), 1.90 (dt, 2H), 2.12 (t, 2H), 2.27 (t, 7H), 2.72 (t, 2H), 3.41 (dd, 1H), 4.08 (t, 6H), 4.93 (t, 1H). |
| II-4 | δ : 0.88 (t, 12H), 1.25-1.66 (m, 83H), 1.78 (m, 3H), 1.99 (d, 1H), 2.28 (m, 10H), 2.56 (m, 1H), 3.80 (m, 1H), 4.05 (m, 6H), 4.93 (m, 1H). |
| II-5 | δ : 0.88 (t, 12H), 1.25-1.40 (m, 53H), 1.51-1.62 (m, 23H), 2.28 (t, 4H), 2.35 (s, 3H), 2.97 (q, 2H), 3.65 (m, 2H), 3.99 (s, 2H), 4.08 (t, 4H), 4.15 (m, 1H), 4.94 (m, 1H). |
| II-6 | δ: 0.88 (t, 12H), 1.17-1.46 (m, 54H), 1.51-1.72 (m, 15H), 1.84-2.02 (m, 3H), 2.03-2.16 (m, 2H), 2.23-2.32 (m, 7H), 2.70 (brs, 2H), 3.25-3.33 (m, 1H), 3.43-3.52 (m, 4H), 4.08 (t, 4H), 5.81 (t, 1H). |
| II-7 | δ: 0.88 (t, 13H), 1.17-1.46 (m, 61 H), 1.51-1.70 (m, 8H), 1.81-1.92 (m, 3H), 2.04-2.16 (m, 2H), 2.22-2.32 (m, 7H), 2.64 (brs, 3H), 2.98 (s, 1H), 3.12 (s, 2H), 3.29 (brs, 1H), 3.48-3.63 (m, 4H), 4.08 (t, 4H). |
| II-9 | δ : 0.88 (t, 13H), 1.15-1.40 (m, 66H), 1.48-1.60 (m, 29H), 1.95 (m,2H), 2.28 (t, 5H), 2.38 (m, 2H), 2.44 (s, 3H), 2.95 (brs, 2H), 3.78 (t, 1H), 4.08 (t, 6H), 4.92 (t, 1H). |
| 11-10 | δ : 0.88 (t, 12H), 1.25-1.40 (m, 52H), 1.52-1.60 (m, 22H), 1.76 (m, 2H), 1.89 (m, 1H), 2.03 (m, 2H), 2.28 (t, 4H), 2.33 (s, 3H), 2.45 (m, 1H), 2.55 (m, 2H), 2.67 (m, 1H), 3.65 (m, 1H), 4.04 (s, 2H), 4.08 (t, 4H), 4.93 (t, 1H). |
| 11-11 | δ : 0.88 (t, 13H), 1.25-1.40 (m, 56H), 1.52-1.61 (m, 30H), 2.02 (d, 2H), 2.28 (t, 4H), 2.34 (s, 3H), 2.42 (m, 1H), 2.57 (m, 2H), 2.76 (m, 1H), 3.63 (m, 1H), 4.08 (t, 4H), 4.27 (q, 2H), 4.93 (t, 1H). |
| 11-12 | δ : 0.88 (t, 12H), 1.25-1.40 (m, 53H), 1.48-1.59 (m, 27H), 1.71 (m, 1H), 2.28 (t, 4H), 2.37 (s, 4H), 2.42 (m, 1H), 3.10 (s, 1H), 3.71 (d, 1H), 4.03 (s, 2H), 4.08 (t, 4H), 4.94 (m, 1H). |
| 11-13 | δ : 0.88 (t, 12H), 1.26-1.42 (m, 53H), 1.53-1.69 (m, 24H), 1.91 (d, 1H), 2.13 (dd, 1H), 2.28 (m, 7H), 2.47 (s, 1H), 2.73 (dd, 1H), 3.12 (s, 1H), 3.55 (d, 1H), 4.03 (s, 2H), 4.08 (t, 4H), 4.94 (m, 1H). |
| 11-14 | δ : 0.88 (t, 13H), 1.25-1.40 (m, 54H), 1.53-1.79 (m, 29H), 1.91 (d, 1H), 2.06 (m, 1H), 2.28 (m, 8H), 2.50 (brs, 4H), 4.08 (t, 4H), 4.48 (t, 1H), 4.88 (m, 1H). |
| 11-15 | δ : 0.88 (t, 12H), 1.25-1.31 (m, 50H), 1.43 (m, 5H), 1.52-1.70 (m, 26H), 1.99 (m, 2H), 2.21 (t, 1H), 2.28 (m, 10H), 3.58 (s, 1H), 4.08 (m, 6H), 4.93 (m, 1H). |
| 11-16 | δ : 0.88 (t, 12H), 1.25-1.40 (m, 52H), 1.52-1.60 (m, 24H), 2.15 (m, 10H), 2.28 (t, 4H), 2.83 (m , 1H), 3.97 (s, 2H), 4.08 (t, 4H), 4.13 (m, 1H), 4.94 (m, 1H). |
| 11-17 | δ : 0.88 (t, 12H), 1.25-1.59 (m, 77H), 1.72 (m, 1H), 1.84 (m, 1H), 2.28 (m, 10H), 2.49 (d ,1H), 3.66 (m, 1H), 3.95 (m, 1H), 4.08 (t, 4H), 4.93 (m, 1H). |
| 11-18 | δ : 0.88 (t, 12H), 1.25-1.40 (m, 56H), 1.52-1.60 (m, 22H), 1.93 (d, 2H), 2.14 (m, 3H), 2.28 (m, 10H), 3.28 (m, 1H), 4.08 (t, 6H), 4.94 (m, 1H). |
| 11-19 | δ : 0.88 (t, 14H), 1.01 (s, 6H), 1.17 (s, 6H), 1.20-1.66 (m, 98H), 1.90 (dd, 2H), 2.23 (s, 3H), 2.27 (t, 4H), 3.60-3.70 (m, 1H), 4.05-4.11 (m, 6H), 4.93-4.99 (m, 1H). |
| II-20 | δ : 0.88 (t, 12H), 1.25-1.40 (m, 57H), 1.52-1.59 (m, 29H), 2.00 (m, 2H), 2.28 (m, 7H), 2.37 (m, 5H), 2.73 (brs, 2H), 4.01 (s, 2H), 4.08 (m, 5H), 4.93 (m, 1H). |
| II-21 | δ : 0.88 (t, 13H), 1.25-1.40 (m, 54H), 1.53-1.59 (m, 23H), 2.28 (t, 4H), 2.35 (s, 3H), 2.61 (t, 2H), 2.71 (t, 2H), 3.60 (t, 2H), 3.64 (t, 2H), 4.08 (t, 6H), 4.95 (m, 1H). |

(continued)

| | |
|---|---|
| II-22 | δ : 0.88 (t, 13H), 1.25-1.40 (m, 60H), 1.51-1.59 (m, 31H), 1.77 (brs, 2H), 1.89 (brs, 2H), 2.06 (brs, 2H), 2.28 (t, 7H), 2.52 (brs, 1H), 2.84 (brs, 1H), 3.50 (m, 1H), 4.08 (m, 6H), 4.93 (m, 1H). |
| II-23 | δ : 0.88 (t, 12H), 1.25-1.40 (m, 52H), 1.52-1.62 (m, 27H), 1.87 (m, 2H), 2.13 (s, 6H), 2.28 (m, 5H), 2.44 (m ,2H), 3.80 (t, 1H), 3.98 (s, 2H), 4.08 (t, 4H), 4.94 (m, 1H). |

ALN-319 and YS-119 used as comparative examples were synthesized by the methods described in Patent Documents 4 and 5, respectively.

ALN-319 represents the following compound.

[Chemical Formula 124]

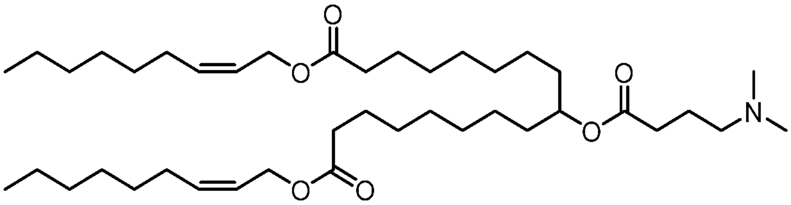

YS-119 represents the following compound.

[Chemical Formula 125]

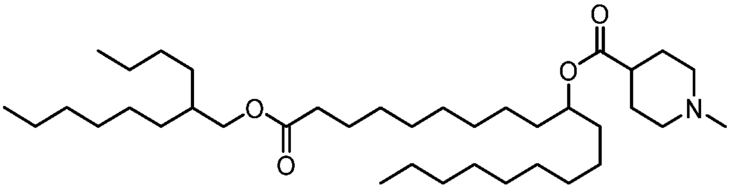

Preparation Example 1 Preparation of LNPs encapsulating siRNA 1

To obtain a lipid solution with the total lipid concentration of 40 mmol/L, the cationic lipid of the present invention or the comparative example, DSPC (Nippon Fine Chemical Co., Ltd.), Cholesterol (Nippon Fine Chemical Co., Ltd.) and DMG-PEG 2000 (NOF Corporation) were dissolved in ethanol at a molar ratio of 60/8.5/30/1.5. To obtain a nucleic acid solution with the concentration of 0.033 mmol/L, siRNA 1 was dissolved in 50 mmol/L acetic acid/sodium acetate buffer (pH 4.0, FUJIFILM Wako Pure Chemical Corporation). The lipid solution and the nucleic acid solution were mixed at flow rates of 3 mL/min and 9 mL/min, respectively, to obtain a mixed solution of nucleic acid and lipid. The obtained solution was replaced with phosphate buffered saline (pH 7.4, Thermo Fisher Scientific) using a dialysis membrane (REPLIGEN, 50 kDa MWCO or Thermo Fisher Scientific, 10 kDa MWCO) to obtain a LNP encapsulating siRNA 1.

The encapsulated nucleic acid is siRNA 1 targeting HPRT1 having the sequences shown in (SEQ ID NO: 1) and (SEQ ID NO: 2).

the sense strand:

G(F)^A(M)^U(F)^G(M)^A(F)^U(M)C(F)U(F)C(F)U(M)C(F)A(M)A(M)C(M)^U(F)^U(M)^U(F)^A(M)^A(F) (SEQ ID NO: 1);

the antisense strand:

PO-U(M)^U(F)^A(M)A(F)A(M)G(F)U(F)U(F)G(M)A(F)G(M)A(M)G(M)A(F)U(M)C(F)A(M)U(F)C(M)^T(D)^T(D) (SEQ ID NO: 2)

In the notation, (F) is 2'-F modified nucleic acid, (M) is 2'-OMe modified nucleic acid, (D) is DNA, ^ is phosphorothioate linkage, and PO is 5' terminal phosphate modification. In this description, they are used in common.

Preparation Example 2 Preparation of LNPs encapsulating siRNA 2

A LNP encapsulating siRNA 2 was obtained in the same manner as in Preparation Example 1 using the cationic lipid of the present invention or the comparative example and siRNA 2.

The encapsulated nucleic acid is siRNA 2 targeting SOD1 having the sequences shown in (SEQ ID NO: 3) and (SEQ ID NO: 4).

the sense strand:

G(F)^G(M)^G(F)^C(M)^A(F)^A(M)A(F)G(F)G(F)U(M)G(F)G(M)A(M)A(M)^A(F)^U(M)^G(F)^A(M)^A(F) (SEQ ID NO: 3);

the antisense strand:

PO-U(M)^U(F)^C(M)A(F)U(M)U(F)U(F)C(F)C(M)A(F)C(M)C(M)U(M)U(F)U(M)G(F)C(M)C(F)C(M)^T(D)^T(D)) (SEQ ID NO: 4)

Preparation Example 3 Preparation of LNPs encapsulating mRNA 1

To obtain a lipid solution with the total lipid concentration of 10 mmol/L, the cationic lipid of the present invention or the comparative example, DSPC, Cholesterol and DMG-PEG 2000 were dissolved in ethanol at a molar ratio of 60/8.5/30/1.5. To obtain a nucleic acid solution with the concentration of 100 mg/L, mRNA 1 was dissolved in 50 mmol/L acetic acid/sodium acetate buffer (pH 4.0). The lipid solution and the nucleic acid solution were mixed at flow rates of 3 mL/min and 9 mL/min, respectively, to obtain a mixed solution of nucleic acid and lipid. The external solution of the obtained solution was replaced with phosphate buffered saline (pH 7.4) using a dialysis membrane to obtain a LNP encapsulating mRNA 1.

The encapsulated nucleic acid is mRNA 1 targeting OVA (TriLink BioTechnologies, L-7210).

Test Example 1 Evaluation of particle size and encapsulation rate of the LNP encapsulating nucleic acid.

The particle size and encapsulation rate of the LNPs encapsulating nucleic acid prepared in Preparation Examples 1 to 3 were evaluated.

The average particle size of the LNP encapsulating nucleic acid was measured using Zetasizer Nano ZS (Malvern Panalytical Ltd.).

The encapsulation rate of the LNP encapsulating nucleic acid was measured using a Quant-iT™ RiboGreen™ RNA Assay Kit (Thermo Fisher Scientific). The total nucleic acid concentration was determined by measuring the composition collapsedin the presence of 2% Triton X-100. The unencapsulated nucleic acid concentration was determined by measuring the composition without collapsed in the absence of 2% Triton X-100. The encapsulation rate was calculated by the following formula.

Encapsulation rate (%) = 100 - (unencapsulated nucleic acid concentration/total nucleic acid concentration) x 100

The average particle size (nm) and encapsulation rate (%) of the LNPs encapsulating siRNA 1 are shown in Table 11. The leftmost column of Table 11 indicates the used cationic lipids.

[Table 11]

| Cationic lipid | Particle size (nm) | Encapsulation rate (%) |
|---|---|---|
| I - 8 | 82. 9 | 9 5. 6 |
| I - 16 | 97. 4 | 97. 2 |
| I - 28 | 90. 4 | 97. 8 |
| I-38 | 83. 0 | 97. 2 |
| I-39 | 82. 8 | 96. 0 |
| I-44 | 79. 7 | 95. 5 |
| 1-48 | 94. 9 | 95. 2 |
| YS-119 | 70. 5 | 89. 1 |
| ALN-319 | 79. 8 | 88. 3 |

As shown in Table 11, the LNPs encapsulating siRNA 1 prepared using the present invention exhibited particle sizes similar to those of YS-119 and ALN-319, which are comparative examples. Furthermore, it was revealed that the LNP encapsulating siRNA 1 prepared using the present invention formed a complex with a higher nucleic acid encapsulation rate compared to the comparative examples.

The average particle size (nm) and encapsulation rate (%) of the LNPs encapsulating siRNA 2 are shown in Table 12 and 13. The leftmost columns of Table 12 and 13 indicate the used cationic lipids.

[Table 12]

| Cationic lipid | Particle size (nm) | Encapsulation rate (%) |
|---|---|---|
| I-3 | 80. 1 | 95. 2 |
| I-8 | 81. 4 | 96. 5 |
| I-12 | 80. 4 | 95. 1 |
| I-16 | 90. 8 | 97. 2 |
| I-28 | 84. 7 | 96. 7 |
| I-38 | 81. 6 | 97. 4 |
| I-39 | 82. 3 | 96. 2 |
| I-44 | 78. 8 | 96. 7 |
| I-45 | 80. 2 | 96. 1 |
| I-48 | 93. 9 | 96. 3 |
| I-54 | 83. 1 | 95. 8 |
| YS-119 | 68. 5 | 90. 2 |

Table 13]

| Cationic lipid | Particle size (nm) | Encapsulation rate (%) |
|---|---|---|
| I-55 | 83.6 | 91.8 |
| I-58 | 82.9 | 91.1 |
| I-60 | 76.2 | 91.2 |
| I-61 | 119 | 95. 1 |

(continued)

| Cationic lipid | Particle size (nm) | Encapsulation rate (%) |
|---|---|---|
| I-65 | 69.0 | 98.0 |
| I-66 | 76.1 | 91.6 |
| I-69 | 74.0 | 97.3 |
| I-70 | 80.0 | 98.5 |
| I-72 | 82.2 | 91.2 |
| I-73 | 65. 6 | 98. 0 |
| I-75 | 65.6 | 100 |
| I-80 | 60.6 | 99.8 |
| I-81 | 87.9 | 87.4 |
| I-82 | 76.3 | 97.8 |
| I-87 | 71.8 | 96.9 |
| I-88 | 87.8 | 94.2 |
| II-3 | 79.6 | 94.3 |
| II-4 | 89.5 | 98.2 |
| II-5 | 82.9 | 92.1 |
| II-6 | 67.5 | 100 |
| II-7 | 74.5 | 92.3 |
| II-8 | 78.6 | 96.3 |
| II-9 | 75.6 | 96.4 |
| II-10 | 72.0 | 99.3 |
| II-12 | 74.6 | 91.9 |
| II-13 | 90.0 | 101 |
| II-14 | 92.9 | 93.3 |
| II-15 | 69.2 | 100 |
| II-16 | 86.8 | 99.0 |
| II-17 | 96.1 | 95.9 |
| II-18 | 80.5 | 100 |
| II-19 | 98.8 | 100 |
| II-21 | 75.6 | 97.1 |
| YS-119 | 75. 3 | 76.0 |

As shown in Tables 12 and 13, the LNPs encapsulating siRNA 2 prepared using the present invention exhibited particle sizes similar to those of the comparative example YS-119. Furthermore, it was revealed that the LNP encapsulating siRNA 2 prepared using the present invention formed a complex with a higher nucleic acid encapsulation rate compared to the comparative example.

The average particle size (nm) and encapsulation rate (%) of the LNPs encapsulating mRNA 1 are shown in Table 14. The leftmost column of Table 14 indicates the used cationic lipids.

[Table 14]

| Cationic lipid | Particle size (nm) | Encapsulation rate (%) |
|---|---|---|
| I-6 | 107.1 | 96. 5 |
| I-12 | 91.0 | 97. 3 |
| I-39 | 127. 1 | 97. 3 |
| ALN-319 | 110. 1 | 86. 3 |

As shown in Table 14, the LNPs encapsulating mRNA 1 prepared using the present invention exhibited average particle sizes similar to those of the comparative example ALN-319. Furthermore, it was revealed that the LNP encapsulating mRNA 1 prepared using the present invention formed a complex with a higher nucleic acid encapsulation rate compared to the comparative example.

As shown in Tables 11 to 14, it was revealed that the LNP encapsulating nucleic acid prepared using the present invention formed a complex with a higher nucleic acid encapsulation rate compared to the comparative example.

Test Example 2 Evaluation of storage stability of the composition

The LNPs encapsulating siRNA 1 and the LNPs encapsulating siRNA 2 prepared in Preparation Examples 1 and 2 were stored at room temperature in sealed vials, and the encapsulation rates before and after 4 weeks of storage were measured in the same manner as in Test Example 1. The change in encapsulation rate was calculated by the following formula.

$$\text{Change in encapsulation rate } \Delta\ (\%) = \text{Encapsulation rate (after 4 weeks)} - \text{Encapsulation rate (before storage)}$$

The change in encapsulation rate Δ (%) of the LNPs encapsulating siRNA 1 are shown in Table 15. The leftmost column of Table 15 indicates the used cationic lipids.

[Table 15]

| Cationic lipid | Change in encapsulation rate Δ (%) |
|---|---|
| I-3 | -0. 1 |
| I-8 | -0. 9 |
| I-10 | -0. 4 |
| I-11 | -0. 8 |
| I-16 | -0. 6 |
| I-37 | -0. 8 |
| I-38 | -0. 7 |
| I-44 | -0. 7 |
| I-45 | -0. 5 |
| I-48 | -0. 5 |
| I-53 | -0. 8 |
| ALN-319 | -12. 9 |

As shown in Table 15, it was revealed that the LNP encapsulating siRNA 1 prepared using the present invention showed less change in encapsulation rate during storage compared to the comparative example ALN-319 and had high storage stability.

The change in encapsulation rate Δ (%) of the LNPs encapsulating siRNA 2 are shown in Table 16. The leftmost column of Table 16 indicates the used cationic lipids.

[Table 16]

| Cationic lipid | Change in encapsulation rate Δ (%) |
|---|---|
| 1-38 | -0. 1 |
| I-44 | -0. 5 |
| I-46 | -0. 9 |
| 1-48 | -0. 9 |
| YS-119 | -5. 2 |

As shown in Table 16, it was revealed that the LNP encapsulating siRNA 2 prepared using the present invention showed less change in encapsulation rate during storage compared to the comparative example YS-119 and had high storage stability.

As shown in Tables 15 and 16, it was revealed that the LNP encapsulating nucleic acid prepared using the present invention showed less change in encapsulation rate during storage compared to the comparative examples and had high storage stability.

Test Example 3 Evaluation of suppressing activity of SOD1 gene expression of the composition using siRNA 2

A cell suspension of HeLa cell, human cervical cancer cell line, was prepared at 80000 cells/mL in DMEM containing 10% fetal bovine serum (FBS) and 500 units/mL Penicillin-Streptomycin. 0.10 mL of the cell suspension was seeded in a 96-well flat-bottom plate (CORNING) and cultured at 37°C for 1 day under 95 to 98% humidity and 5% $CO_2$. The LNP encapsulating siRNA 2 prepared in Preparation Example 2 was added to the medium so that the final concentration of siRNA 2 was 3 nM and the culture was continued. As a vehicle control, PBS was added in the medium.

24 hours after adding the LNP encapsulating nucleic acid, RNA was extracted from the cells and cDNA was synthesized using SuperPrep Cell Lysis&RT Kit for qPCR (Toyobo Co., Ltd.), and real-time PCR was performed using Fast SYBR Green Master Mix (ThermoFisher Scientific). GAPDH was used as an endogenous control. The test was performed with N=3 for each formulation.

The primer sequences for measuring the expression level of human SOD1 are

Fw primer: AGTGCAGGGCATCATCAATTTC (SEQ ID NO: 5); and
Rv primer: CCATGCAGGCCTTCAGTCAG (SEQ ID NO: 6).

The primer sequences for measuring the expression level of human GAPDH are

Fw primer: GCACCGTCAAGGCTGAGAAC (SEQ ID NO: 7); and
Rv primer: TGGTGAAGACGCCAGTGGA (SEQ ID NO: 8).

ΔΔCt is a value calculated by subtracting the difference (ΔCt) between the expression level of SOD1 (Ct value) and the expression level of GAPDH (Ct value) in cells to which each composition was added and the ΔCt of SOD1 in vehicle control cells. A change in the expression level of SOD1 was calculated as a relative expression level (RQ) with respect to the vehicle control using the following formula.

$$RQ\ (\%) = 2^{-\Delta\Delta Ct} \times 100$$

The SOD1 gene relative expression level (SOD1-RQ) adding each LNP encapsulating siRNA 2 are shown in Tables 17 and 18. The leftmost columns of Table 17 and 18 indicate the used cationic lipids.

[Table 17]

| Cationic lipid | SOD1-RQ (%) |
|---|---|
| 1-1 | 3.1 |
| I-3 | 8.7 |
| 1-4 | 8.2 |

(continued)

| Cationic lipid | SOD1-RQ (%) |
|---|---|
| I-6 | 7.4 |
| I-7 | 9.5 |
| 1-10 | 11.3 |
| 1-11 | 8.3 |
| 1-12 | 6.5 |
| 1-16 | 1.7 |
| I-28 | 8.7 |
| 1-37 | 4.3 |
| 1-39 | 7.9 |
| 1-46 | 11.4 |
| 1-48 | 19.1 |
| I-54 | 5.3 |
| YS-119 | 42.7 |

[Table 18]

| Cationic lipid | SOD 1-RQ (%) |
|---|---|
| I-55 | 6.5 |
| I-58 | 5.7 |
| I-60 | 5.7 |
| I-61 | 6.5 |
| I-66 | 5.8 |
| I-70 | 5.2 |
| I-72 | 6.2 |
| I-75 | 6.5 |
| I-80 | 8.8 |
| I-81 | 7.4 |
| I-82 | 5.8 |
| II-1 | 8.2 |
| II-3 | 5.8 |
| II-5 | 6.7 |
| II-6 | 4.3 |
| II-7 | 2.9 |
| II-12 | 7.3 |
| II-21 | 8.1 |
| YS-119 | 28.6 |

As shown in Tables 17 and 18, the LNPs encapsulating siRNA 2 prepared using the present invention exhibited a higher suppressing activity of SOD1 gene expression compared to the comparative example YS-119. Furthermore, it

was revealed that the encapsulated siRNA 2 could be efficiently transported into the cytoplasm.

Test Example 4 Evaluation of suppressing activity of Hprt1 gene expression of the composition using siRNA 1

A cell suspension of HeLa cell, human cervical cancer cell line, was prepared at 80000 cells/mL in DMEM containing 10% fetal bovine serum (FBS) and 500 units/mL Penicillin-Streptomycin. 0.10 mL of the cell suspension was seeded in a 96-well flat-bottom plate and cultured at 37°C for 1 day under 95 to 98% humidity and 5% $CO_2$. The nanoparticle encapsulating siRNA 1 prepared in Preparation Example 1 was added to the medium so that the final concentration of siRNA 1 was 1 nM and the culture was continued. As a vehicle control, PBS was added in the medium.
24 hours after adding the composition, RNA was extracted from the cells and cDNA was synthesized using SuperPrep Cell Lysis&RT Kit for qPCR, and real-time PCR was performed using Fast SYBR Green Master Mix. GAPDH was used as an endogenous control. The test was performed with N=3 for each formulation.
The primer sequences for measuring the expression level of human HPRT1 are

Fw primer: GGCAGTATAATCCAAAGATGGTCAA (SEQ ID NO: 9); and
Rv primer: GTCAAGGGCATATCCTACAACAAAC (SEQ ID NO: 10).

The primer sequences for measuring the level of human GAPDH expression are the sequence described in SEQ ID NO: 7 and the sequence described in SEQ ID NO: 8.
ΔΔCt is a value calculated by subtracting the difference (ΔCt) between the expression level of HPRT1 (Ct value) and the expression level of GAPDH (Ct value) in cells to which each LNP encapsulating siRNA 1 was added and the ΔCt of HPRT1 in vehicle control cells. A change in the expression level of HPRT1 was calculated as a relative expression level (RQ) with respect to the vehicle control using the following formula.

$$RQ\ (\%) = 2^{-\Delta\Delta Ct} \times 100$$

The HPRT1 gene relative expression level (HPRT1-RQ) adding each LNP encapsulating siRNA 1 are shown in Table 19. The leftmost column of Table 19 indicates the used cationic lipids.

[Table 19]

| Cationic lipid | HPRT1-RQ (%) |
|---|---|
| I-1 | 15. 7 |
| I-3 | 17. 1 |
| I-12 | 10. 4 |
| I-16 | 7. 5 |
| I-28 | 4. 9 |
| I-39 | 14. 2 |
| I-54 | 14. 9 |
| YS-119 | 28. 3 |

As shown in Table 19, it was indicated that the LNPs encapsulating siRNA 1 prepared using the present invention had a higher suppressing activity of HPRT1 gene expression compared to the comparative example YS-119. Furthermore, it was revealed that the encapsulated siRNA 1 could be efficiently transported into the cytoplasm.
As shown in Tables 17 to 19, it was revealed that the LNPs encapsulating nucleic acid prepared using the present invention could efficiently transport the encapsulated nucleic acid into the cytoplasm.

Test Example 5 Evaluation of activity of OVA protein expression of the composition using mRNA 1

A cell suspension of HeLa cell, human cervical cancer cell line, was prepared at 10000 cells/well in DMEM containing 10% fetal bovine serum (FBS) and 100 units/mL Penicillin, 0.10 mg/mL Streptomycin. 90 pL of the cell suspension was seeded in a 96-well flat-bottom plate (Greiner) and cultured at 37°C for 1 day under 95 to 98% humidity and 5% $CO_2$. The LNP encapsulating nucleic acid prepared in Preparation Example 3 was added to the medium so that

the nucleic acid amounts were 25 and 200 ng/well, respectively, and the culture was continued. As a vehicle control (control 1), DMEM was added in the medium. For comparison (control 2), only mRNA 1 was added to the medium at 25 ng/well or 200 ng/well. The test was performed three times for each LNP encapsulating nucleic acid and the added amount of nucleic acid, and the average value was calculated

24 hours after adding the LNP encapsulating nucleic acid, cell culture supernatants was collected. The OVA protein secreted into the culture supernatant was quantified using an OVA ELISA kit (ITEA Inc.).

The concentration of OVA protein in the culture supernatant adding each LNP encapsulating nucleic acid are shown in Table 19. The leftmost column of Table 20 indicates the type of control or the used cationic lipid. N. D. indicates not detected.

[Table 20]

| Control / Cationic lipid | RNA (ng/well) | OVA (ng/mL) |
|---|---|---|
| controll | 0 | N. D. |
| control2 | 25 | N. D. |
| | 200 | N. D. |
| I-6 | 25 | 59.2 |
| | 200 | 126. 4 |
| I-12 | 25 | 60.2 |
| | 200 | 117.6 |
| I-39 | 25 | 147.5 |
| | 200 | 306. 4 |
| YS-119 | 25 | N. D. |
| | 200 | N. D. |
| ALN-319 | 25 | N. D. |
| | 200 | 9.8 |

As shown in Table 20, it was indicated that the LNPs encapsulating mRNA 1 prepared using the present invention promoted OVA protein expression compared to the comparative examples YS-119 and ALN-319. In other words, it was revealed that the LNPs encapsulating nucleic acid prepared using the present invention could efficiently transport the encapsulated nucleic acid into the cytoplasm.

[INDUSTRIAL APPLICABILITY]

As the above, the lipid particles containing the novel cationic lipid of the present invention can efficiently encapsulate nucleic acid and maintain a high encapsulation rate even after storage for a certain period of time. Furthermore, the lipid particles containing the cationic lipid of the present invention exhibit high knockdown activity in vitro and promotion of protein expression. In view of the above, the cationic lipid of the present invention can be used as a pharmaceutical composition for intracellular nucleic acid delivery.

## Claims

**1.** A compound represented by Formula (I):

[Chemical Formula 1]

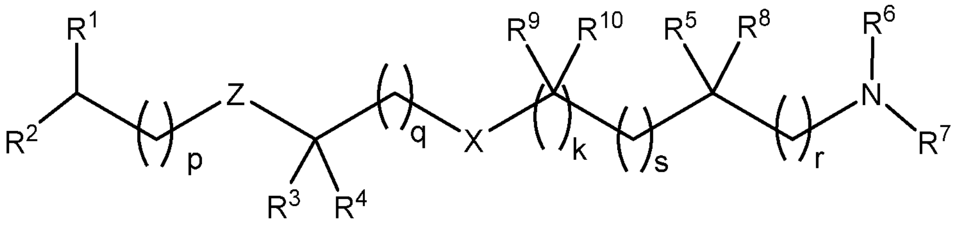

(I)

wherein

$R^1$ is a substituted or unsubstituted formula $-(CH_2)_a-L_1(CH_2)_b-CH_3$;
$R^2$ is substituted or unsubstituted $C_5$-$C_{20}$ alkyl or a substituted or unsubstituted formula: $-(CH_2)_c-L_2-(CH_2)_d-CH_3$;
$L_1$ and $L_2$ are each independently -C(=O)O-, -OC(=O)- or -OC(=O)O-;
a and b are each independently an integer of 1 or more, the total of a and b is an integer of 5 to 25;
c and d are each independently an integer of 1 or more, the total of c and d is an integer of 5 to 25;
substituents for the formula: $-(CH_2)_a-L_1-(CH_2)_b-CH_3$ in $R^1$, and $C_5$-$C_{20}$ alkyl and the formula: $-(CH_2)_c-L_2-(CH_2)_d-CH_3$ in $R^2$ are each independently selected from substituent group a;
the substituent group a is the group consisting of a halogen atom, oxo, cyano, nitro, sulfanyl, carboxy, $C_1$-$C_{10}$ alkyl, halogenated $C_1$-$C_{10}$ alkyl, $C_2$-$C_{10}$ alkenyl, halogenated $C_2$-$C_{10}$ alkenyl, $C_2$-$C_{10}$ alkynyl, halogenated $C_2$-$C_{10}$ alkynyl, $C_1$-$C_{10}$ alkoxy, halogenated $C_1$-$C_{10}$ alkoxy, $C_1$-$C_{10}$ alkylsulfanyl, halogenated $C_1$-$C_{10}$ alkylsulfanyl, $C_1$-$C_{11}$ alkanoyl, $C_1$-$C_{11}$ alkanoyloxy, $C_1$-$C_{10}$ alkoxy $C_1$-$C_{10}$ alkoxy, $C_1$-$C_{10}$ alkoxycarbonyl, $C_1$-$C_{10}$ alkylcarbamoyl, di($C_1$-$C_{10}$ alkyl)carbamoyl, $C_1$-$C_{10}$ alkoxycarbonyloxy, $C_1$-$C_{11}$ alkanoyl($C_1$-$C_{10}$ alkyl)amino, $C_1$-$C_{10}$ alkoxycarbonylamino and $C_1$-$C_{10}$ alkylcarbamoyloxy;
$R^3$ and $R^4$ are each independently a hydrogen atom or substituted or unsubstituted $C_1$-$C_6$ alkyl;
or $R^3$ and $R^4$ may be taken together to form a substituted or unsubstituted $C_3$-$C_5$ non-aromatic carbocycle;
$R^5$ is a hydrogen atom or substituted or unsubstituted $C_1$-$C_6$ alkyl;
$R^6$ and $R^7$ are each independently a hydrogen atom, substituted or unsubstituted $C_1$-$C_6$ alkyl, substituted or unsubstituted $C_2$-$C_6$ alkenyl, substituted or unsubstituted $C_2$-$C_6$ alkynyl or substituted or unsubstituted $C_3$-$C_7$ non-aromatic carbocyclyl;
$R^8$, $R^9$ and $R^{10}$ are a hydrogen atom;
or $R^3$ and $R^7$ may be taken together with the atom to which each $R^3$ and $R^7$ are attached to form a substituted or unsubstituted non-aromatic heterocycle;
$R^3$ and $R^8$ may be taken together with the atom to which each $R^3$ and $R^8$ are attached to form a substituted or unsubstituted non-aromatic heterocycle;
$R^5$ and $R^6$ may be taken together with the atom to which each $R^5$ and $R^6$ are attached to form a substituted or unsubstituted non-aromatic heterocycle;
$R^6$ and $R^7$ may be taken together with the atom to which each $R^6$ and $R^7$ are attached to form a substituted or unsubstituted non-aromatic heterocycle;
$R^8$ and $R^9$ may be taken together with the atom to which each $R^8$ and $R^9$ are attached to form a substituted or unsubstituted non-aromatic carbocycle or a substituted or unsubstituted non-aromatic heterocycle, when $R^8$ and $R^9$ form the ring, k is 1 or 2;
$R^7$ and $R^8$, when $R^5$ and $R^6$ form a ring, may be taken together with the atom to which each $R^7$ and $R^8$ are attached to form a substituted or unsubstituted non-aromatic heterocycle;
Z is -OC(=O)-, -C(=O)O-, -OC(=O)O-, -C(=O)N(R)-, -N(R)C(=O)-, - OC(=O)N(R)-, -N(R)C(=O)N(R)-, -N(R)C(=O)O-, -C(=S)N(R)-, -N(R)C(=S)-, - C(=O)S-, -SC(=O)-, $-N(R)S(=O)_2-$, $-OS(=O)_2-$, -OP(=O)(-OR)O-, -OP(=S)(-OR)O-, $- OS(=O)_2-O-$, $-S(=O)_2-N(R)-$, -OP(=O)(-NR)O-, -C(=S)O- or -OC(=S)-;
X is O or S;
p, q, s and k are each independently an integer of 0 to 2;
r is an integer of 0 to 5;
R are each independently a hydrogen atom or substituted or unsubstituted $C_1$-$C_6$ alkyl;
substituents for $C_1$-$C_6$ alkyl in $R^3$ to $R^7$ and R, and $C_2$-$C_6$ alkenyl or $C_2$-$C_6$ alkynyl in $R^6$ and $R^7$ are each independently selected from substituent group β1;
the substituent group β1 is the group consisting of a halogen atom, oxo, hydroxy, cyano, sulfanyl, amino, $C_1$-$C_6$ alkoxy, $C_1$-$C_6$ alkylsulfanyl, $C_1$-$C_6$ alkylamino, di$C_1$-$C_6$ alkylamino and $C_1$-$C_7$ alkanoyl;
substituents for a ring formed by $R^3$ and $R^4$, $C_3$-$C_7$ non-aromatic carbocyclyl in $R^6$ and $R^7$, a ring formed by $R^3$ and $R^7$, a ring formed by $R^3$ and $R^8$, a ring formed by $R^5$ and $R^6$, a ring formed by $R^6$ and $R^7$, a ring formed by

$R^7$ and $R^8$ and a ring formed by $R^8$ and $R^9$ are each independently selected from substituent group β2; the substituent group β2 is the group consisting of the substituent group β1, $C_1$-$C_6$ alky and halogenated $C_1$-$C_6$ alkyl,

or a pharmacologically acceptable salt thereof.

**2.** The compound according to claim 1, wherein $L_1$ is -C(=O)O- or - OC(=O)-, or a pharmacologically acceptable salt thereof.

**3.** The compound according to claim 1 or 2, wherein a and b are each independently an integer of 5 to 10, or a pharmacologically acceptable salt thereof.

**4.** The compound according to any one of claims 1 to 3, wherein $R^1$ is a formula: $-(CH_2)_a-L_1-(CH_2)_b-CH_3$, substituted with $C_1$-$C_{10}$ alkyl, or a pharmacologically acceptable salt thereof.

**5.** The compound according to any one of claims 1 to 4, wherein $R^2$ is a substituted or unsubstituted formula: $-(CH_2)_c-L_2-(CH_2)_d-CH_3$, or a pharmacologically acceptable salt thereof.

**6.** The compound according to claim 5, wherein $R^2$ is a formula: $-(CH_2)_c-L_2-(CH_2)_d-CH_3$, substituted with $C_1$-$C_{10}$ alkyl, or a pharmacologically acceptable salt thereof.

**7.** The compound according to claim 5 or 6, wherein $L_2$ is -C(=O)O- or - OC(=O)-, or a pharmacologically acceptable salt thereof.

**8.** The compound according to any one of claims 5 to 7, wherein c and d are each independently an integer of 5 to 10, or a pharmacologically acceptable salt thereof.

**9.** The compound according to any one of claims 1 to 4, wherein $R^2$ is substituted or unsubstituted $C_5$-$C_{20}$ alkyl, or a pharmacologically acceptable salt thereof.

**10.** The compound according to any one of claims 1 to 9, wherein

Z is -OC(=O)-, -C(=O)O-, -OC(=O)O-, -C(=O)N(R)-, -N(R)C(=O)O- or - N(R)C(=O)-;
R are each independently a hydrogen atom or unsubstituted $C_1$ alkyl;

or a pharmacologically acceptable salt thereof.

**11.** The compound according to any one of claims 1 to 9, wherein Z is - OC(=O)-, -C(=O)O- or -OC(=O)O-, or a pharmacologically acceptable salt thereof.

**12.** The compound according to any one of claims 1 to 11, wherein $R^6$ and $R^7$ are each independently substituted or unsubstituted $C_1$-$C_6$ alkyl, or a pharmacologically acceptable salt thereof.

**13.** The compound according to any one of claims 1 to 11, wherein $R^5$ and $R^6$ are taken together with the atom to which each $R^5$ and $R^6$ are attached to form a substituted or unsubstituted non-aromatic heterocycle, or a pharmacologically acceptable salt thereof.

**14.** The compound according to any one of claims 1 to 11, wherein $R^6$ and $R^7$ are taken together with the atom to which each $R^6$ and $R^7$ are attached to form a substituted or unsubstituted non-aromatic heterocycle, or a pharmacologically acceptable salt thereof.

**15.** The compound according to any one of claims 1 to 11, wherein

$R^5$ and $R^6$ are taken together with the atom to which each $R^5$ and $R^6$ are attached to form a substituted or unsubstituted non-aromatic heterocycle;
and $R^7$ and $R^8$ are taken together with the atom to which each $R^7$ and $R^8$ are attached to form a substituted or unsubstituted non-aromatic heterocycle;

or a pharmacologically acceptable salt thereof.

**16.** The compound according to any one of claims 1 to 15, wherein r is an integer of 0 to 3, or a pharmacologically acceptable salt thereof.

**17.** The compound according to claim 1, selected from the group consisting of compounds I-6, I-8, I-37, I-39, I-55, I-56, I-58, I-60, I-65, I-66 and I-69, or a pharmacologically acceptable salt thereof.

**18.** A pharmaceutical composition comprising the compound according to any one of claims 1 to 17 or a pharmaceutically acceptable salt thereof and a nucleic acid.

**19.** The pharmaceutical composition according to claim 18, wherein the nucleic acid is siRNA or mRNA.

## INTERNATIONAL SEARCH REPORT

International application No.
**PCT/JP2022/004118**

**A. CLASSIFICATION OF SUBJECT MATTER**

***C07C 217/08***(2006.01)i; ***A61K 9/127***(2006.01)i; ***A61K 31/7105***(2006.01)i; ***A61K 31/713***(2006.01)i; ***A61K 47/18***(2006.01)i; ***A61K 48/00***(2006.01)i; ***A61P 25/00***(2006.01)i; ***C07C 217/12***(2006.01)i; ***C07C 311/26***(2006.01)i; ***C07D 205/04***(2006.01)i; ***C07D 207/12***(2006.01)i; ***C07D 211/46***(2006.01)i; ***C07D 211/54***(2006.01)i; ***C07D 223/08***(2006.01)i; ***C07D 295/03***(2006.01)i; ***C07D 295/088***(2006.01)i; ***C07D 309/08***(2006.01)i; ***C07D 471/10***(2006.01)i; ***C07D 471/22***(2006.01)i; ***C07D 491/113***(2006.01)i; ***C07F 9/59***(2006.01)i; ***C12N 5/10***(2006.01)i; ***C12N 15/11***(2006.01)i; ***C12N 15/113***(2010.01)i; ***C12N 15/88***(2006.01)i

FI: C07C217/08; A61K9/127; A61K31/7105; A61K31/713; A61K47/18; A61K48/00; A61P25/00; C07C217/12 ZNA; C07C311/26; C07D205/04; C07D207/12; C07D211/46; C07D211/54; C07D223/08; C07D295/03; C07D295/088; C07D309/08; C07D471/10 101; C07D471/22; C07D491/113 CSP; C07F9/59; C12N5/10; C12N15/11 Z; C12N15/113 Z; C12N15/88 Z

According to International Patent Classification (IPC) or to both national classification and IPC

**B. FIELDS SEARCHED**

Minimum documentation searched (classification system followed by classification symbols)

C07C217/08; A61K9/127; A61K31/7105; A61K31/713; A61K47/18; A61K48/00; A61P25/00; C07C217/12; C07C311/26; C07D205/04; C07D207/12; C07D211/46; C07D211/54; C07D223/08; C07D295/03; C07D295/088; C07D309/08; C07D471/10; C07D471/22; C07D491/113; C07F9/59; C12N5/10; C12N15/11; C12N15/113; C12N15/88

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | REGISTRY (STN) [online], 08 June 2015, retrieved on 06 April 2022 particularly, CAS registry number: 1776154-46-1, etc. | 1-2, 4-7, 14, 16 |
| X | US 2016/0016986 A1 (DESHPANDE, Milind) 21 January 2016 (2016-01-21) example 6 | 1-2, 4-7, 14, 16 |
| X | CN 101307076 A (QIN, Yinlin) 19 November 2008 (2008-11-19) examples 15-18 | 1-2, 4-7, 14, 16 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 April 2022** | **19 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/004118

C. DOCUMENTS CONSIDERED TO BE RELEVANT

| Category* | Citation of document, with indication, where appropriate, of the relevant passages | Relevant to claim No. |
|---|---|---|
| A | WO 2019/235635 A1 (FUJIFILM CORP.) 12 December 2019 (2019-12-12) claims, examples | 1-19 |
| A | JP 2013-533224 A (ALNYLAM PHARMACEUTICALS, INC.) 22 August 2013 (2013-08-22) claims, examples | 1-19 |
| P, X | WO 2021/038509 A1 (GLAXOSMITHKLINE INTELLECTUAL PROPERTY (NO. 2) LIMITED) 04 March 2021 (2021-03-04) claim 13 | 1-2, 4-7, 10-11, 14, 16 |
| P, A | US 2021/0230112 A1 (GUIDE THERAPEUTICS, INC.) 29 July 2021 (2021-07-29) entire text, all drawings | 1-19 |

Form PCT/ISA/210 (second sheet) (January 2015)

## INTERNATIONAL SEARCH REPORT

International application No.
**PCT/JP2022/004118**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT
**Information on patent family members**

International application No.
**PCT/JP2022/004118**

| Patent document cited in search report | Publication date (day/month/year) | Patent family member(s) | Publication date (day/month/year) |
|---|---|---|---|
| US 2016/0016986 A1 | 21 January 2016 | WO 2016/014527 A2<br>example 6 | |
| CN 101307076 A | 19 November 2008 | (Family: none) | |
| WO 2019/235635 A1 | 12 December 2019 | US 2021/0085604 A1<br>claims, examples<br>EP 3805198 A1<br>CN 112262122 A<br>KR 10-2021-0015948 A | |
| JP 2013-533224 A | 22 August 2013 | US 2012/0027803 A1<br>claims, examples<br>WO 2011/153493 A2<br>EP 2575764 A2<br>CN 103096875 A<br>KR 10-2013-0082141 A | |
| WO 2021/038509 A1 | 04 March 2021 | (Family: none) | |
| US 2021/0230112 A1 | 29 July 2021 | WO 2021/114969 A1<br>entire text, all drawings | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010144740 A
- WO 2010054405 A
- WO 2015105131 A
- WO 2011153493 A
- WO 2016104580 A
- WO 2015005253 A
- WO 2019235635 A
- JP 2003040847 A
- WO 2020118041 A
- WO 2017222016 A
- WO 2019131580 A


### Non-patent literature cited in the description


- *Molecular Therapy,* 2013, vol. 21 (8), 1570-1578
- *Angewandte Chemie,* 2012, vol. 51 (34), 8529-33
- *International Journal of Pharmaceutics,* 2017, vol. 519, 34-43
- Labeled Compounds (Part A). Isotopes in the Physical and Biomedical Sciences. 1987, vol. 1